(19) Europäisches Patentamt
European Patent Office
Office européen des brevets

(11) **EP 1 117 802 B1**

(12) **EUROPÄISCHE PATENTSCHRIFT**

(45) Veröffentlichungstag und Bekanntmachung des Hinweises auf die Patenterteilung:
**13.01.2010 Patentblatt 2010/02**

(21) Anmeldenummer: **99952542.1**

(22) Anmeldetag: **08.10.1999**

(51) Int Cl.:
***C12N 15/54*** *(2006.01)* ***C12N 15/63*** *(2006.01)*
***C12N 1/21*** *(2006.01)* ***C12N 5/10*** *(2006.01)*
***C12N 9/10*** *(2006.01)* ***C12P 21/02*** *(2006.01)*
***C07K 16/12*** *(2006.01)* ***A01H 5/00*** *(2006.01)*
***C08B 30/00*** *(2006.01)*

(86) Internationale Anmeldenummer:
**PCT/EP1999/007562**

(87) Internationale Veröffentlichungsnummer:
**WO 2000/022140 (20.04.2000 Gazette 2000/16)**

(54) **NUCLEINSÄUREMOLEKÜLE CODIEREND EIN VERZWEIGUNGSENZYM AUS BAKTERIEN DER GATTUNG NEISSERIA SOWIE VERFAHREN ZUR HERSTELLUNG VON ALPHA-1,6-VERZWEIGTEN ALPHA-1,4-GLUCANEN**

NUCLEIC ACID MOLECULES WHICH CODE A BRANCHING ENZYME FROM BACTERIA OF THE GENUS NEISSERIA, AND A METHOD FOR PRODUCING ALPHA-1,6-BRANCHED ALPHA-1,4-GLUCANS

MOLECULES D'ACIDE NUCLEIQUE CODANT UNE ENZYME DE RAMIFICATION DE BACTERIES DU GENRE NEISSERIA ET PROCEDE POUR PRODUIRE DES ALPHA-1,4 GLUCANES RAMIFIEES EN ALPHA-1,6

(84) Benannte Vertragsstaaten:
**AT BE CH CY DE DK ES FI FR GB GR IE IT LI LU MC NL PT SE**

(30) Priorität: **09.10.1998 DE 19846635**
**27.05.1999 DE 19924342**

(43) Veröffentlichungstag der Anmeldung:
**25.07.2001 Patentblatt 2001/30**

(60) Teilanmeldung:
**06026449.6 / 1 806 399**

(73) Patentinhaber:
- **Bayer BioScience GmbH**
  **14473 Potsdam (DE)**
- **Max-Planck-Gesellschaft zur Förderung der Wissenschaften e.V.**
  **Berlin (DE)**

(72) Erfinder:
- **BÜTTCHER, Volker**
  **D-37697 Lauenförde (DE)**
- **QUANZ, Martin**
  **D-10997 Berlin (DE)**

(74) Vertreter: **Vossius & Partner**
**Siebertstrasse 4**
**81675 München (DE)**

(56) Entgegenhaltungen:
**EP-A- 0 418 945** **WO-A-97/29186**
**WO-A-98/44780** **DE-A- 4 447 388**
**US-A- 4 454 161**

- **BIRKHED D. ET AL: "Structure of extracellular polysaccharides synthesized from sucrose by *Neisseria* isolated from human dental plaque" ARCHIVES OF ORAL BIOLOGY_(_ARCH. ORAL BIOL._), 24/1 (63-66), XP002128946 United Kingdom**
- **BECHTEL D R; ET AL.: "Size-distribution of wheat starch granules during endosperm development." CEREAL CHEMISTRY, Bd. 67, Seiten 59-63, XP009072558**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Nucleinsäuremoleküle codierend ein Verzweigungsenzym aus Bakterien der Gattung Neisseria, Vektoren, Wirtszellen, Pflanzenzellen und Pflanzen enthaltend solche Nucleinsäuremoleküle, sowie Verfahren zur Herstellung von Stärke.

α-1,6 verzweigte α-1,4-Glucaue sind in verschiedener Hinsicht von außerordentlichem Interesse, da sie sich beispielsweise für die Herstellung von Produkten im Bereich der pharmazeutischen und kosmetischen Industrie eignen. Beispielsweise können sie als Bindemittel für Tabletten, als Trägerstoffe für pharmazeutische Wirkstoffe, als Verpakkungsmaterial, als Trägerstoff für Puderzusatzstoffe, als UV-absorbierender Zusatzstoff in Sonnenschutzcremes und als Trägermaterial von Aroma- oder Duftstoffen verwendet werden.

α-1,6-verzweige α-1,4-Glucane kommen im Pflanzenreich hauptsächlich als Amylopektin als ein Bestandteil der Stärke vor. Im Tierreich und in Bakterien treten diese verzweigten Glucane hauptsächlich in Form von Glycogen auf. Das Polysaccharid Stärke ist aus chemisch einheitlichen Grundbausteinen, den Glucosemolekülen, aufgebaut, stellt jedoch ein komplexes Gemisch unterschiedlicher Molekülformen dar, die Unterschiede hinsichtlich des Polymerisations- und des Verzweigungsgrades aufweisen und sich somit in ihren physikalisch-chemischen Eigenschaften stark voneinander unterscheiden. Man differenziert zwischen Amylosestärke, einem im wesentlichen unverzweigten Polymer aus α-1,4-glycosidisch verknüpften Glucoseeinheiten, und der Amylopektinstärke, einem verzweigten Polymer, bei dem die Verzweigungen durch das Auftreten zusätzlicher α-1,6-glycosidischer Verknüpfungen zustande kommen. Nach Lehrbuchangaben (Voet and Voet, Biochemistry, John Wiley & Sons, 1990) treten die α-1,6-Verzweigungen durchschnittlich alle 24 bis 30 Glucosereste auf. Dies entspricht einem Verzweigungsgrad von ca. 3% - 4%. Die Angaben zum Verzweigungsgrad sind variabel und abhängig von der Herkunft (z.B. Pflanzenspezies, Pflanzensorte usw.) der jeweiligen Stärke. In typischen für die industrielle Stärkeproduktion verwendeten Pflanzen variiert der Amyloseanteil am Gesamtstärkegehalt zwischen 10 und 25%. Zur Herstellung von α-1,6-verzweigten α-1,4-Glucanen unterschiedlichen Verzweigungsgrades wurden bereits verschiedene Ansätze beschrieben, die die Verwendung von (transgenen) Pflanzen umfassen. So führt beispielsweise die heterologe Expression einer bakteriellen Glycogensynthase in Kartoffelpflanzen zu einer leichten Absenkung des Amylosegehaltes, zu einer Steigerung des Verzweigungsgrades und zu einer Veränderung des Verzweigungsmusters des Amylopektins im Vergleich zu Wildtyp-Pflanzen (Shewmaker et al., Plant. Physiol. 104 (1994), 1159-1166). Ferner wurde beobachtet, daß die heterologe Expression des Verzweigungsenzyms aus E. coli (glgB) in amylose-freien Kartoffelmutanten (amf) (Jacobsen et al., Euphytica 44 (1989), 43-48) zu Amylopektinmolekülen mit 25 % mehr Verzweigungspunkten führte (Kortstee et al., Plant J. 10 (1996), 83-90) als die Kontrolle (amf). Dies Isolierung der in transgenen Pflanzen erzeugten Glucane unterschiedlichen Verzweigungsgrades bedarf zur Entfernung z. B. der Amylosekomponente zusätzlicher Reinigungsschritte, die sehr aufwendig und daher zeit- und kostenintensiv sind. Ferner ist eine gezielte Einstellung des Verzweigungsgrades mit derartigen Ansätzen nicht möglich. Weiterhin besitzen solche in-vivo-Verfahren aufgrund schwankender Versuchsbedingungen (Umweltfaktoren, Standort) eine hohe Variabilität bezüglich der Produktqualität.

Einen höheren Verzweigungsgrad als das Amylopektin weist Glycogen auf. Auch dieses Polysaccharid enthält α-1,6-verzweigte α-1,4- Glucane. Glycogen unterscheidet sich von Stärke auch in der durchschnittlichen Seitenkettenlänge und im Polymerisationsgrad. Glycogen enthält nach Lehrbuchangaben (Voet and Voet, Biochemistry, John Wiley & Sons, 1990) durchschnittlich alle 8 bis 12 Glucosereste einen α-1,6-Verzweigungspunkt. Dies entspricht einem Verzweigungsgrad von ca. 8% bis 12%. Für das Molekulargewicht von Glycogen findet man unterschiedliche Angaben, die von 1 Million bis über 1000 Millionen reichen (D. J. Manners in: Advances in Carbohydrate Chemistry, Ed. M. L. Wolfrom, Academic Press, New York (1957), 261-298; Geddes et al., Carbohydr. Res. 261 (1994), 79-89). Auch diese Angaben sind stark abhängig vom jeweiligen Ursprungsorganismus, dessen Ernährungszustand sowie von der Art der Isolierung des Glycogens. Glycogen wird in der Regel aus Muscheln (z. B. Mytillus edulis), aus Säugerlebern oder -muskeln (z.B. Kaninchen, Ratten) gewonnen (Bell et al., Biochem. J. 28 (1934), 882; Bueding and Orrell, J. Biol. Chem. 236 (1961), 2854). Dies macht eine Herstellung im industriellen Maßstab Zeit- und kostenintensiv.

Die beschriebenen natürlich auftretenden α-1,6-verzweigten α-1,4-Glucane, Stärke und Glycogen, verhalten sich je nach ihrem Anteil an 1,6-glycosidischen Verzweigungen sehr unterschiedlich. Dies gilt u. a. in Bezug auf ihre Löslichkeit, Transparenz, enzymatische Hydrolyse, Rheologie, Gelbildungs- und Retrogradationseigenschaften. Eine derartige Schwankung in den Eigenschaften ist jedoch für viele industrielle Anwendungen nicht immer tolerierbar.

Eine Alternative zu der Gewinnung von α-1,6-verzweigten α-1,4-Glucanen aus Pflanzen oder tierischen Organismen sind in-vitro-Ansätze. Diese zeichnen sich gegenüber den in-vivo-Verfahren generell durch eine bessere Steuerbarkeit und eine höhere Reproduzierbarkeit aus, weil die Reaktionsbedingungen in vitro, im Gegensatz zu den Bedingungen in einem lebenden Organismus, definiert eingestellt werden können. Dies ermöglicht in der Regel die Herstellung von gleichbleibenden Produkten großer Einheitlichkeit und Reinheit und damit von großer Qualität, die für die weitere industrielle Nutzung von großer Bedeutung ist. Die Aufarbeitung von Produkten gleichbleibender Qualität führt zu Kostensenkungen, weil die Verfahrensparameter, die für die Aufarbeitung erforderlich sind, nicht für jeden Aufarbeitungsansatz neu optimiert werden müssen. Ein weiterer Vorteil bestimmter in-vitro-Verfahren liegt darin, daß die Produkte frei von den Organismen sind, die im in-vivo-Verfahren verwendet werden. Für bestimmte Anwendungen in der Nahrungsmittelindustrie und der Pharmaindustrie ist dies dringend erforderlich.
Die in-vitro-Verfahren lassen sich generell in zwei verschiedene Gruppen unterteilen.
Bei der einen Gruppe von Verfahren werden verschiedene Substrate, wie z.B. Amylose, Amylopektin und Glycogen, der Aktivität eines Verzweigungsenzyms ausgesetzt.
So konnten Borovsky et al. (Eur. J. Biochem. 59 (1975), 615-625) zeigen, daß die Verwendung des Verzweigungsenzyms aus Kartoffel in Verbindung mit dem Substrat Amylose zu Produkten führt, die zwar amylopektinähnlich sind, sich von diesem jedoch strukturell unterscheiden.
Boyer und Preiss (Biochemistry 16 (1977), 3693-3699) zeigten ferner, daß gereinigtes Verzweigungsenzym (α-1,4-Glucan:α-1,4-Glucan 6-Glycosyltransferase) aus E. coli verwendet werden kann, um den Verzweigungsgrad von Amylose oder Amylopektin zu erhöhen.
Wird hingegen Glycogen aus E. coli oder Kaninchenleber mit dem Verzweigungsenzym aus E. coli inkubiert, so erreicht man nur eine schwache Zunahme des Verzweigungsgrades (Boyer und Preiss, a. a. O.).
Auch Rumbak et al. (J. Bacteriol. 173 (1991), 6732-6741) konnten den Verzweigungsgrad von Amylose, Amylopektin und Glycogen nachträglich erhöhen, indem sie diese Substrate mit dem Verzweigungsenzym aus Butyrivibrio fibrisolvens inkubierten.
Ein ähnlicher Ansatz wurde von Okada et al. verfolgt (Patent-Nr. US 4454161), um die Eigenschaften von stärkeenthaltenden Nahrungsmitteln zu verbessern. Dabei wurden Substanzen wie Amylose, Amylopektin, Stärke oder Dextrin zusammen mit einem Verzweigungsenzym inkubiert. Dies hatte vorteilhafte Effekte auf die Haltbarkeit von Nahrungsmitteln, die die entsprechend modifizierten Substanzen enthielten. Ferner beschreibt die Patentanmeldung EP-A1 0 690 170 die Umsetzung gelierter Stärke in wäßriger Lösung unter Verwendung eines Verzweigungsenzyms. Dies führt zu Stärken mit vorteilhaften Eigenschaften bei der Papierherstellung.
Die vorangehend beschriebenen in-vitro-Verfahren weisen jedoch den Nachteil auf, daß bereits aufgrund des schwankenden Verzweigungsgrades der Edukte (z. B. Stärke, Amylopektin etc.) die Herstellung einheitlicher Produkte nicht möglich ist. Ferner ist eine gezielte Steuerung des Verzweigungsgrades nicht möglich und darüber hinaus sind die verwendeten Substrate verhältnismäßig teuer.

Die andere Gruppe von in-vitro-Verfahren umfaßt die de-novo-Synthese α-1,6-verzweigter α-1,4-Glucane ausgehend von verschiedenen Substraten (Glucose-1-phosphat, ADP-Glucose, UDP-Glucose) unter Verwendung einer Enzymkombination bestehend aus einem 1,4-Glucankettenbildenden Enzym (Phosphorylase, Stärkesynthase, Glycogensynthase) und einem Verzweigungsenzym.
So konnte Illingwort et al. (Proc. Nat. Acad. Sci. USA 47 (1961), 469-478) für ein in-vitro-Verfahren unter Verwendung einer Phosphorylase a aus Muskeln (Organismus unbekannt) in Kombination mit einem Verzweigungsenzym (Organismus unbekannt) zeigen, daß die de-novo-Synthese glycogenähnlicher Moleküle unter Verwendung des Substrats Glucose-1-phosphat möglich ist. Boyer und Preiss (a. a. O.) kombinierten die enzymatische Aktivität einer Phosphorylase aus Kaninchenmuskeln bzw. einer Glycogensynthase aus E. coli mit der eines Verzweigungsenzyms aus E. coli unter Verwendung des Substrats Glucose-1-phosphat bzw. UDP-Glucose und erzeugten auf diese Weise verzweigte α-Glucane. Auch Borovsky et al. (Eur. J. Biochem. 59 (1975), 615-625) untersuchten die de-novo-Synthese von α-1,6-verzweigten α-1,4-Glucanen aus Glucose-1-phosphat unter Verwendung eines Verzweigungsenzyms aus Kartoffel in Kombination mit einer Phosphorylase (1,4-α-D-glucan: orthophosphat α-glycosyltransferase [EC 2.4.1.1]) aus Mais. Doi (Biochimica et Biophysica Acta 184 (1969), 477-485) zeigte, daß die Enzymkombination einer Stärkesynthase (ADP-D-Glucose: α-1,4-Glucan α-4-Glucosyltransferase) aus Spinat und einem Verzweigungsenzym aus Kartoffel unter Einsatz des Substrats ADP-Glucose zu amylopektinähnlichen Produkten führt. Parodi et al. (Arch. Biochem. Biophys. 132 (1969), 11-117) verwendeten für die de-novo-Synthese verzweigter Glucane aus UDP-Glucose eine Glycogensynthase aus Rattenleber kombiniert mit einem Verzweigungsenzym aus Rattenleber. Sie erhielten ein Polymer, das nativem Glycogen ähnelt und das sich von den auf Glucose-1-phosphat basierenden Polymeren unterscheidet.
Auch diese zweite Gruppe von in-vitro-Verfahren weist den Nachteil auf, daß die Substrate, z. B. Glucose-1-Phosphat, UDP-Glucose und ADP-Glucose, sehr teuer sind. Ferner erscheint auch hier eine gezielte Steuerung des Verzweigungsgrades nicht möglich zu sein.

Büttcher et al. (J. Bacteriol. 179 (1997), 3324-3330) beschreiben ein in-vitro-Verfahren zur Herstellung wasserunlöslicher α-1,4-Glucane unter Verwendung einer Amylosucrase und Saccharose als Substrat. Es werden hierbei jedoch nur lineare α-1,4-Glucane ohne Verzweigungen synthetisiert.

Der vorliegenden Erfindung liegt somit die Aufgabe zugrunde, ein Verfahren zur Verfügung zu stellen, das die kostengünstige Herstellung von α-1,6-verzweigten α-1,4-Glucanen für industrielle Zwecke ermöglicht, sowie Nucleinsäuremoleküle bereitzustellen, die in solchen Verfahren einsetzbare Enzyme, insbesondere Verzweigungsenzyme, codieren.

Diese Aufgabe wird durch die in den Ansprüchen bezeichneten Ausführungsformen gelöst.

Somit betrifft die vorliegende Erfindung Nucleinsäuremoleküle codierend ein Verzweigungsenzym (EC 2.4.1.18) aus Bakterien der Gattung Neisseria ausgewählt aus der Gruppe bestehend aus

(a) Nucleinsäuremolekülen, die ein Protein codieren, das die in SEQ ID NO:2 dargestellte Aminosäuresequenz umfaßt;

(b) Nucleinsäuremolekülen, die die in SEQ ID NO:1 dargestellte Nucleotidsequenz der codierenden Region umfassen;

(c) Nucleinsäuremolekülen, die ein Protein codieren, das die Aminosäuresequenz umfaßt, die von der Insertion des Plasmids DSM 12425 codiert wird;

(d) Nucleinsäuremolekülen, die die ein Verzweigungsenzym aus Neisseria denitrificans codierende Region der Insertion des Plasmids DSM 12425 umfassen;

(e) Nucleinsäuremolekülen, die ein Protein codieren, dessen Sequenz im Bereich der ersten 100 Aminosäuren eine Sequenzidentität von mindestens 65% zu der unter SEQ ID NO: 2 angegebenen Sequenz aufweist;

(f) Nucleinsäuremoleküle, deren komplementärer Strang mit einem Nucleinsäuremolekül nach (a), (b), (c) , (d) und/ oder (e) hybridisiert und die ein Verzweigungsenzym aus einem Bakterium der Gattung Neisseria codieren; und

(g) Nucleinsäuremolekülen, deren Nucleotidsequenz von der Sequenz eines Nucleinsäuremoleküls nach (f) aufgrund der Degeneration des genetischen Codes abweicht.

Die in SEQ ID NO:1 dargestellte Nucleinsäuresequenz ist eine genomische Sequenz, die eine codierende Region für ein Verzweigungsenzym aus Neisseria denitrificans umfaßt. Ein Plasmid enthaltend diese DNA-Sequenz wurde hinterlegt als DSM 12425. Mit Hilfe dieser Sequenz bzw. dieses Moleküls ist es dem Fachmann nun möglich, homologe Sequenzen aus anderen Neisseria-Arten oder -Stämmen zu isolieren. Dies kann beispielsweise mit Hilfe konventioneller Methoden, wie dem Durchmustern von cDNA oder genomischen Banken mit geeigneten Hybridisierungsproben erfolgen. Die Isolierung homologer Sequenzen kann auch wie in Beispiel 1 beschrieben erfolgen. Auf diese Weise ist es beispielsweise möglich, Nucleinsäuremoleküle zu identifizieren und isolieren, die mit der unter SEQ ID NO: 1 angegebenen Sequenz hybridisieren und die ein Veraweigungsenzym codieren.
Die erfindungsgemäßen Nucleinsäuremoleküle können prinzipiell ein Verzweigungsenzym aus jedem beliebigen Bakterium der Gattung Neisseria codieren, vorzugsweise codieren sie ein Verzweioungsenzym aus Neisseria denitrificans.

Der Begriff "Hybridisierung" bedeutet im Rahmen der vorliegenden Erfindung eine Hybridisierung unter konventionellen Hybridisierungsbedingungen, vorzugsweise unter stringenten Bedingungen, wie sie beispielsweise in Sambrock et al., Molecular Cloning, A Laboratory Manual, 2. Aufl. (1989) Cold Spring Harbor Laboratory Press, Cold Spring Harbor, NY) beschrieben sind. Besonders bevorzugt bedeutet "Hybridisierung" eine Hybridisierung unter den folgenden Bedingungen:

| | |
|---|---|
| Hybridisierungspuffer: | 2xSSC; 10xDenhardt-Lösung (Fikoll 400+PEG+BSA; Verhältnis 1:1:1); 0,1% SDS; 5 mM EDTA; 50 mM $Na_2HPO_4$; 250 μg/ml Heringssperma DNA; 50 μg/ml tRNA; oder 25 M Natriumphoshphatpuffer pH 7,2; 1 mM EDTA; 7% SDS |
| Hybridisierungstemperatur: | T=65 bis 68°C |
| Waschpuffer: | 0,2xSSC; 0,1% SDS |
| Waschtemperatur: | T=65 bis 68°C. |

Nucleinsäuremoleküle, die mit den erfindungsgemäßen Nucleinsäuremolekülen hybridisieren, können prinzipiell aus jedem beliebigen Bakterium der Gattung Neisseria stammen, der ein entsprechendes Protein exprimiert, vorzugsweise stammen sie aus Neisseria denitrificans. Nucleinsäuremoleküle, die mit den erfindungsgemäßen Molekülen hybridisieren, können z.B. aus genomischen oder aus cDNA-Bibliotheken isoliert werden. Die Identifizierung und Isolierung derartiger Nucleinsäuremoleküle kann dabei unter Verwendung der erfindungsgemäßen Nucleinsäuremoleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY) oder durch Amplifikation mittels PCR.
Als Hybridisierungsprobe können z.B. Nucleinsäuremoleküle verwendet werden, die exakt die oder im wesentlichen die unter SEQ ID NO:1 angegebene Nucleotidsequenz oder Teile dieser Sequenz aufweisen. Bei den als Hybridisierungsprobe verwendeten Fragmenten kann es sich auch um synthetische Fragmente handeln, die mit Hilfe der gängigen Synthesetechniken hergestellt wurden und deren Sequenz im wesentlichen mit der eines erfindungsgemäßen Nucleinsäuremoleküls übereinstimmt. Hat man Gene identifiziert und isoliert, die mit den erfindungsgemäßen Nucleinsäuresequenzen hybridisieren, sollte eine Bestimmung der Sequenz und eine Analyse der Eigenschaften der von dieser Sequenz codierten Proteine erfolgen, um festzustellen, ob es sich um ein Verzweigungsenzym handelt. Hierzu eignen sich insbesondere Homologievergleiche auf der Ebene der Nucleinsäure- oder Aminosäuresequenz sowie die Bestimmung der enzymatischen Aktivität.
Die mit den erfindungsgemäßen Nucleinsäuremolekülen hybridisierenden Moleküle umfassen insbesondere Fragmente, Derivate und allelische Varianten der oben beschriebenen Nucleinsäuremoleküle, die ein Verzweigungsenzym aus Bakterien der Gattung Neisseria, vorzugsweise aus Neisseria denitrificans codieren. Der Ausdruck Derivat bedeutet in diesem Zusammenhang, daß die Sequenzen dieser Moleküle sich von den Sequenzen der oben beschriebenen Nucleinsäuremoleküle an einer oder mehreren Positionen unterscheiden und einen hohen Grad an Homologie zu diesen Sequenzen aufweisen. Homologie bedeutet dabei eine Sequenzidentität über die gesamte Länge von mindestens 60 %, insbesondere eine Identität von mindestens 70 %, vorzugsweise über 80 %, besonders bevorzugt über 90 % und insbesondere von mindestens 95 %. Die Abweichungen zu den oben beschriebenen Nucleinsäuremolekülen können dabei z.B. durch Deletion, Addition, Substitution, Insertion oder Rekombination entstanden sein.

Homologie bedeutet ferner, daß funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Neisseria-Arten oder -Stämmen oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.

Die von den verschiedenen Varianten der erfindungsgemäßen Nucleinsäuremoleküle codierten Proteine weisen bestimmte gemeinsame Charakteristika auf. Dazu können z.B. biologische Aktivität, Molekulargewicht, immunologische Reaktivität, Konformation etc. gehören, sowie physikalische Eigenschaften wie z.B. das Laufverhalten in Gelelektrophoresen, chromatographisches Verhalten, Sedimentationskoeffizienten, Löslichkeit, spektroskopische Eigenschaften, Stabilität; pH-Optimum, Temperatur-Optimum etc.

Das von der Aminosäuresequenz abgeleitete Molekulargewicht des Verzweigungsenzym aus Neisseria denitrificans beträgt 86,3 kDa. Das abgeleitete Molekulargewicht eines erfindungsgemäßen Proteins liegt daher vorzugsweise im Bereich von 70 kDa bis 100 kDa, bevorzugt im Bereich von 77 kDa bis 95 kDa und besonders bevorzugt bei ca. 86 kDa.

Die vorliegende Erfindung betrifft auch Nucleinsäuremoleküle, die ein Protein mit der enzymatischen Aktivität eines Verzweigungsenzyms codieren, wobei das codierte Protein im Bereich des N-Terminus, vorzugsweise in den ersten 100 Aminosäuren, bevorzugt in den ersten 110 Aminosäuren und besonders bevorzugt in der ersten 120 Aminosäuren eine Homologie von mindestens 65% bevorzugt von mindestens 80% und besonders bevorzugt von mindestens 95% zu der unter SEQ ID NO:2 angegebenen Aminosäuresequenz aufweist.

In einer weiteren Ausführungsform betrifft die vorliegende Anmeldung Nucleinsäuremoleküle codierend ein Protein mit Verzweigungsenzymaktivität das, mindestens eines, bevorzugt mindestens 5, insbesondere mindestens 10 und besonders bevorzugt mindestens 20 der folgenden Peptidmotive umfaßt:

(a) MNRNRHI (SEQ ID NO:8),
(b) RPDAHH (SEQ ID NO:9),
(c) HAPDYAL (SEQ ID NO;10),
(d) EGEAA (SEQ ID NO:11),
(e) DDYRF (SEQ ID NO:12),
(f) SALQH (SEQ ID NO:13),
(g) YETLG (SEQ ID NO:14),
(h) VSGVR (SEQ ID NO:15),
(i) VSVIG (SEQ ID NO:16),
(j) FNGWD (SEQ ID NO:17),
(k) LYKFS (SEQ ID NO:18),
(1) PYAFG (SEQ ID NO:19),
(m) RPTTAS (SEQ ID NO:20),
(n) FRRRA (SEQ ID NO:21),
(o) DELVNY (SEQ ID NO:22),
(p) LPLSEY (SEQ ID NO:23),
(q) YQATGL (SEQ ID NO:24).
(r) DDHGL (SEQ ID NO:25),
(s) HQDWN (SEQ ID NO:26),
(t) DGIRV (SEQ ID NO:27),
(u) YGGSEN (SEQ ID NO:28),
(v) SFAEES (SEQ ID NO:29),
(w) DPVHR (SEQ ID NO:30),
(x) WQQFAN (SEQ ID NO:31),
(y) EILNS (SEQ ID NO:32),

(z) ATEIQTAL (SEQ ID NO:33),
(aa) VKDKQAKAK (SEQ ID NO:34).

Die erfindungsgemäßen Nucleinsäuremoleküle können beliebige Nucleinsäuremoleküle sein, insbesondere DNA- oder RNA-Moleküle, beispielsweise cDNA, genomische DNA, mRNA etc. Sie können natürlich vorkommende Moleküle sein, oder durch gentechnische oder chemische Syntheseverfahren hergestellte Moleküle. Sie können einzelsträngige Moleküle sein, die entweder den codierenden oder den nicht codierenden Strang enthalten, oder doppelsträngige Moleküle.

Ferner betrifft die vorliegende Offenbarung Nucleinsäuremoleküle von mindestens 15, vorzugsweise mehr als 50 und besonders bevorzugt mehr als 200 Nucleotiden Länge, die spezifisch mit mindestens einem erfindungsgemäßen Nucleinsäuremolekül hybridisieren. Spezifisch hybridisieren bedeutet hierbei, daß diese Moleküle mit Nucleinsäuremolekülen hybridisieren, die ein erfindungsgemäßes Protein codieren, jedoch nicht mit Nucleinsäuremolekülen, die andere Proteine codieren. Hybridisieren bedeutet dabei vorzugsweise Hybridisieren unter stringenten Bedingungen (s.o.). Insbesondere betrifft die Offenbarung solche Nucleinsäuremoleküle, die mit Transkripten von erfindungsgemäßen Nucleinsäuremolekülen hybridisieren und dadurch deren Translation verhindern können. Solche Nucleinsäuremoleküle, die spezifisch mit den erfindungsgemäßen Nucleinsäuremolekülen hybridisieren, können beispielsweise Bestandteile von antisense-Konstrukten oder Ribozymen sein oder können als Primer für die Amplifikation mittels PCR verwendet werden.

Weiterhin betrifft die Erfindung Vektoren, insbesondere Plasmide, Cosmide, Viren, Bacteriophagen und andere in der Gentechnik gängige Vektoren, die die oben beschriebenen erfindungsgemäßen Nucleinsäuremoleküle enthalten.

In einer bevorzugten Ausführungsform sind die in den Vektoren enthaltenen Nucleinsäuremoleküle in sense Orientierung verknüpft mit regulatorischen Elementen, die die Expression in prokaryontischen oder eukaryontischen Zellen gewährleisten. Der Begriff "Expression" kann dabei Transkription als auch Transkription und Translation bedeuten.

Die Expression der erfindungsgemäßen Nucleinsäuremoleküle in prokaryontischen Zellen, beispielsweise in *Escherichia coli,* ermöglicht beispielsweise eine genauere Charakterisierung der enzymatischen Aktivitäten der codierten Proteine. Darüber hinaus ist es möglich, mittels gängiger molekularbiologischer Techniken (siehe z.B. Sambrook et al., a.a.O.) verschiedenartige Mutationen in die erfindungsgemäßen Nucleinsäuremoleküle einzuführen, wodurch es zur Synthese von Proteinen mit eventuell veränderten biologischen Eigenschaften kommt. Möglich ist die Erzeugung von Deletionsmutanten, bei denen durch fortschreitende Deletionen vom 5'- oder vom 3'-Ende der codierenden DNA-Sequenz Nucleinsäuremoleküle erzeugt werden, die zur Synthese entsprechend verkürzter Proteine führen. Möglich ist auch die Einführung von Punktmutationen an Positionen, die einen Einfluß beispielsweise auf die Enzymaktivität oder die Regulierung des Enzyms haben. Auf diese Weise können z.B. Mutanten hergestellt werden, die einen veränderten $K_m$-Wert besitzen oder nicht mehr den normalerweise in der Zelle vorliegenden Regulationsmechanismen über allosterische Regulation oder kovalente Modifizierung unterliegen. Des weiteren können Mutanten hergestellt werden, die eine veränderte Substrat- oder Produktspezifität aufweisen. Weiterhin können Mutanten hergestellt werden, die ein verändertes Aktivitäts-Temperatur-Profil aufweisen. Die gentechnische Manipulation in prokaryontischen Zellen können nach dem Fachmann bekannten Methoden erfolgen (vgl. Sambrook et al., a.a.O.).

Regulatorische Sequenzen zur Expression in prokaryontischen Organismen, z.B. E. coli, und in eukaryontischen Organismen sind ausreichend in der Literatur beschrieben, insbesondere solche zur Expression in Hefe, wie z. B. Saccharomyces cerevisiae. Eine Übersicht verschiedener Systeme zur Expression für Proteine in verschiedenen Wirtsorganismen findet man z. B. in Methods in Enzymology 153 (1987), 383-516 und in Bitter et al. (Methods in Enzymology 153 (1987), 516-544).

Vorzugsweise ist das in einem erfindungsgemäßen Vektor insertierte erfindungsgemäße Nucleinsäuremolekül derart modifiziert, daß das codierte Protein nach Expression in einem geeigneten Wirtsorganismus leichter aus dem Kulturmedium isoliert werden kann. So besteht beispielsweise die Möglichkeit, das codierte Verzweigungssystem als Fusionsprotein mit einer weiteren Polypeptidsequenz zu exprimieren, deren spezifische Bindungseigenschaften die Isolierung des Fusionsproteins über Affinitätschromatographie erlauben (siehe z. B. Chong et al., Gene 192 (1997), 271-281; Hopp et al., Bio/Technology 6 (1988), 1204-1210; Sassenfeld, Trends Biotechnol. 8 (1990), 88-93).
Weiterhin ist bevorzugt, daß das in einem erfindungsgemäßen Vektor enthaltene Nucleinsäuremolekül Nucleotidsequenzen umfaßt, die die Sekretion des Verzweigungsenzyms in das Kulturmedium erlauben. Vorzugsweise wird eine Sequenz verwendet, die das Signalpeptid der $\alpha$-CGTase aus Klebsiella oxytoca M5A1 codiert (Fiedler et al., J. Mol. Biol. 256 (1996), 279-291; Genebank Acc. No. X86014, CDS 11529-11618). Durch die Sekretion des Enzyms in das Kulturmedium wird die Gewinnung und Aufreinigung vereinfacht. Es wird ein Aufschluß der Zellen vermieden und das Enzym kann aus dem Kulturmedium gewonnen werden, wobei zur Entfernung von Restbestandteilen des Kulturmediums gängige Methoden, wie. z. B. Dialyse, Osmose, chromatographische Methoden etc., eingesetzt werden können. Weiterhin können die erfindungsgemäßen Vektoren weitere Funktionseinheiten umfassen, die eine Stabilisierung des Vektors in einem Wirtsorganismus bewirken, wie z. B. einen bakteriellen Replikationsursprung oder die 2$\mu$-DNA zur Stabilisierung in S. cerevisiae.

In einer weiteren Ausführungsform betrifft die Erfindung Wirtszellen, insbesondere prokaryontische oder eukaryontische Zellen, die mit einem oben beschriebenen Vektor transformiert wurden, sowie Zellen, die von derartigen Wirtszellen abstammen und die beschriebenen Vektoren enthalten. Die Wirtszellen können Bakterien-, (z.B. *E. coli*) oder Pilzzellen (z.B. Hefe, insbesondere *S. cerevisiae*), sowie pflanzliche oder tierische Zellen sein. Der Begriff "transformiert" bedeutet dabei, daß die erfindungsgemäßen Zellen mit einem erfindungsgemäßen Nucleinsäuremolekül genetisch modifiziert sind insofern, als sie zusätzlich zu ihrem natürlichen Genom mindestens ein erfindungsgemäßes Nucleinsäuremolekül enthalten. Dieses kann in der Zelle frei, gegebenenfalls als selbstreplizierendes Molekül, vorliegen oder es kann stabil in das Genom der Wirtszelle integriert vorliegen.
Vorzugsweise sind die Wirtszellen Mikroorganismen. Darunter werden im Rahmen der vorliegenden Anmeldung alle Bakterien und alle Protisten (z. B. Pilze, insbesondere Hefen und Algen) verstanden, so wie sie z. B. in Schlegel "Allgemeine Mikrobiologie" (Georg Thieme Verlag (1985), 1-2) definiert sind.

Besonders bevorzugt sind die erfindungsgemäßen Wirtszellen Pflanzenzellen. Dabei kann es sich prinzipiell um Pflanzenzellen aus jeder beliebigen Pflanzenspezies handeln, d. h. sowohl monokotyle als auch dikotyle Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Vorzugsweise betrifft die Erfindung Pflanzenzellen aus faserbildenden (z. B. Flachs, Hanf, Baumwolle), ölspeichernden (z. B. Raps, Sonnenblume, Sojabohne), zuckerspeichernden (z. B. Zuckerrübe, Zuckerrohr, Zuckerhirse, Banane) und proteinspeichernden Pflanzen (z. B. Leguminosen).
In einer weiteren Ausführungsform betrifft die Erfindung Pflanzenzellen aus Futterpflanzen (z. B. Futter- und Weidegräser (Alfalfa, Klee etc.)), Gemüsepflanzen (z. B. Tomate, Salat, Chicorée).
In einer bevorzugten Ausführungsform betrifft die Erfindung Pflanzenzellen aus stärkespeichernden Pflanzen (z.B. Weizen, Gerste, Hafer, Roggen, Kartoffel, Mais, Reis, Erbse, Maniok, Mungbohne), besonders bevorzugt sind Pflanzenzellen aus Mais-, Reis-, Weizen- und Kartoffelpflanzen.

Ferner betrifft die vorliegende Erfindung Verfahren zur Herstellung eines Verzweigungsenzyms aus Bakterien der Gattung Neisseria, bei dem erfindungsgemäße Wirtszellen unter Bedingungen kultiviert werden, die die Expression des Proteins erlauben, und das Protein aus der Kultur, d.h. aus den Zellen und/oder dem Kulturmedium gewonnen wird. Vorzugsweise wird dabei ein Wirtsorganismus verwendet, der das Verzweigungsenzym sekretiert.

Ferner betrifft die vorliegende Erfindung ein Verfahren zur Herstellung eines Verzweigungsenzyms aus Bakterien der Gattung Neisseria, wobei das Protein in einem in-vitro-Transkriptions- und -Translationssystem unter Verwendung eines erfindungsgemäßen Nucleinsäuremoleküls hergestellt wird. Solche Systeme sind dem Fachmann geläufig.

Die Erfindung betrifft auch Proteine, die durch die erfindungsgemäßen Nucleinsäuremoleküle codiert werden, oder die durch ein erfindungsgemäßes Verfahren erhältlich sind.

Die vorliegende Erfindung betrifft weiterhin Antikörper, die spezifisch ein erfindungsgemäßes Protein erkennen. Diese können z.B. monoclonale oder polyclonale Antikörper sein. Es können auch Fragmente von Antikörpern sein, die erfindungsgemäße Proteine erkennen. Methoden zur Herstellung derartiger Antikörper bzw. Fragmente sind dem Fachmann geläufig.

Ferner betrifft die vorliegende Erfindung die Verwendung eines erfindungsgemäßen Verzweigungsenzyms zur Herstellung von $\alpha$-1,6-verzweigten $\alpha$-1,4-Glucanen in in-vitro Systemen.

Die vorliegende Erfindung betrifft insbesondere auch transgene Pflanzenzellen, die die erfindungsgemäßen Nucleinsäuremoleküle oder Vektoren enthalten. Vorzugsweise sind die erfindungsgemäßen Zellen **dadurch gekennzeichnet, daß** das eingeführte erfindungsgemäße Nucleinsäuremolekül stabil in das Genom integriert ist und unter der Kontrolle eines in pflanzlichen Zellen aktiven Promotors steht.

Für die Expression eines erfindungsgemäßen Nucleinsäuremoleküls in pflanzlichen Zellen stehen eine Vielzahl von Promotoren bzw. regulatorischen Elementen zur Verfügung. Regulatorische Elemente für die Expression in pflanzlichen Zellen sind dabei prinzipiell alle in pflanzlichen Zellen aktiven Promotoren, Enhancer, Terminatoren, etc. Im Prinzip kann jeder in den für die Transformation gewählten Pflanzen funktionale Promotor verwendet werden. Der Promotor kann homolog oder heterolog in bezug auf die verwendete Pflanzenspezies sein. Er kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. Geeignet ist beispielsweise der 35S-Promotor des Cauliflower-Mosaik-Virus (Odell et al., Nature 313 (1985), 810-812 oder US 5 352 605), der eine konstitutive Expression in allen Geweben einer Pflanze gewährleistet und das in der WO/9401571 beschriebene Promotorkonstrukt. Ein anderes Beispiel ist der Ubiquitinpromoter (siehe z.B. US 5 614 399) sowie die Promotoren der Polyubiquitingene aus Mais (Christensen et al., a.a.O.). Es können jedoch auch Promotoren verwendet werden, die nur zu einem durch äußere Einflüsse determinierten Zeitpunkt aktiviert werden (siehe beispielsweise WO/9307279). Von besonderem Interesse können hierbei Promotoren von heat-shock-Proteinen sein, die eine einfache Induktion erlauben. Ferner können die Promotoren verwendet werden, die in einem bestimmten Gewebe der Pflanze zu einer Expression nachgeschalteter Sequenzen führen, beispielsweise in photosynthetisch aktivem Gewebe. Beispiele hierfür sind der ST-LS1-Promotor (Stockhaus et al., Proc. Natl. Acad. Sci. USA 84 (1987), 7943-7947; Stockhaus et al., EMBO J. 8 (1989), 2445-2451), der Ca/b-Promotor (siehe z.B. US 5 656 496, US 5 639 952, Bansal et al., Proc. Natl. Acad. Sci. USA 89 (1992), 3654-3658) und der Rubisco SSU-Promotor (siehe z.B. US 5 034 322 und US 4 962 028). Zu erwähnen sind ferner Promotoren, die in den stärkespeichernden Organen von zu transformierenden Pflanzen aktiv sind. Dies sind z.B. bei Mais die Maiskörner, während es bei der Kartoffel die Knollen sind. Zur Überexpression der erfindungsgemäßen Nucleinsäuremoleküle in der Kartoffel kann beispielsweise der knollenspezifische Patatigen-Promotor B33 (Rocha-Sosa et al., EMBO J. 8 (1989), 23-29) verwendet werden. Samenspezifische Promotoren sind bereits für verschiedene Pflanzenspezies beschrieben worden. So z.B. der USP-Promotor aus Vicia faba, der eine samenspezifische Expression in V. faba und anderen Pflanzen gewährleistet (Fiedler et al., Plant Mol. Biol. 22 (1993), 669-679; Bäumlein et al., Mol Gen. Genet. 225 (1991), 459-467).

Ferner können auch fruchtspezifische Promotoren eingesetzt werden, wie z.B. beschrieben in der WO 9101373. Besonders bevorzugt werden Promotoren für eine endosperm-spezifische Expression verwendet, wie z. B. der Glutelinpromotor (Leisy et al., Plant Mol. Biol. 14 (1990), 41-50; Zheng et al., Plant J. 4 (1993), 357-366), der HMG-Promotor aus Weizen, der USP-Promotor, der Phaseolinpromotor oder Promotoren von Zein-Genen aus Mais (Pedersen et al., Cell 29 (1982), 1015-1026; Quatroccio et al., Plant Mol. Biol. 15 (1990), 81-93). Mit Hilfe endospermspezifischer Promotoren ist es möglich, die Transkriptmenge der erfindungsgemäßen Nucleinsäuremoleküle im Endosperm im Vergleich zum Endosperm entsprechender Wildtyp-Pflanzen zu erhöhen.

Besonders bevorzugt wird der shrunken-1-Promotor (sh-1) aus Mais (Werr et al., EMBO J. 4 (1985), 1373-1380) verwendet.

Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z. B. Gielen et al., EMBO J. 8 (1989), 23-29) und sind beliebig austauschbar.

Möglich ist somit die Expression der erfindungsgemäßen Nucleinsäuremoleküle in pflanzlichen Zellen.

Somit betrifft die vorliegende Erfindung ebenfalls ein Verfahren zur Herstellung transgener Pflanzenzellen, umfassend die Einführung eines erfindungsgemäßen Nucleinsäuremoleküls oder Vektors in Pflanzenzellen. Verschiedene Pflanzentransformationssysteme stehen dabei dem Fachmann zur Verfügung, z. B. ist die Verwendung von T-DNA für die Transformation von Pflanzenzellen intensiv untersucht und beschrieben in EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46 und An, EMBO J. 4 (1985), 277-287.

Für den Transfer der DNA in die Pflanzenzelle können Pflanzen-Explantate zweckmäßigerweise mit Agrobacterium tumefaciens oder Agrobacterium rhizogenes cokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z.B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).

Alternative Systeme zur Transformation von monokotylen Pflanzen sind die Transformation mittels des biolistischen Ansatzes, die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimenden Pollen und die DNA-Aufnahme in Embryonen durch Quellung (siehe z. B. Lusardi, Plant J. 5 (1994), 571-582; Paszkowski, Biotechnology 24 (1992), 387-392).

Während die Transformation dikotyler Pflanzen über Ti-Plasmid-Vektorsysteme mit Hilfe von Agrobacterium tumefaciens wohl etabliert ist, weisen neuere Arbeiten darauf hin, daß auch monokotyle Pflanzen der Transformation mittels auf Agrobacterium basierender Vektoren sehr wohl zugänglich sind (Chan, Plant Mol. Biol. 22 (1993), 491-506; Hiei, Plant J. 6 (1994), 271-282; Bytebier, Proc. Natl. Acad. Sci. USA 84 (1987), 5345 - 5349; Raineri, Bio/Technology 8 (1990), 33 - 38; Gould, Plant Physiol. 95 (1991), 426 - 434; Mooney, Plant, Cell Tiss. & Org. Cult. 25 (1991), 209 - 218; Li, Plant Mol. Biol. 20 (1992), 1037 - 1048).

Drei der oben genannten Transformationssysteme konnten in der Vergangenheit für verschiedene Getreide etabliert werden: die Elektroporation von Gewebe, die Transformation von Protoplasten und der DNA-Transfer durch Partikel-Beschuß in regenerierbare Gewebe und Zellen (zur Übersicht: Jähne, Euphytica 85 (1995), 35 - 44). Die Transformation von Weizen wird in der Literatur verschiedentlich beschrieben (zur Übersicht: Maheshwari, Critical Reviews in Plant Science 14 (2) (1995), 149 - 178).

Insbesondere die Transformation von Mais wurde in der Literatur mehrfach beschrieben (vgl. z. B. WO 95/06128, EP 0513849, EO 0465875, EP 292435; Fromm et al., Biotechnology 8 (1990), 833-844; Gordon-Kamm et al., Plant Cell 2 (1990), 603-618; Koziel et al., Biotechnology 11 (1993), 194-200; Moroc et al., Theor. Appl. Genet. 80 (1990), 721-726). Auch die erfolgreiche Transformation anderer Getreidearten wurde bereits beschrieben, z. B. für Gerste (Wan und Lemaux, a.a.O.; Ritala et al., a.a.O.; Krens et al., Nature 296 (1982), 72-74) und für Weizen (Nehra et al., Plant J. 5 (1994), 285-297).

Bei der Expression der erfindungsgemäßen Nucleinsäuremoleküle in Pflanzen besteht grundsätzlich die Möglichkeit, daß das synthetisierte Protein in jedem beliebigen Kompartiment der pflanzlichen Zelle lokalisiert sein kann. Um die Lokalisation in einem bestimmten Kompartiment zu erreichen, muß die codierende Region gegebenenfalls mit DNA-Sequenzen verknüpft werden, die die Lokalisierung in dem jeweiligen Kompartiment gewährleisten. Derartige Sequenzen sind bekannt (siehe beispielsweise Braun, EMBO J. 11 (1992), 3219-3227; Sonnewald, Plant J. 1 (1991), 95-106; Rocha-Sosa, EMBO J. 8 (1989), 23-29).

Als plastidäre Signalsequenz kann beispielsweise die der Ferrodoxin:NADP+ oxidoreductase (FNR) aus Spinat verwendet werden. Diese Sequenz enthält den 5' nichttranslatierten Bereich sowie die flankierende Transitpeptidsequenz der cDNA des plastidären Proteins Ferrodoxin:NADP+ oxidoreductase aus Spinat (Nucleotid -171 bis + 165; Jansen et al., Current Genetics 13 (1988), 517-522).

Ferner kann als plastidäre Signalsequenz beispielsweise das Transitpeptid des waxy-Proteins aus Mais plus die ersten 34 Aminosäuren des maturen waxy-Proteins (Klösgen et al., Mol Gen Genet. 217 (1989), 155-161) verwendet werden. Darüber hinaus kann das Transitpeptid des waxy-Proteins aus Mais (s. o.) auch ohne die ersten 34 Aminosäuren des maturen waxy-Proteins verwendet werden.

Ferner ist die Verwendung folgender plastidärer Signalsequenzen denkbar: Signalssequenz der Ribulose bisphosphate carboxylase small subunit (Wolter et al., Proc. Natl. Acad. Sci. USA 85 (1988), 846-850; Nawrath et al., Proc. Natl. Acad. Sci. USA 91 (1994), 12760-12764); Signalsequenz der NADP-malate dehydrogenase (Gallardo et al., Planta 197 (1995), 324-332); Signalsequenz der Glutathion-Reduktase (Creissen et al., Plant J. 8 (1995), 167-175).

Die vorliegende Erfindung betrifft somit auch transgene Pflanzenzellen, die mit einem oder mehreren erfindungsgemäßen Nucleinsäuremolekül(en) transformiert wurden, sowie transgene Pflanzenzellen, die von derartig transformierten Zellen abstammen. Derartige Zellen enthalten ein oder mehrere erfindungsgemäße(s) Nucleinsäuremolekül (e), wobei diese(s) vorzugsweise mit regulatorischen DNA-Elementen verknüpft ist/sind, die die Transkription in pflanzlichen Zellen gewährleisten, insbesondere mit einem Promotor. Derartige Zellen lassen sich von natürlicherweise vorkommenden Pflanzenzellen dadurch unterscheiden, daß sie mindestens ein erfindungsgemäßes Nucleinsäuremolekül enthalten.

Die transgenen Pflanzenzellen können nach dem Fachmann bekannten Techniken zu ganzen Pflanzen regeneriert werden. Die durch Regeneration der erfindungsgemäßen transgenen Pflanzenzellen erhältlichen Pflanzen sind ebenfalls Gegenstand der vorliegenden Erfindung.

Ferner sind Gegenstand der Erfindung Pflanzen, die die oben beschriebenen erfindungsgemäßen Pflanzenzellen enthalten. Bei den erfindungsgemäßen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, d.h. Pflanzen, die vom Menschen kultiviert werden für Zwecke der Ernährung oder für technische, insbesondere industrielle Zwecke. Vorzugsweise betrifft die Erfindung Pflanzenzellen aus faserbildenden (z. B. Flachs, Hanf, Baumwolle), ölspeichernden (z. B. Raps, Sonnenblume, Sojabohne), zuckerspeichernden (z. B. Zuckerrübe, Zuckerrohr, Zuckerhirse, Banane) und proteinspeichernden Pflanzen (z. B. Leguminosen).

In einer weiteren Ausführungsform betrifft die Erfindung Futterpflanzen (z. B. Futter- und Weidegräser (Alfalfa, Klee etc.)), Gemüsepflanzen (z.B. Tomate, Salat, Chicorée).

In einer bevorzugten Ausführungsform betrifft die Erfindung stärkespeichernde Pflanzen (z. B. Weizen, Gerste, Hafer, Roggen, Kartoffel, Mais, Reis, Erbse, Maniok, Mungbohne), insbesondere bevorzugt sind Mais-, Reis-, Weizen- und Kartoffelpflanzen.

In einer bevorzugten Ausführungsform weisen Zellen der erfindungsgemäßen Pflanzen im Vergleich zu entsprechenden nicht genetisch modifizierten Pflanzenzellen von Wildtyp-Pflanzen eine erhöhte Aktivität eines erfindungsgemäßen Proteins auf. Dies sind bevorzugt Zellen stärkespeichernder Gewebe, insbesondere Zellen aus Knollen oder des Endosperms, besonders bevorzugt Zellen aus Kartoffelknollen oder des Endosperms von Mais-, Weizen-oder Reispflanzen.

Der Begriff "Erhöhung der Aktivität" bedeutet dabei im Rahmen der vorliegenden Erfindung eine Erhöhung der Expression eines erfindungsgemäßen Nucleinsäuremoleküls, das für ein Protein mit Verzweigungsenzymaktivität codiert, eine Erhöhung der Menge an Protein mit Verzweigungsenzymaktivität und/oder eine Erhöhung der Aktivität eines Proteins mit Verzweigungsenzymaktivität in den Pflanzen.

Die Erhöhung der Expression kann beispielsweise bestimmt werden durch Messung der Menge an für solche Proteine codierenden Transkripten, z. B. durch Northern Blot Analyse oder RT-PCR. Eine Erhöhung bedeutet dabei vorzugsweise eine Erhöhung der Menge an Transkripten im Vergleich zu nicht genetisch modifizierten Pflanzenzellen um mindestens 10 %, bevorzugt um mindestens 20 %, insbesondere um mindestens 50 % und besonders bevorzugt um mindestens 75 %. Die Erhöhung der Menge an Protein mit Verzweigungsenzymaktivität kann beispielsweise bestimmt werden durch Western-Blot Analyse. Eine Erhöhung bedeutet dabei vorzugsweise eine Erhöhung der Menge an Protein mit Verzweigungsenzymaktivität im Vergleich zu entsprechenden nicht genetisch modifizierten Zellen um mindestens 10 %, bevorzugt um mindestens 20 %, insbesondere um mindestens 50 % und besonders bevorzugt um mindestens 75 %.

Die Erhöhung der Aktivität des Verzweigungsenzyms kann beispielsweise bestimmt werden nach der in Lloyd et al. (Biochem. J. 338 (1999), 515 - 521) beschriebenen Methode. Eine Erhöhung bedeuted dabei vorzugsweise eine Erhöhung der Verzweigungsenzymaktivität um mindestens 10 %, bevorzugt um mindestens 20 %, besonders bevorzugt um mindestens 50 % und ganz besonders bevorzugt um mindestens 75 %.

Es wurde überraschenderweise gefunden, daß Pflanzen, die erfindungsgemäße Pflanzenzellen mit erhöhter Aktivität eines erfindungsgemäßen Verzweigungsenzyms enthalten, eine modifizierte Stärke synthetisieren im Vergleich zu entsprechenden nicht genetisch modifizierten Wildtyp-Pflanzen. Die modifizierte Stärke kann beispielsweise in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, dem Viskositätsverhalten, der Stärkekomgröße, der Seitenkettenverteilung und/oder der Stärkekornform im Vergleich zu in Wildtyp-Pflanzen synthetisierter Stärke verändert sein, so daß diese für spezielle Verwendungszwecke besser geeignet ist.

Es wurde ferner überraschenderweise gefunden, daß in erfindungsgemäßen Pflanzenzellen, bei denen die Aktivität des erfindungsgemäßen Verzweigungsenzyms erhöht ist, die Zusammensetzung der Stärke in der Weise verändert ist, daß sie im Vergleich zu Stärke aus entsprechenden Wildtyp-Pflanzen eine erhöhte Gelfestigkeit und/oder einen verringerten Phosphatgehalt und/oder eine verringerte Peakviskosität und/oder eine verringerte Verkleisterungstemperatur und/oder eine verringerte Stärkekorngröße und/oder eine veränderte Seitenkettenverteilung aufweist.

Der Begriff "erhöhte Gelfestigkeit" bedeutet in diesem Zusammenhang eine Erhöhung um mindestens 10 %, bevorzugt um mindestens 50 %, insbesondere um mindestens 100 %, um mindestens 200 % und besonders bevorzugt um mindestens 300 % im Vergleich zur Gelfestigkeit von Stärke aus Wildtyp-Pflanzen. Die Bestimmung der Gelfestigkeit erfolgt dabei wie weiter unten beschrieben.

Der Begriff "verringerter Phosphatgehalt" bedeutet im Zusammenhang mit der vorliegenden Erfindung, daß der Gesamtgehalt an kovalent gebundenem Phosphat und/oder der Gehalt an Phosphat in C-6-Position der in den erfindungsgemäßen Pflanzenzellen synthetisierten Stärke um mindestens 20 %, bevorzugt um mindestens 40 %, besonders bevorzugt um mindestens 60 % und insbesondere um mindestens 80 % im Vergleich zu Stärke aus Pflanzenzellen von entsprechenden Wildtyp-Pflanzen verringert ist.

Die Bestimmung des Gesamt-Phosphatgehalts bzw. des Gehalts an Phosphat in C-6-Position kann nach der unten beschriebenen Methode erfolgen.

Der Begriff "verringerte Peakviskosität" bedeutet im Zusammenhang mit der vorliegenden Erfindung eine Verringerung der Peakviskosität um mindestens 10 %, bevorzugt um mindestens 25 %, insbesondere um mindestens 50 %, besonders bevorzugt um mindestens 75 % im Vergleich zur Peakviskosität von Stärken aus Wildtyp-Pflanzen.

Der Begriff "verringerte Verkleisterungstemperatur" bedeutet im vorliegenden Zusammenhang eine Verringerung der Verkleisterungstemperatur um mindestens 0,5°C, bevorzugt um mindestens 1,0°C, insbesondere um mindestens 2,0°C, besonders bevorzugt um mindestens 3,0°C im Vergleich zur Verkleisterungstemperatur von Stärken aus Wildtyp-Pflanzen.

Die Bestimmung der Peakviskosität und der Verkleisterungstemperatur erfolgt mit Hilfe eines Rapid Visco Analyzers in der unten beschriebenen Weise.

Die Begriffe "Peakviskosität" und "Verkleisterungstemperatur" sind dem Fachmann geläufig.

Der Begriff "verringerte Stärkekomgröße" bedeutet, daß der prozentuale Anteil an Stärkekörnern mit einer Korngröße von bis zu 15 μm im Vergleich zu Wildtyp-Pflanzen um mindestens 10 %, bevorzugt um mindestens 30 %, insbesondere um mindestens 50 %, 100 % und besonders bevorzugt um mindestens 150 % erhöht ist.

Die Bestimmung der Stärkekorngröße erfolgt mit Hilfe eines Fotosedimentometer des Typs "Lumosed" der Firma Retsch GmbH, Deutschland in der unten beschriebenen Weise.

Unter dem Begriff "veränderte Seitenkettenverteilung" soll in diesem Zusammenhang eine Erhöhung des Anteils an Seitenketten mit einem DP von 6 bis 9 um mindestens 25%, bevorzugt um mindestens 50%, insbesondere um mindestens 100% und besonders bevorzugt um mindestens 200% im Vergleich zum Anteil an Seitenketten mit einem DP von 6 bis 9 von Amylopektin aus Wildtyp-Pflanzen verstanden werden.

In einer weiteren Ausführungsform der Erfindung soll unter einer "veränderten Seitenkettenverteilung" eine Erhöhung des Anteils an Seitenketten mit einem DP von 6 bis 8, bevorzugt von 6 bis 7 um mindestens 25 %, bevorzugt um mindestens 50%, insbesondere im mindestens 100% und besonders bevorzugt um mindestens 200% im Vergleich zum Anteil an Seitenketten mit entsprechendem Polymerisationsgrad von Amylopektin aus Wildtyp-Pflanzen verstanden werden.

Die Bestimmung des Anteils an Seitenketten erfolgt über die Bestimmung des prozentualen Anteils einer bestimmten Seitenkette am Gesamtanteil aller Seitenketten. Der Gesamtanteil aller Seitenketten wird ermittelt über die Bestimmung der Gesamtfläche unter den Peaks, die im HPCL-Chromatogramm die Polymerisationsgrade von DP6 bis 30 repräsentieren. Der prozentuale Anteil einer bestimmten Seitenkette am Gesamtanteil aller Seitenketten wird ermittelt über die Bestimmung des Verhältnisses der Fläche unter dem Peak, der diese Seitenkette im HPLC-Chromatogramm repräsentiert zur Gesamtfläche. Vorzugsweise wird dazu das Programm AI-450 Version 3.31 der Firma Dionex, USA, verwendet.

Die vorliegende Offenbarung betrifft eine Stärke, deren Amylopektin im Vergleich zum Amylopektin von Stärken aus Wildtyp-Pflanzen Seitenketten mit einem DP=5 aufweist.

Die vorliegende Erfindung betrifft ferner ein Verfahren zur Herstellung einer transgenen Pflanze, die eine modifizierte Stärke synthetisiert, wobei

(a) eine pflanzliche Zelle genetisch modifiziert wird durch die Einführung eines erfindungsgemäßen Nucleinsäuremoleküls und/oder eines erfindungsgemäßen Vektors, dessen Vorhandensein oder dessen Expression zur Erhöhung der Aktivität eines Proteins mit der Aktivität eines Verzweigungsenzym führt;
(b) aus der gemäß Schritt (a) hergestellten Zelle eine Pflanze regeneriert wird; und
(c) aus der gemäß Schritt (c) erzeugten Pflanze gegebenenfalls weitere Pflanzen erzeugt werden, die Zellen enthalten, die ein erfindungsgemäßes Nucleinsäuremolekül oder einen erfindungsgemäßen Vektor enthalten.

In einer bevorzugten Ausführungsform des Verfahrens ist die Stärke derart modifiziert, als daß sie eine erhöhte Gelfestigkeit und/oder einen verringerten Phosphatgehalt und/oder eine verringerte Peakviskosität und/oder einer verringerten Verkleisterungstemperatur und/oder eine verringerte Stärkekorngröße und/oder eine veränderte Seitenkettenverteilung aufweist im Vergleich zu Stärke aus entsprechenden Wildtyp-Pflanzen.
Die Begriffe "erhöhte Gelfestigkeit"; "verringerter Phosphatgehalt", "verringerte Peakviskosität", "verringerten Verkleisterungstemperatur", "verringerte Stärkekorngröße" und "veränderten Seitenkettenverteilung" werden in diesem Zusammenhang wie bereits oben definiert.

Für die laut Schritt (a) eingeführt genetische Modifikation gilt dasselbe, was bereits oben in anderem Zusammenhang mit den erfindungsgemäßen Pflanzen erläutert wurde.
Die Regeneration von Pflanzen gemäß Schritt (b) kann nach dem Fachmann bekannten Methoden erfolgen.
Die Erzeugung weiterer Pflanzen gemäß Schritt (b) der erfindungsgemäßen Verfahren kann z. B. erfolgen durch vegetative Vermehrung (beispielsweise über Stecklinge, Knollen oder über Calluskulutur und Regeneration ganzer Pflanzen) oder durch sexuelle Vermehrung. Die sexuelle Vermehrung findet dabei vorzugsweise kontrollen statt, d. h. es werden ausgewählte Pflanzen mit bestimmten Eigenschaften miteinander gekreuzt und vermehrt.

Die vorliegende Erfindung betrifft auch die durch die erfindungsgemäßen Verfahren erhältlichen Pflanzen.

Die vorliegende Erfindung betrifft auch Vermehrungsmaterial erfindungsgemäßer Pflanzen sowie der gemäß den erfindungsgemäßen Verfahren hergestellten transgenen Pflanzen. Der Begriff Vermehrungsmaterial umfaßt dabei jene Bestandteile der Pflanze, die geeignet sind zur Erzeugung von Nachkommen auf vegetativem oder generativem Weg. Für die vegetative Vermehrung eignen sich beispielsweise Stecklinge, Calluskulturen, Rhizome oder Knollen. Anderes Vermehrungsmaterial umfaßt beispielsweise Früchte, Samen, Sämlinge, Protoplasten, Zellkulturen etc.. Vorzugsweise handelt es sich bei dem Vermehrungsmaterial um Knollen und Samen.

Die erfindungsgemäßen transgene Pflanzenzellen und Pflanzen synthetisieren aufgrund der Expression eines erfindungsgemäßen Nucleinsäuremoleküls oder eines erfindungsgemäßen Vektors, dessen Vorhandensein oder dessen Expression zur Erhöhung der Aktivität eines Verzweigungsenzyms führt, im Vergleich zu entsprechenden nicht genetisch modifizierten Pflanzenzellen von Wildtyp-Pflanzen, eine Stärke, die in ihren physikalisch-chemischen Eigenschaften, insbesondere der Gelfestigkeit und/oder dem Verkleisterungsverhalten und/oder der Stärkekorngröße und/oder dem Phosphatgehalt und/oder der Seitenkettenverteilung im Vergleich zu in Wildtyp-Pflanzen synthetisierter Stärke verändert ist.

Die Begriffe "erhöhte Gelfestiekeit", "verringerter Phosphatgehalt", "verringerten Peakviskosität", "verringerte Verkleisterungstemperatar", "verringerte Stärkekorngröße" und "veränderte Seitenkettenverteilung" werden in diesem Zusammenhang wie bereits oben definiert.

Die vorliegende Erfindung betrifft ferner ein Verfahren zur Herstellung einer modifizierten Stärke umfassend den Schritt der Extraktion der Stärke aus einer oben beschriebenen erfindungsgemäßen Pflanze(nzelle) und/oder aus stärkespeichernden Teilen einer solchen Pflanze. Vorzugsweise umfaßt ein solches Verfahren auch den Schritt des Emtens der kultivierten Pflanzen und/oder stärkespeichernder Teile dieser Pflanzen vor der Extraktion der Stärke und besonders bevorzugt ferner den Schritt der Kultivierung erfindungsgemäßer Pflanzen vor dem Ernsten. Verfahren zur Extraktion der Stärke von Pflanzen oder von stärkespeichernden Teilen von Pflanzen sind dem Fachmann bekannt. Weiterhin sind Verfahren zur Extraktion der Stärke aus verschiedenen stärkespeichernden Pflanzen beschrieben, z. B. in "Starch: Chemistry and Technology (Hrsg.: Whistler, BeMiller und Paschall (1994), 2. Ausgabe, Academic Press Inc. London Ltd; ISBN 0-12-746270-8; siehe z. B. Kapitel XII, Seite 412-468: Mais und Sorghum-Stärken: Herstellung; von Watson; Kapitel XIII, Seite 469-479: Tapioca-, Arrowroot- und Sagostärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996), 54-57, die Extraktion von Maisstärke im industriellen Maßstab wird in der Regel durch das sogenannte "wet milling" erreicht.)). Verrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

Die von den erfindungsgemäßen Pflanzen, Pflanzenzellen oder Pflanzenteilen erhältlichen Stärken können nach dem Fachmann bekannten Verfahren nachträglich modifiziert werden und eignen sich in unmodifizierter oder modifizierter Form für verschiedene Verwendungen im Nahrungsmittel- oder Nicht-Nahrungsmittelbereich. Grundsätzlich läßt sich die Einsatzmöglichkeit der Stärke in zwei große Bereiche unterteilen. Der eine Bereich umfaßt die Hydrolyseprodukte der Stärke, hauptsächlich Glucose und Glucanbausteine, die über enzymatische oder chemische Verfahren erhalten werden. Sie dienen als Ausgangsstoff für weitere chemische Modifikationen und Prozesse, wie Fermentation. Für eine Reduktion der Kosten kann hierbei die Einfachheit und kostengünstige Ausführung eines Hydrolyseverfahrens von Bedeutung sein. Gegenwärtig verläuft es im Wesentlichen enzymatisch unter Verwendung von Amyloglucosidase. Vorstellbar wäre eine Kosteneinsparung durch einen geringeren Einsatz von Enzymen. Eine Strukturveränderung der Stärke, z. B. Oberflächenvergrößerung des Korns oder eine sterische Struktur, die die Zugänglichkeit für die eingesetzten Enzyme begrenzt, könnte dies bewirken.

Der andere Bereich, in dem die Stärke wegen ihrer polymeren Struktur als sogenannte native Stärke verwendet wird, gliedert sich in zwei weitere Einsatzgebiete:

1. Nahrungsmittelindustrie

Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z. B. anorganischen oder organischen Ionen.

2. Nicht-Nahrungsmittelindustrie

In diesem großen Bereich kann die Stärke als Hilfsstoff für unterschiedliche Herstellungsprozesse bzw. als Zusatzstoff in technischen Produkten eingesetzt werden. Bei der Verwendung der Stärke als Hilfsstoff ist hier insbesondere die Papier- und Pappeindustrie zu nennen. Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in Bezug auf die Steifigkeit, die Härte, den Klang, den Griff, den Glanz, die Glätte, die Spaltfestigkeit sowie die Oberflächen ausgenutzt.

2.1 Papier- und Pappeindustrie

Innerhalb des Papierherstellungsprozesses sind vier Anwendungsbereiche, nämlich Oberfläche, Strich, Masse und Sprühen, zu unterscheiden.

Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.

2.2 Klebstoffindustrie

Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.

2.3 Textil- und Textilpflegemittelindustrie

Ein großes Einsatzfeld für die Stärken als Hilfsmittel und Zusatzstoff ist der Bereich Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechternden Vorbehandlungen, wie Bleichen, Färben usw.,

Stärke als Verdickungsmittel bei der Herstellung von Farbpasten zur Verhinderung von Farbstoffdiffusionen sowie Stärke als Zusatz zu Kettungsmitteln für Nähgarne.

2.4 Baustoffindustrie

Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte zur Verzögerung der Abbindung eingesetzt.

2.5 Bodenstabilisation

Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.

2.6 Einsatz bei Pflanzenschutz- und Düngemitteln

Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.

2.7 Pharmaka, Medizin und Kosmetikindustrie

Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbieren und nach kurzer Zeit soweit quellen, daß der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.

2.8 Stärkezusatz zu Kohlen und Briketts

Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.

2.9 Erz- und Kohleschlammaufbereitung

Die Stärke kann ferner bei der Erz- und Kohleschlammaufbereitung als Flockungsmittel eingesetzt werden.

2.10 Gießereihilfsstoff

Ein weiterer Einsatzbereich besteht als Zusatz zu Gießereihilfsstoffen. Bei verschiedenen Gußverfahren werden Kerne benötigt, die aus Bindemittel-versetzten Sänden hergestellt werden. Als Bindemittel wird heute überwiegend Bentonit eingesetzt, das mit modifizierten Stärken, meist Quellstärken, versetzt ist.

Zweck des Stärkezusatzes ist die Erhöhung der FlieBfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.

2.11 Einsatz in der Kautschukindustrie

In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.

2.12 Herstellung von Lederersatzstoffen

Eine weitere Absatzmöglichkeit der modifizierten Stärken besteht bei der Herstellung von Lederersatzstoffen.

2.13 Stärke in synthetischen Polymeren
Auf dem Kunststoffsektor zeichnen sich folgende Einsatzgebiete ab: die Einbindung von Stärkefolgeprodukten in den Verarbeitungsprozeß (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyäthylen. Hierbei werden die Stärke und das synthetische Polymer durch Koexpression im Verhältnis von 1 : 1 zu einem 'master batch' kombiniert, aus dem mit granuliertem Polyäthylen unter Anwendung herkömmlicher Verfahrenstechniken diverse Produkte hergestellt werden. Durch die Einbindung von Stärke in Polyäthylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wäßrigen Farben erreicht werden.
Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit. '
Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des weiteren sind Stärke/ Polymermischungen günstig zu beurteilen, da sie eine sehr viel höhere biologische Abbaubarkeit aufweisen.
Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten wie Windeln und Unterlagen sowie im landwirtschaftlichen Sektor, z. B. bei Saatgutpillierungen.
Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und - form sowie Kristallinität, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur und -transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.
Die Erzeugung modifizierter Stärken mittels gentechnischer Eingriffe in einer transgenen Pflanze kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderten Stärken weiteren chemischen und/oder physikalischen Modifikationen unterworfen werden, was zu weiteren Verbesserungen der Qualität für bestimmte der oben beschriebenen Einsatzgebiete führt. Diese chemischen und physikalischen Modifikationen sind grundsätzlich bekannt. Insbesondere handelt es sich dabei um Modifikationen durch:

- Hitzebehandlung,
- Säurebehandlung,
- Erzeugung von Stärkeethern
  Stärke-Alkylether, O-Allylether, Hydroxylalkylether,
  O-Carboxylmethylether, N-haltige Stärkeether, P-haltige Stärkeether, S-haltige Stärkeether
- Erzeugung von vernetzten Stärken
- Erzeugung von Stärke-Pfropf-Polymerisaten
- Oxidation und
- Veresterungen, welche zur Entstehung von Phosphat-, Nitrat-, Sulfat-, Xanthat-, Acetat- und Citratstärken führen. Weitere organische Säuren können ebenfalls zur Veresterung eingesetzt werden.

In einer weiteren Ausführungsform betrifft die vorliegende Erfindung erntebare Pflanzenteile erfindungsgemäßer Pflanzen, wie Früchte, Speicherwurzeln. Wurzeln, Blüten, Knospen, Sprosse oder Stämme, vorzugsweise Samen oder Knollen, enthalten, wobei diese erntebaren Teile erfindungsgemäße Pflanzenzellen enthalten.
In einem weiteren Aspekt betrifft die vorliegende Erfindung eine regulatorische Region, die natürlicherweise die Transkription eines erfindungsgemäßen oben beschriebenen Nucleinsäuremoleküls, das ein Verzweigungsenzym aus Bakterien der Gattung Neisseria codiert, in bakteriellen Zellen kontrolliert, wobei die regulatorische Region eine Nucleotidsequenz enthält ausgewählt aus der Gruppe bestehend aus:

(a) Nucleotidsequenzen, die die Nucleotide 1 bis 169, der in SEQ ID NO: 1 dargestellten Nucleotidsequenz umfassen; und
(b) der Nucleotidsequenz der regulatorischen Region, die in der Insertion des Plasmids DSM 12425 enthalten ist.

Im Rahmen der vorliegenden Erfindung wird unter dem Begriff "regulatorische Region" eine Region verstanden, die die Spezifität und/oder das Ausmaß der Expression einer Gensequenz beeinflusst, beispielsweise derart, dass die Expression in Antwort auf bestimmte äußere Reize oder zu einem bestimmten Zeitpunkt auftritt: Derartige regulatorische Regionen liegen in der Regel in einem Bereich, der als Promotor bezeichnet wird. Der Begriff "Promotor" umfasst im Rahmen der vorliegenden Erfindung Nucleotidsequenzen, die für die Initiation der Transkription notwendig sind, d. h. für die Bindung der RNA-Polymerase, und kann z. B. auch die TATA-Box(en) umfassen.
Die Nucleotide 1 bis 169 der in SEQ ID NO:1 dargestellten Sequenz gehören zu der regulatorischen Region des Verzweigungsenzym-Gens aus Neisseria denitrificans. Putative Promotorsequenzen befinden sich an den Positionen 36 bis 44, 51 bis 55 und 157 bis 162, wobei es sich bei der Sequenz "GGGAGA" möglicherweise um eine Shine-Dalgamo-Sequenz handelt.
Die vorliegende Offenbarung betrifft ebenfalls rekombinante DNA-Moleküle, die eine erfindungsgemäße regulatorische Region umfassen.
Vorzugsweise ist in einem solchen rekombinanten DNA-Molekül die regulatorische Region mit einer heterologen DNA-Sequenz verknüpft. Dabei bedeutet der Ausdruck "heterolog", daß eine derartige Sequenz natürlicherweise nicht mit der regulatorischen Region verknüpft ist.
Ferner kann ein solches rekombinantes DNA-Molekül weitere regulatorische Elemente enthalten, die für die Transkription und/oder Translation in bakteriellen Zellen von Bedeutung sind, z. B. Transkriptions- oder Translations-Enhancer.
Weiterhin betrifft die vorliegende Offenbarung Wirtszellen, die mit einer erfindungsgemäßen regulatorischen Region, einem rekombinanten DNA-Molekül oder einem Vektor transformiert sind.
Die vorliegende Offenbarung betrifft ferner Vektoren, die eine erfindungsgemäße regulatorische Region oder ein erfindungsgemäßes rekombinantes DNA-Molekül enthalten. Solche Vektoren umfassen z. B. auch Plasmide, Cosmide, Bakteriophagen, Viren usw., die herkömmlicherweise für molekulargenetische Methoden verwendet werden.
Das erfindungsgemäße Verzweigungsenzym kann in einem in-vitro-Verfahren zur Herstellung von α-1,6-verzweigten α-1,4-Glucanen unter Verwendung des Substrats Saccharose und einer Enzymkombination aus einer Amylosucrase und dem Verzweigungsenzym verwendet werden. Unter einem "in-vitro-Verfahren" wird im Rahmen der vorliegenden Erfindung eine Umsetzung, d, h. eine Reaktion verstanden, die außerhalb eines lebenden Organismus abläuft. "In-vitro" bedeutet insbesondere, dass das erfindungsgemäße Verfahren in einem Reaktionsgefäß abläuft. Besonders bevorzugt bedeutet "in vitro", dass die Reaktion in Abwesenheit von lebenden Zellen stattfindet.
Vorteil eines solchen Verfahrens ist, daß es möglich ist, den Veizweigungsgrad zu steuern und daß durch diese Steuerung die Eigenschaften des synthetisierten Glucans an die jeweilige geplante Verwendung des Glucans angepaßt werden können. So besteht möglicherweise im Bereich der Verwendung als Verkapselungsmaterial im pharmazeutischen Bereich die Möglichkeit, über eine gezielte Einstellung des Verzweigungsgrades eine Optimierung der Freisetzungsrate von Arzneirnittelwirkstoffen zu erreichen.
Im Zusammenhang mit dem oben erwähnten in-vitro-Verfahren wird unter einer Amylosucrase (Saccharose: 1,4-α-D-Glucan 4-α-Glucosyltransferase, E.C. 2.4.1.4.) ein Enzym verstanden, das die Umsetzung von Saccharose zu wasserunlöslichen α-1,4-Glucanen und Fructose katalysiert. Für dieses Enzym wird das folgende Reaktionsschema vorgeschlagen:
Saccharose+ $(\alpha\text{-1,4-D-Glucan})_n \rightarrow$ D-Fructose+$(\alpha\text{-1,4-D-Glucan})_{n+1}$
Es handelt sich dabei um eine Transglycosylierungsreaktion. Die Produkte dieser Reaktion sind wasserunlösliche α-1,4-Glucane und Fructose. Die Transglucosylierung kann in Abwesenheit oder in Gegenwart von Akzeptormolekülen stattfinden. Solche. Akzeptormoleküle können z. B. Polysaccharide, wie z. B. Maltooligosaccharide, Dextrin oder Glycogen sein. Wenn ein solches Akzeptormolekül ein lineares, oligomeres α-1,4-Glucan ist, ist das resultierende Produkt der Transglucosylierunwreaktion durch die Amylosucrase ein polymeres lineares α-1,4-Glucan. Wenn die Transglucosylierungsreaktion mittels der Amylosucrase in Abwesenheit von jeglichen Akzeptormölekülen durchgeführt wird, wird ein Glucan mit einem terminalen Fructosemolekül erhalten. Im Rahmen der vorliegenden Offenbarung werden alle mittels einer Amylosucrase, in Abwesenheit oder Anwesenheit von Akzeptormoleküfen, erhältlichen Produkte als α-1,4-Glucane bezeichnet.
Für den Reaktionsmechanismus einer Transglucosylierung mittels einer Amylosucrase in Abwesenheit eines Akzeptormoleküls wird folgendes Reaktionsschema vorgeschlagen: G-F+n(G-F) $\rightarrow G_n$-G-F +nF, wobei G-F Saccharose ist, G Glucose ist, F Fructose ist und $G_n$-G-F ein $\alpha$-1,4-Glucan ist.
Für den Reaktionsmechanismus einer Transglucosylierung mittels Amylosucrase in Gegenwart eines Aktzeptormoleküls wird folgendes Reaktionsschema vorgeschlagen:

$$mG\text{-}F+G_n \rightarrow G_{n\text{-}m}+mF,$$

wobei $G_n$ ein Polysaccharid-Akzeptorrnolekül ist, $G_{n-m}$ ein Polysaccharid bestehend aus Akzeptor plus heransynthetisierter $\alpha$-1,4-Glucankette ist, G-F Saccharose ist, F Fructose ist und G Glucose ist.
Für die Transglucosylierung durch eine Amylosucrase sind keine Cofaktoren notwendig. Zur Durchführung des Verfahrens sind im Prinzip alle Amylosucrasen geeignet, die die Synthese linearer $\alpha$-1,4-Glucane ausgehend von Saccharose katalysieren.
Amylosucrasen sind bisher aus einigen Bakterienarten bekannt, insbesondere hauptsächlich aus Neisseria-Spezies (MacKenzie et al., Can. J. Microbiol. 24 (1978), 357-362).
Bevorzugt wird daher eine Amylosucrase prokaryontischen Ursprungs verwendet. Amylosucrasen sind beispielsweise bekannt aus Neisseria perflava (Okada und Hehre, J. Biol. Chem. 249 (1974), 126-135; MacKenzie et al., Can. J. Microbiol. 23 (1977), 1303-1307) oder Neisseria canis, Neisseria cinerea, Neisseria denitrificans, Neisseria sicca und Neisseria subflava (MacKenzie et al., Can. J. Microbiol. 24 (1978, 357-362). Weiterhin beschreibt die WO 95/31553 eine Amylosucrase aus Neisseria polysaccharea. Besonders bevorzugt wird eine natürlicherweise von einem Prokaryonten sekretierte Amylosucrase verwendet.
In einer bevorzugten Ausführungsform des Verfahrens wird eine Amylosucrase aus Neisseria polysaccharea verwendet Das Enzym, das in Neisseria polysaccharea exprimiert wird, ist extrem stabil, bindet sehr fest an die Polymerisationsprodukte und wird kompetitiv durch das Reaktionsprodukt Fructose inhibiert (MacKenzie et al., Can. J. Microbiol. 23 (1977)1303-1307). Bei der Neisseria-Spezies Neisseria polysaccharea wird die Amylosucrase sekretiert (Riou et al., Can. J. Microbiol. 32 (1986), 909-911), wohingegen sie bei anderen Neisseria-Arten in der Zelle verbleibt Ganz besonders bevorzugt wird eine Amylosucrase mit der in Seq ID No. 5 angegebenen Aminosäuresequenz verwendet.

In einer weiteren bevorzugten Ausführungsform wird eine gereinigte Amylosucrase verwendet.
Unter einer gereinigten Amylosucrase wird dabei ein Enzym verstanden, das weitgehend frei ist von Zellbestandteilen der Zellen, in denen das Protein synthetisiert wird. Vorzugsweise bedeutet der Begriff "gereinigte Amylosucrase" eine Amylosucrase, die einen Reinheitsgrad von mindestens 70 %, bevorzugt von mindestens 85 % und besonders bevorzugt von mindestens 90 % aufweist.
Der Einsatz eines gereinigten Proteins zur Herstellung von $\alpha$-1,4-Glucanen bietet verschiedene Vorteile. Im Vergleich zu Verfahren, die mit partiell aufgereinigten Proteinextrakten arbeiten, enthält das Reaktionsmedium des erfindungsgemäBen Verfahrens keine Reste des Produktionsstammes (Mikroorganismus), der verwendet wird, um das Protein zu reinigen oder gentechnisch herzustellen.
Des weiteren sind durch den Einsatz des gereinigten Proteins, Vorteile für die Anwendung in der Lebensmittel- und Pharmaindustrie zu sehen. Durch die definierte und von allen unnötigen Bestandteilen befreite Zusammensetzung des Reaktionsmediums ist auch das Produkt in seinen Bestandteilen genauer definiert. Dies führt zu einem wesentlich weniger umfangreichen Zulassungsverfahren für diese biotechnologisch erzeugten Produkte in der Lebensmittel- und Pharmaindustrie, insbesondere deshalb, weil diese Produkte keine Spuren eines transgenen Mikroorganismus aufweisen sollten.

Im Rahmen der vorliegenden Erfindung wird unter einem Verzweigungsenzym ($\alpha$-1,4-Glucan: $\alpha$-1,4-Glucan 6-Glycosyltransferase, E.C. 2.4.1.18) ein Protein verstanden, das eine Transglycosylierungsreaktion katalysiert, in der $\alpha$-1,4-Verknüpfungen eines $\alpha$-1,4-Glucandonors hydrolysiert und die dabei freigesetzten $\alpha$-1,4-Glucanketten auf eine $\alpha$-1,4-Glucanakzeptorkette transferiert und dabei in $\alpha$-1,6-Verknüpfungen überführt werden.

In einer bevorzugten Ausführungsform des beschriebenen in-vitro-Verfahrens wird gereinigtes Verzweigungsenzym verwendet. Unter einem gereinigten Verzweigungsenzym wird dabei ein Enzym verstanden, das weitgehend frei ist von Zellbestandteilen der Zellen, in denen das Protein synthetisiert wird. Vorzugsweise bedeutet "gereinigtes Verzweigungsenzym", daß das Enzym einen Reinheitsgrad von mindestens 70%, bevorzugt von mindestens 85% und besonders bevorzugt von mindestens 90% aufweist.

Weiterhin werden in dem beschriebenen in-vitro-Verfahren bevorzugt rekombinant hergestellte Proteine verwendet. Darunter werden Proteine verstanden, die dadurch hergestellt wurden, daß eine das jeweilige Protein codierende DNA-Sequenz in eine Wirtszelle eingebracht und dort zur Expression gebracht wird. Das Protein kann dann anschließend aus der Wirtszelle und/oder aus dem Kulturmedium gewonnen werden. Die Wirtszelle ist dabei vorzugsweise ein Bakterium oder ein Protist (z.B. Pilze, insbesondere Hefen, Algen) wie z. B. definiert in Schlegel "Allgemeine Mikrobiologie" (Georg Thieme Verlag, 1985, 1-2). Besonders bevorzugt werden die Proteine von der Wirtszelle sekretiert. Die Herstellung derartiger Wirtszellen zur Produktion eines rekombinanten Proteins kann nach dem Fachmann bekannten Methoden erfolgen.

Eine Übersicht über verschiedene Expressionssysteme findet man z.B. in Methods in Enzymology 153 (1987), 385-516, Bitter et al. (Methods in Enzymology 153 (1987), 516-544), Sawers et al., Applied Microbiology and Biotechnology 46 (1996), 1-9, Billman-Jacobe, Current Opinion in Biotechnology 7 (1996), 500-504, Hockney, Trends in Biotechnology 12 (1994), 456-463, und Griffiths et al., Methods in Molecular Biology 75 (1997), 427-440. Expressionsvektoren sind in großem Umfang in der Literatur beschrieben. Sie enthalten neben einem Selektionsmarkergen und einem die Replikation in dem gewählten Wirt sicherstellenden Replikationsursprung in der Regel einen bakteriellen oder viralen Promotor, sowie meist ein Terminationssignal für die Transkription. Zwischen Promotor und Terminationssignal befinden sich mindestens eine Restriktionsschnittstelle oder ein Polylinker, die die Insertion einer codierenden DNA-Sequenz ermöglichen. Als Promotorsequenz kann, sofern sie in dem gewählten Wirtsorganismus aktiv ist, die natürlicherweise die Transkription des entsprechenden Gens steuernde DNA-Sequenz verwendet werden. Diese Sequenz kann aber auch gegen andere Promotorsequenzen ausgetauscht werden. Es können sowohl Promotoren verwendet werden, die eine konstitutive Expression des Gens bewirken, als auch induzierbare Promotoren, die eine gezielte Regulation der Expression des nachgeschalteten Gens erlauben. Bakterielle und virale Promotorsequenzen mit diesen Eigenschaften sind in der Literatur ausführlich beschrieben. Regulatorische Sequenzen zur Expression in Mikroorganismen (z.B. E. coli, S. cerevisiae) sind ausreichend in der Literatur beschrieben. Promotoren, die eine besonders starke Expression des nachgeschalteten Gens erlauben, sind z.B. der 17-Promotor (Studier et al., Methods in Enzymology 185 (1990), 60-89), lacuv5, trp, trp-lacUV5 (DeBoer et al., in Rodriguez and Chamberlin (Eds), Promotors, Structure and Function; Praeger, New York, (1982), 462-481; DeBoer et al, Proc. Natl. Acad. Sci. USA (1983), 21-25), λp1, rac (Boros et al., Gene 42 (1986), 97-100). In der Regel erreichen die Proteinmengen von der Mitte bis gegen Ende der logarithmischen Phase des Wachstumszyklus der Mikroorganismen ihren Höhepunkt. Zur Synthese von Proteinen werden daher bevorzugt induzierbare Promotoren verwendet. Diese führen oft zu höheren Ausbeuten an Protein als konstitutive Promotoren. Die Verwendung starker konstitutiver Promotoren führt über die ständige Transkription und Translation eines clonierten Gens oft dazu, daß Energie für andere wesentliche Zellfunktionen verlorengeht und dadurch das Zellwachstum verlangsamt wird (Bernard R. Glick/ Jack J. Pasternak, Molekulare Biotechnologie (1995), Spektrum Akademischer Verlag GmbH, Heidelberg Berlin Oxford, S. 342.). Um ein Optimum an Proteinmenge zu erreichen, wird daher oft ein zweistufiges Verfahren angewendet. Zuerst werden die Wirtszellen unter optimalen Bedingungen bis zu einer relativ hohen Zelldichte kultiviert. Im zweiten Schritt wird dann die Transkription je nach Art des eingesetzten Promotors induziert. Besonders geeignet ist in diesem Zusammenhang ein durch Lactose- oder IPTG (=Isopropyl-β-D-thiögalacto-pyranosid) induzierbarer tac-Promotor (deBoer et al., Proc. Natl. Acad. Sci. USA 80 (1983), 21-25). Terminationssignale für die Transkription sind ebenfalls in der Literatur beschrieben.

Die Transformation der Wirtszelle mit der ein entsprechendes Protein codierenden DNA kann in der Regel nach Standardmethoden durchgeführt werden, wie z.B. beschrieben in Sambrook et al. (Molecular Cloning: A Laboratory Course Manual, 2nd edition (1989), Cold Spring Harbor Press, New York). Die Kultivierung der Wirtszelle erfolgt in Nährmedien, die den Bedürfnissen der jeweils verwendeten Wirtszelle entsprechen, insbesondere unter Berücksichtigung von pH-Wert, Temperatur, Salzkonzentration, Belüftung, Antibiotika, Vitaminen, Spurenelementen usw.

Die Reinigung des von den Wirtszellen produzierten Enzyms kann nach herkömmlichen Reinigungsmethoden wie Fällung, Ionenaustausch-Chromatographie, AffinitätsChromatographie, Gelfiltration, HPLC Reverse Phase Chromatographie usw. erfolgen.

Durch Modifikation der in den Wirtszellen exprimierten DNA läßt sich in der Wirtszelle ein Polypeptid herstellen, das aufgrund bestimmter Eigenschaften leichter aus dem Kulturmedium isoliert werden kann. So besteht die Möglichkeit, das zu exprimierende Protein als Fusionsprotein mit einer weiteren Polypeptidsequenz zu exprimieren, deren spezifische Bindungseigenschaften die Isolierung des Fusionsproteins über Affinitätschromatographie ermöglichen (z.B. Hopp et al., Bio/Technology 6 (1988), 1204-1210; Sassenfeld, Trends Biotechnol. 8 (1990), 88-93).

In einer bevorzugten Ausführungsform des beschriebenen in-vitro-Verfahrens werden Enzyme verwendet, die rekombinant hergestellt wurden und von der Wirtszelle in das Nährmedium sekretiert wurden, so daß kein Aufschluß von Zellen und keine weitere Aufreinigung des Proteins erforderlich ist, weil das sekretierte Protein aus dem Überstand gewonnen werden kann. Zur Entfernung von Restbestandteilen des Kulturmediums können in der Verfahrenstechnik gängige Methoden, wie z.B. Dialyse, reverse Osmose, chromatographische Methoden etc. eingesetzt werden. Gleiches gilt auch für die Aufkonzemrierung des in das Kulturmedium sekretierten Proteins. Die Sekretion von Proteinen durch Mikroorganismen wird normalerweise durch N-terminale Signalpeptide (Signalsequenz, leader-peptid) vermittelt. Proteine mit dieser Signalsequenz können die Zellmembran des Mikroorganismus durchdringen. Eine Sekretion von Proteinen kann dadurch erreicht werden, daß die DNA-Sequenz, die dieses Signalpeptid codiert, an die entsprechende, das Enzym codierende Region angefügt wird.

Bevorzugt ist ein gegebenenfalls natürlicherweise vorhandenes Signalpeptid, beispielsweise das der Amylosucrase aus Neisseria polysaccharea.

Ganz besonders bevorzugt ist das Signalpeptid der α-CGTase aus Klebsiella oxytoca M5A1 (Fiedler et al., J. Mol. Biol.

256 (1996), 279-291) oder ein Signalpeptid, wie es von den Nucleotiden 11529-11618 der unter der Zugriffsnummer X86014 in der GenBank zugänglichen Sequenz codiert wird.
Alternativ können die in dem beschriebenen in-vitro-Verfahren verwendeten Enzyme auch unter Verwendung eines in vitro-Transkriptions- und Translationssystem, das zur Expression der Proteine führt, ohne Einsatz von Mikroorganismen hergestellt worden sein.

In einer weiteren bevorzugten Ausführungsform sind die Amylosucrase und/oder das Verzweigungsenzym an einem Trägermaterial immobilisiert.
Eine Immobilisierung der Enzyme bietet den Vorteil, daß die Enzyme als Katalysatoren der Synthesereaktion auf einfache Weise aus dem Reaktionsgemisch wiedergewonnen und mehrfach verwendet werden können. Da die Aufreinigung von Enzymen in der Regel kosten-und zeitintensiv ist, ermöglicht eine Immobilisierung und Wiederverwertung eine erhebliche Kosteneinsparung. Ein weiterer Vorteil ist der Reinheitsgrad der Reaktionsprodukte, die keine Reste an Proteinen enthalten.
Für die Immobilisierung von Proteinen stehen eine Vielzahl von Trägermaterialien zur Verfügung, wobei die Kopplung an das Trägermaterial über kovalente oder nicht-kovalente Bindungen erfolgen kann (für eine Übersicht siehe: Methods in Enzymology 135, 136, 137). Weite Verbreitung als Trägermaterial haben z. B. Agarose, Algenat, Cellulose, Polyacrylamid. Silica oder Nylon.

In einer weiteren bevorzugten Ausführungsform des Verfahrens wird ein (partiell aufgereinigter) Enzymrohextrakt einer Amylosucrase und/oder des erfindungsgemäßen Verzweigungsenzyms verwendet. Unter einem Rohextrakt wird in diesem Zusammenhang eine Amylosucrase- und/oder Verzweigungsenzympräparation mit einem im Gegensatz zu einem aufgereinigten Enzym (siehe beispielsweise Beispiele 5 und 6) verringerten Reinheitsgrad verstanden.

In einer bevorzugten Ausführungsform wird bei dem beschriebenen in-vitro-Verfahren die Veränderung des Verzweigungsgrades der α-1,6-verzweigten α-1,4-Glucane durch die Veränderung des Proteinaktivitätsverhältnisses von Verzweigungsenzym zu Amylosucrase erreicht. Unter dem. Proteinaktivitätsverhälmis wird dabei das Verhältnis der eingesetzten Proteinaktivitäten (u) aus Amylosucrase zu Verzweigungsenzym verstanden. Die Bestimmung der Proteinaktivitäten kann dabei wie in den Beispielen 7 und 8 beschrieben erfolgen. Bei der Durchführung des Verfahrens (siehe Beispiel 9) kann ein Proteinaktivitätsverhältnis (Units Amylosucrase/Units Verzweigungsenzym) im Bereich von 1/4000 bis 2000/1 verwendet werden.
In einer bevorzugten Ausführungsform liegt das Proteinaktivitätsverhältnis in einem Bereich von 1/1500 bis 1500/1.
In einer weiteren bevorzugten Ausführungsform liegt das Proteinaktivitätsverhältnis in einem Bereich von 1/800 bis 1300/1.
In einer besonders bevorzugten Ausführungsform liest das Proteinaktivitätsverhältnis in einem Bereich von 1/400 bis 1200/1.
Über die Veränderung des Proteinaktivitätsverhältnisses ist die Veränderung des Verzweigungsgrades der erhaltenen α-1,6-verzweigten α-1,4-Glucane von 0,05% bis 35% möglich.
In einer bevorzugten Ausführungsform ist die Veränderung des Verzweigungsgrades der erhaltenen α-1,6-verzweigten α-1,4-Glucane in 6-Position von 0,15% bis 25% möglich, insbesondere von 0,20% bis 15% und ganz besonders bevorzugt von 0,25% bis 12%.
Mit Hilfe des erfindungsgemäßen Verfahrens ist es insbesondere möglich, Produkte herzustellen, die einen höheren Verzweigungsgrad als Glycogen aufweisen.

Unter dem Verzweigungsgrad wird im Rahmen der vorliegenden Erfindung der durchschnittliche Anteil an Verzweigungen in O-6-Position im Vergleich zu allen andersartig verknüpften Glucoseeinheiten verstanden, der durch Methylierungsanalyse ermittelt werden kann (siehe Beispiel 10).

In einer weiteren bevorzugten Ausführungsform wird bei dem beschriebenen in-vitro-Verfahren die Veränderung des Molekulargewichtes des Produktes durch die Veränderung des Proteinaktivitätsverhältnisses von Verzweigungsenzym zu Amylosucrase erreicht. Hierbei ist es insbesondere möglich, daß das Proteinaktivitätsverhältnis während der Reaktion, die zur Synthese der α-1,6-verzweigten α-1,4-Glucane führt, verändert wird.

In einer weiteren bevorzugten Ausführungsform des beschriebenen in-vitro-Verfahrens ist vorgesehen, daß das Verfahren bei unterschiedlichen Saccharosekonzentrationen durchgeführt wird. Möglich ist prinzipiell die Durchführung in einem Konzentrationsbereich von vorzugsweise 1% bis 80% Saccharose (Gew./Vol.), bevorzugt in einem Bereich von 5% bis 50% und besonders bevorzugt in einem Bereich von 10% bis 40%.

Im Zusammenhang mit der vorliegenden Erfindung wird das Molekulargewicht durch Lichtstreuungsexperimente (Light Scattering from Polymer solutions, editor. Huglin. M. B., Academic Press, London, 1972) nach Berry (J. Chem. Phys. 44 (1966), 4550ff.) bestimmt. Mit Hilfe des beschriebenen in-vitro-Verfahrens ist es insbesondere möglich, das Molekulargewicht der mittels des Verfahrens hergestellten α-1,6-verzweigten α-1,4-Glucane im Bereich von 1.000 bis 3000 x $10^5$ einzustellen. Vorzugsweise haben die hergestellten α-1,6-verzweigten α-1,4-Glucane ein Molekulargewicht im Bereich von 100.000 bis 1500 x $10^6$, insbesondere im Bereich von 100.000 bis 1000 x $10^6$, bevorzugt im Bereich von 262.000 bis 1000 x $10^6$ und besonders bevorzugt im Bereich von 262.000 bis 499 x $10^6$.

Die durch das beschriebene in-vitro-Verfahren erhältlichen α-1,6-venweigten α-1,4-Glucane weisen einen Verzweigungsgrad auf, der über dem Verzweigungsgrad liegt, der erreicht wird, wenn nur die Aktivität einer Amylosucrase eingesetzt wird, und der maximal bei 25-Mol% liegt.

Bevorzugt weisen die erhältlichen $\alpha$-1,6-verzweigten $\alpha$-1,4-Glucane einen Venweigungsgrad von 0,05 % bis 20 % auf, vorzugsweise im Bereich von 0,15 % bis 17 %, insbesondere im Bereich von 0,2 % bis 15 %, besonders bevorzugt im Bereich von 0,25 % bis 13 %, und ganz besonders bevorzugt im Bereich von 0,3 % bis 12 %. Bevorzugt liegt der Verzweigungsgrad im Bereich von 0.35 % bis 11 %, und insbesondere im Bereich von 0,4 % bis 10,5 %.

Die durch das beschriebene in-vitro-Verfahren erhältlichen $\alpha$-1,6-verzweigten $\alpha$-1,4-Glucane können wie oben für die Stärke beschrieben in der Nahrungsmittelindustrie und Nicht-Nahrungsmittelindustrie verwendet werden.

Das im Rahmen der vorliegenden Erfindung hergestellte Plasmid pBB48 wurde bei der als internationale Hinterlegungsstelle anerkannten Deutschen Sammlung von Mikroorganismen und Zellkulturen (DSMZ) in Braunschweig, Bundesrepublik Deutschland, entsprechend den Anforderungen des Budapester Vertrages am 25. September 1998, unter der Hinterlegungsnummer DSM 12425 hinterlegt.

| | |
|---|---|
| **Figur 1** | zeigt schematisch den Aufbau des Plasmids pBB48 (DSM 12425) |
| **Figur 2** | zeigt eine Reihe verschieden stark $\alpha$-1,6 verzweigter $\alpha$-1,4-Glucane, die durch das erfindungsgemäße Verfahren erzeugt wurden und die anschließend mit Lugolscher Lösung angefärbt wurden.<br>Von links nach rechts: Amylosucrase (links). Amylosucrase + fallende Mengen an Verzweigungsenzymaktivität. Die Absorptionsmaxima der entsprechenden Proben lagen bei: 615 nm, 483 nm 500 nm, 526 nm, 534 nm, 560 nm, 577 nm. |
| **Figur 3** | zeigt ein HPLC-Chromatogramm eines mit Isoamylase entzweigten hochverzweigten Verfahrensproduktes (A) und einer mit Isoamylase entzweigten Rattenleberglycogenprobe (B). |
| **Figur 4** | zeigt das Schema der Methylierungsänalyse |
| **Figur 5** | zeigt ein Diagramm der Analyseergebnisse der in Beispiel 9 und 10 beschriebenen Probe 7 nach einem und nach zwei Methylierungsschritten. Die Werte für die 2, 3, 6-Methylierung betragen 96,12% bzw. 96.36%. |
| **Figur 6** | zeigt eine graphische Darstellung der Anteile terminaler ("2346 Me") und 6-verknüpfter ("23 Me") Glucoseeinheiten der untersuchten Glucanproben. |
| **Figuren 7 und 8** | zeigen Gaschromatogramme der in den Beispielen beschriebenen Proben 3 und 7. |
| **Figur 9** | zeigt schematisch das Plasmid pBE-fnr-Km. |
| **Figur 10** | zeigt ein Aktivitätsgel für das Verzweigungsenzym. |
| **Figur 11** | zeigt die schematische Darstellung eines RVA-Profils. |
| **Figur 12** | zeigt die Korngrößenverteilung der Linien 143-13A und 143-59A im Vergleich zum Wildtyp. |
| **Figur 13** | zeigt die mikroskopische Vergrößerung der Stärkekörner der Linien 143-13A, 143-34A und 143-59A im Vergleich zu Stärkekörnern von Wildtyp-Pflanzen (Lichtmikroskop der Firma Leitz, Deutschland). |
| **Figur 14** | zeigt die Gelfestigkeit der Stärken verschiedener transgener Linien im Vergleich zu Stärken aus Wildtyp-Pflanzen bestimmt mit Hilfe eines Texture Analyzers. |
| **Figur 15** | zeigt das RVA-Profil der Stärken der Linien 143-11A, 143-13A, 143-59A im Vergleich zum Wildtyp. |
| **Figuren 16 bis 18** | zeigen die Ergebnisse von HPLC-Chromotographien, die das Muster der Seitenkettenverteilungen der Linien 143-WT (= Wildtyp), 143-13A und 143-59A darstellen. |
| **Figur 19** | zeigt den für die in den Figuren 16 bis 18 dargestellten Chromatographien verwendeten Elutionsgradienten. |
| **Figur 20** | zeigt die prozentuale Abweichung von Seitenketten bestimmter Kettenlängen der in Figur 16 bis 18 untersuchten Stärken vom Wildtyp. |

Die folgenden Beispiele erläutern die Erfindung

**Materialien:**

| | |
|---|---|
| Aufschlußpuffer: | 100 mM Tris/HCl pH 8,5; 5mM $Na_2$EDTA; 2mM DTT;1 mM Pefabloc® |
| Waschpuffer: | 50 mM Tris/HCl pH 8,5; 5mM $Na_2$EDTA; 10% Glycerol) |
| HIC-Puffer: | 50 mM Kaliumphosphat-Puffer pH 7,0; 5mM EDTA; 2mM DTT; 10% Glycerol |

Austern-Glycogen Type II from Oyster (Sigma G8751)

**Methoden:**

Stärkeanalytik

(a) Bestimmung des Amylose/Amylopektinverhältnisses

Stärke wurde nach Standardmethoden aus Kartoffelpflanzen isoliert, und das Verhältnis Amylose zu Amylopektin wurde nach der von Hovenkamp-Hermelink et al. beschriebenen Methode (Potato Research 31 (1988), 241-246) bestimmt.

(b) Bestimmung des Phosphatgehaltes

In der Stärke können die Positionen C2, C3 und C6 der Glucoseeinheiten phosphoryliert sein.
Zur Bestimmung des Phoshatgruppengehaltes an C6-Position wurden 100 mg Stärke in 1ml 0,7 M HCl für 4 Stunden bei 95°C hydrolysiert (Nielsen et al., Plant Physiol. 105 (1994), 11-117). Nach Neutralisation mit 0,7 M KOH wurden zur Glucose-6-Phosphat-Bestimmung 50 ml des Hydrolysats einem optisch-enzymatischen Test unterzogen. Die Änderung der Absorption des Testansatzes (100 mM Imidazol/HCl; 10 mM MgCl2; 0,4 mM NAD; 2 Units Glucose-6-phosphat-dehydrogenase aus Leuconostoc mesenteroides; 30°C) wurde bei 334 nm verfolgt.
Die Bestimmung des Gesamtphosphatgehaltes erfolgte nach der Methode von Ames (Methods in Enzymology VIII (1966), 115-118).
Es werden ca. 50 mg Stärke mit 30 μl ethanolischer Magnesiumnitrat-Lösung versetzt und drei Stunden bei 500°C im Muffelofen verascht. Der Rückstand wird mit 300 μl 0,5 M Salzsäure versetzt und 30 min bei 60°C inkubiert. Anschließend wird ein Aliquot auf 300 μl 0,5 M Salzsäure aufgefüllt, zu einer Mischung aus 100 μl 10%iger Ascorbinsäure und 600 μl 0,42% Ammoniummolybdat in 2 M Schwefelsäure gegeben und 20 min bei 45°C inkubiert.
Es folgt eine photometrische Bestimmung bei 820nm unter Berücksichtigung einer Phosphat-Eichreihe als Standard.

(c) Bestimmung der Gelfestigkeit (Texture Analyser)

2 g Stärke (TS) werden in 25 ml $H_2O$ verkleistert (vgl. RVA) und anschließend für 24 h luftdicht verschlossen bei 25°C gelagert. Die Proben werden unter der Sonde (runder Stempel) eines Texture Analysers TA-XT2 der Firma Stable Micro Systems fixiert und die Gelfestigkeit mit folgenden Parametern bestimmt:

- Test-Geschwindigkeit 0,5 mm/s
- Eindringtiefe 7 mm
- Kontaktfläche 113 $mm^2$
- Druck 2 g

(d) Viskositätsprofil

2 g Stärke (TS) werden in 25 ml $H_2O$ aufgenommen und für die Analyse in einem Rapid Visco Analyser (Newport Scientific Pty Ltd., Investmet Support Group, Warriewod NSW 2102, Australien) verwendet. Der Betrieb des Gerätes erfolgt nach den Angaben des Herstellers. Zur Bestimmung der Viskosität der wäßrigen Lösung der Stärke wird die Stärkesuspension zunächst von 50°C auf 95°C erhitzt, mit einer Geschwindigkeit von 12°C pro Minute. Anschließend wird die Temperatur für 2,5 Min bei 95°C gehalten. Danach wird die Lösung von 95°C auf 50°C abgekühlt, mit einer Geschwindigkeit von 12°C pro Minute. Während der gesamten Dauer wird die Viskosität bestimmt.
Die Bestimmung der Verkleisterungstemperatur erfolgt über die Steigung der Viskositätskurve in Abhängigkeit von der Zeit. Wird die Steigung der Kurve größer als 1,2 (dieser Wert wird vom Benutzer vorgegeben), identifiziert das Computerprogramm die zu diesem Zeitpunkt gemessene Temperatur als Verkleisterungstemperatur.

(e) Bestimmung von Glucose, Fructose und Saccharose

Die Bestimmung des Gehaltes von Glucose, Fructose und Saccharose erfolgt nach der von Stitt et al. (Methods in Enzymology 174 (1989), 518-552) beschriebenen Methode.

(f) Analyse der Seitenkettenverteilung des Amylopektins

Die Seitenkettenverteilung bzw. Vorbereitung wird bestimmt wie in Lloyd et al. (Biochem. J. 338 (1999), 515-521) beschrieben. Dabei sei darauf hingewiesen, daß bei dieser Methode nur das Amylopektin entzweigt wird und daß die Amylose vor Entzweigung des Amylopektins mittels Thymolfällung vom Amylopektin getrennt wird. Es werden folgende Elutionsbedingungen gewählt (vereinfachte Darstellung, genaues Elutionsprofil ist der Figur 19 zu entnehmen)

| **Zeit** | **0,15 M NaOH** | **1 M NaAc in 0,15M NaOH** |
|---|---|---|
| min | % | % |
| 0 | 100 | 0 |
| 5 | 100 | 0 |
| 20 | 85 | 15 |
| 35 | 70 | 30 |
| 45 | 68 | 32 |
| 60 | 0 | 100 |
| 70 | 0 | 100 |
| 72 | 100 | 0 |
| 80 | 100 | 0 |

(g) Korngrößenbestimmung

Die Korngrößenbestimmung wurde mit einem Fotosedimentometer des Typs "Lumosed" der Firma Retsch GmbH, Deutschland durchgeführt.
Die Körngrößenverteilung wurde in wäßriger Lösung bestimmt und erfolgte nach Herstellerangaben sowie basierend auf der Literatur von z.B. H. Pitsch, Korngrößenbestimmung; LABO-1988/3 Fachzeitschrift für Labortechnik, Darmstadt.

(h) Wasserbindevermögen

Zur Bestimmung des Wasserbindevermögens wurde der Rückstand nach der Abtrennung des löslichen Anteils durch Zentrifugation der bei 70°C gequollenen Stärke gewogen. Das Wasserbindevermögen (WBV) der Stärke wurde auf die um die lösliche Masse korrigierte Stärkeeinwaage bezogen.

$$\text{WBV (g/g)} = (\text{Rückstand} - (\text{Einwaage} - \text{löslicher Anteil}))/(\text{Einwaage} - \text{löslicher Anteil})$$

**Beispiel 1**

**Isolierung einer Verzweigungsenzym-codierenden genomischen DNA-Sequenz aus Neisseria denitrificans**

Für die Isolierung des Verzweigungsenzyms aus Neisseria denitrificans wurde zunächst eine genomische DNA-Bibliothek angelegt. Hierzu wurden Neisseria denitrificans-Zellen des Stammes, der als ATCC 14686 bei der ATCC hinterlegt ist, auf Columbia blood agar-Platten kultiviert und anschließend geerntet. Die genomische DNA wurde nach der Methode von Ausubel et al. (in: Current Protocolls in Molecular Biology (1987); J. Wiley & Sons, NY) isoliert und gereinigt. Nach einem partiellen Restriktionsverdau mit der Restriktionsendonuclease Sau3A erfolgte eine Ligation mit BamH I geschnittener Phagen-Vektor-DNA (lamdaZAPExpress von Stratagene). Nach der in-vivo-Excision der Phagen-Bibliothek wurden die erhaltenen Plasmide in die E. coli Mutante (PGM-) (Adhya and Schwartz, J. Bacteriol. 108 (1971), 621-626) transformiert. Diese Mutante bildet beim Wachstum auf Maltose lineare Polysaccharide, die nach dem Anfärben mit Jod blau erscheinen. Es wurden 60 000 Transformanden auf YT-Agar-Platten mit IPTG (1 mM), Kanamycin (12,5 mg/l) und Maltose (1%) ausplattiert und nach 16-stündiger Inkubation bei 37°C mit Jod bedampft. 60 Bakterienkolonien, die nach Jod-Bedampfung eine rote, eine braune oder eine gelbe Farbe aufwiesen, wurden selektiert und aus ihnen Plasmid-DNA isoliert (Birmboim-Doly, Nucleic Acid Res. 7, 1513-1523). Die isolierten Plasmide wurden anschließend zur Retransformation der gleichen E. coli- (PGM-) Mutante verwendet (Adhya and Schwartz, J. Bacteriol. 108 (1971), 621-626). Nach wiederholtem Ausplattieren und Jodbedampfen konnten die Klone von 60 auf vier Isolate reduziert werden. Von diesen vier Plasmiden wurde eine Restriktionsanalyse durchgeführt, die in allen vier Plasmiden ein gleich großes EcoR I -Fragment (1,6 kb) zeigte (Figur 1).

**Beispiel 2**

**Sequenzanalyse des genomischen Fragments des Plasmids pBB48**

Aus einem entsprechend Beispiel 1 erhaltenen Clon (pBB48), der eine ca. 3,9 kb große Insertion im Vektor pBK-CMV (Stratgene) aufwies, wurde das 1,6 kb EcoRI Fragment isoliert (Geneclean, Bio101) und zum Zwecke der DNA-Sequenzierung in den mit EcoRI geschnittenen Vektor pBluescript cloniert. Das so erhaltene Plasmid wurde sequenziert. Mit Hilfe des Ausgangsplasmides pBB48 wurde danach die vollständige Verzweigungsenzymcodierende DNA-Sequenz sowie die Sequenz flankierender Regionen ermittelt (SEQ ID NO:1). Das Plasmid pBB48 ist in der Fig. 1 dargestellt. Das Plasmid ist hinterlegt unter der Nummer DSM 12425.

**Beispiel 3**

**Expression des Verzweigungsenzyms in rekombinanten E. coli Zellen**

In den E. coli Laborstämmen ist im allgemeinen ein endogenes Verzweigungsenzym (glgB) exprimiert. Aus diesem Grunde wurde für den Nachweis der Verzweigungsenzymaktivität die G6MD2-Mutante von E. coli benutzt. Der Stamm E. coli Hfr G6MD2 (E. coli Genetic Stock Center, Yale University, CGSC#5080) trägt eine ausgedehnte Deletion im Bereich der Glucansynthese-Gene (glgA, glgB, glgC). Zum Nachweis der Verzweigungsenzymaktivität wurde diese Mutante mit dem Plasmid pBB48 transformiert und von den vermehrten Zellen ein Rohextrakt bereitet. Die Proteine dieses Rohextrakts wurden in einem Polyacrylamidgel elektrophoretisch getrennt und zum Nachweis der Verzweigungsenzymaktivität nachfolgend mit und ohne Rabbit-Phosphorylase b inkubiert (100mM Na-Citrat pH 7,0; AMP, Glucose-1-Phosphat). Nur in dem Phosphorylase stimulierten Gel traten violette Banden auf, was auf eine starke Verzweigungsenzymaktivität hindeutet.

**Beispiel 4**

**In vitro Produktion α-1,6-verzweigter α-1,4-Glucane mit Proteinrohextrakten im zellfreien System**

Für die Expression des Verzweigungsenzyms wurde die Mutante E. coli G6MD2 mit dem Plasmid pBB48 transformiert. Die Zellen wurden mit YT-Medium mit Kanamycin (12,5mg/l) 16 h unter Schütteln im Erlenmeyerkolben angezogen. Nach einer Zentrifugation (5000xg) wurde das erhaltene Pellet mit 100 mM Tris/HCl pH 7,5, 1mM DTT gewaschen und nach Suspension im gleichen Puffer die Zellen mit einer Ultraschallsonde aufgeschlossen. Eine weitere Zentrifugation (10000xg) trennte die Zelltrümmer von den löslichen Proteinen ab und ergab einen gelblichen Überstand mit einer Proteinkonzentration von ca. 10 mg/ml.
Von diesem so gewonnenen Protein-Rohextrakt wurde dann verschiedene Mengen (100 μl, 10 μl, 1 μl, 0,1 μl, 0,01 μl, 0,001 μl) zusammen mit jeweils gleichbleibender Menge einer Amylosucrase in 50 ml 100 mM Na-citrat pH 7,0 mit 20% Saccharose und 0,02% Na-Azid gegeben. Nach wenigen Stunden konnten die ersten Trübungen im Reaktionsgemisch beobachtet werden. Nach drei Tagen wurde zentrifugiert und die entstandenen Produkte mit deionisiertem Wasser gewaschen.
Die Produkte sind in DMSO löslich und können durch die Messung eines Absorptionsspektrums mit Lugolscher Lösung charakterisiert werden. Hierüber ist eine Abschätzung des Verzweigungsgrades der erzeugten Produkte möglich. Dazu wurde die DMSO-Lösung stark mit Wasser verdünnt, mit Lugolscher Lösung versetzt und sofort das Spektrum von 400nm bis 700 nm in einem Beckmann-Spektrophotometer gemessen (s. Fig. 2).
Eine Auftrennung der mit Isoamylase abgespaltenen Seitenketten auf einer Carbopak PA100-Säule mittels einer HPLC (DIONEX; Laufmittel: 150 mM NaOH mit 1M Na-acetat-Gradient) zeigt für ein stark verzweigtes Produkt das gleiche Muster wie für ein mit Isoamylase entzweigtes Rattenleberglycogen (Fig. 3).
Eine Abspaltung von Seitenketten nach Inkubation mit einer Pullulanase trat nur in sehr geringen Maße auf.

**Beispiel 5**

**Reinigung des Verzweigungsenzyms und N-terminale Sequenzierung des Proteins**

Zur Isolierung des Neisseria-denitrificans-Verzweigungsenzyms aus rekombinanten Hfr G6MD2- E. coli-Zellen (s.o.), die mit dem pBB48 transformiert worden waren, wurde zunächst eine Übernacht-Kultur dieser Zellen zentrifugiert. Der Zellniederschlag wurde anschließend in 3 Volumen Aufschlußpuffer suspendiert und bei einem Druck von ca. 16.000-17.000 psi in der French-Press aufgeschlossen. Nach einer einstündigen Zentrifugation bei 10000 g wurde der Überstand durch Zugabe von Waschpuffer auf das 4fache Volumen verdünnt, im batch-Verfahren an DEAE Cellulose DE52 gebunden und in eine Chromatographiesäule gefüllt, die mit 2 bis 3 Säulenvolumina Waschpuffer gewaschen wurde. Danach wurde zur Elution ein linearer 1M NaCl-Gradient angelegt. Die Fraktionen mit Verzweigungsenzymaktivität (siehe Beispiel 8) wurden vereinigt, mit $(NH_4)_2$ $SO_4$ versetzt (Endkonzentration 20 % (w/v)) und auf eine TSK Butyl-Toyopearl 650M Säule aufgetragen. Nach Waschen mit 2 bis 3 Säulenvolumina an HIC-Puffer, der vorher zusätzlich mit einer Ammoniumsulfatlösung mit einem Sättigungsgrad von 20% (114 g Ammoniumsulfat pro Liter) versetzt worden war, wurde das Verzweigungsenzym unter Einsatz eines von 20% auf 0% linear fallenden Ammoniumsulfatgradienten im HIC-Puffer eluiert. Fraktionen mit Verzweigungsenzymaktivität wurden vereinigt. Zur Aufkonzentrierung des Proteins wurde anschließend der Reinigungsschritt mit den vereinten Fraktionen unter Verwendung einer kleinen TSK Butyl-Toyopearl 650M Säule (Tose Haas (Montgomery Ville, Pensylvania)) wiederholt. Das aufgereinigte Protein wurde anschließend auf ein Polyacrylamidgel aufgetragen, auf PVDF-Membran geblottet, dann wieder in Lösung gebracht und von der Firma WITA GmbH, Teltow, Deutschland, N-terminal nach der Edman-Methode sequenziert. Die erhaltene Sequenz lautete: MNRNXH (SEQ ID NO:3).

**Beispiel 6**

**Reinigung einer Amylosucrase**

Zur Herstellung einer Amylosucrase wurden E. coli-Zellen verwendet, die mit einer DNA, codierend eine Amylosucrase aus Neisseria polysaccharea, transformiert waren. Die DNA hat die unter SEQ ID NO: 4 dargestellte Nucleotidsequenz und stammt aus einer genomischen Bibliothek von N. polysaccharea.

Eine Über-Nacht-Kultur dieser E. coli-Zellen, die die Amylosucrase aus Neisseria polysaccharea sekretieren, wurde abzentrifugiert und in ca. $^1/_{20}$ Volumen 50 mM Natriumcitratpuffer (pH 6,5), 10 mM DTT (Dithiothreitol), 1 mM PMSF (Phenylmethylsulfonylfluorid) resuspendiert. Anschließend wurden die Zellen mit einer French-Press bei 16.000 p.s.i. zweimal aufgeschlossen. Danach wurde dem Zell-Extrakt 1 mM $MgCl_2$ zugegeben sowie Benzonase (von Merck; 100,000 Units; 250 Units $\mu l^{-1}$) in einer Endkonzentration von 12,5 Units $ml^{-1}$. Anschließend wurde der Ansatz bei 37°C unter leichtem Rühren mindestens 30 min inkubiert. Der Extrakt wurde mindestens 1,5 Stunden auf Eis stehen gelassen. Anschließend wurde 30 min bei 4°C bei ca. 40 000 g zentrifugiert, bis der Überstand relativ klar war.

Es wurde eine Vorfiltration mit einer PVDF Membrane (Millipore "Durapore", o.ä.) durchgeführt, die einen Porendurchmesser von 0,45 µm besaß. Der Extrakt wurde über Nacht bei 4°C stehen gelassen. Vor Durchführung der HI-(hydrophobic interaction)-Chromatographie wurde der Extrakt mit festem NaCl versetzt, und auf eine Konzentration von 2 M NaCl eingestellt. Anschließend wurde wiederum für 30 min bei 4°C und ca. 40 000 mg zentrifugiert. Danach wurde der Extrakt von letzten Resten an E. coli befreit, indem er mit einer PVDF Membrane (Millipore "Durapore", o.ä.) filtriert wurde, die einen Porendurchmesser von 0,22 µm aufwies. Der filtrierte Extrakt wurde über eine Butylsepharose-4B-Säule (Pharmacia) aufgetrennt (Volumen der Säule: 93 ml, Länge: 17,5 cm). Ca. 50 ml Extrakt mit einer Amylosucrase-Aktivität von 1 bis 5 Units $\mu l^{-1}$ wurden auf die Säule gegeben. Anschließend wurden mit 150 ml Puffer B nicht-bindende Proteine von der Säule (Puffer B: 50 mM Natriumcitrat pH 6,5, 2 M NaCl) gewaschen. Die Amylosucrase wurde schließlich mit Hilfe eines fallenden, linearen NaCl-Gradient eluiert (von 2 M bis zu 0 M NaCl in 50 mM Natriumcitrat in einem Volumen von 433 ml bei einer Zuflußrate von 1,5 ml $min^{-1}$), welcher mit Hilfe eines automatischen Pumpsystems (FPLC, Pharmacia) generiert wurde. Die Elution der Amylosucrase erfolgte zwischen 0,7 M und 0,1 M NaCl. Die Fraktionen wurden gesammelt, über eine PD10 Sephadex Säule (Pharmacia) entsalzen, mit 8,7 % Glycerol stabilisiert, auf Amylosucrase-Aktivität überprüft und schließlich in Lagerpuffer (8,7 % Glycerol, 50 mM Citrat) eingefroren.

**Beispiel 7**

**Bestimmung der Amylosucrase-Aktivität**

Die Bestimmung der Amylosucrase-Aktivität erfolgt dadurch, daß aufgereinigtes Protein oder Proteinrohextrakt in unterschiedlichen Verdünnungen in 1 ml Ansätzen enthaltend 5 % Saccharose, 0,1 % Dextrin und 100 mM Citrat pH 6,5 gegeben und bei 37°C inkubiert wird. Nach 0 min, 30 min, 60 min, 120 min, 180 min, 240 min, 300 min und 360 min werden diesem Ansatz je 10 µl entnommen und durch sofortiges Erhitzen auf 95°C wird die enzymatische Aktivität der Amylosucrase beendet. Im gekoppelten photometrischen Test wird anschließend der Anteil der durch die Amylosucrase freigesetzten Fructose bestimmt. Dazu werden 1 µl bis 10 µl der inaktivierten Probe in 1 ml 50 mM Imidazolpuffer pH 6,9, 2 mM $MgCl_2$, 1 mM ATP, 0,4 mM $NAD^+$ und 0,5 U/ml Hexokinase gegeben. Nach sequentieller Zugabe von Glucose-6-phosphat-Dehydrogenase (aus Leuconostoc mesenteroides) und Phosphoglucose-Isomerase wird die Absorptionsänderung bei 340 nm gemessen. Anschließend wird mit Hilfe des Lambert-Beerschen-Gesetzes die Menge an freigesetzter Fructose berechnet.

Setzt man den erhaltenen Wert mit dem Zeitpunkt der Probennahme in Beziehung, so läßt sich die Zahl der Units (1U = µmol Fructose/min) (pro µl Proteinextrakt bzw. µg aufgereinigtes Protein) bestimmen.

**Beispiel 8**

**Bestimmung der Enzymaktivität eines Verzweigungsenzyms aus Neisseria denitrificans**

Die Bestimmung der enzymatischen Aktivität des Verzweigungsenzyms wurde in Anlehnung an eine in der Literatur beschriebenen Methode (Krisman et al., Analytical Biochemistry 147 (1985), 491-496; Brown and Brown, Meth. Enzymol. 8 (1966), 395-403) durchgeführt. Die Methode beruht auf dem Prinzip der verringerten Jod-Bindungsaffinität von verzweigten Glucanen im Vergleich zu unverzweigten α-1,4-Glucanen.
Zur Bestimmung der enzymatischen Aktivität des Verzweigungsenzyms wurde in eine gekühlte Mikrotiterplatte eine Probenserie von verschiedenen Verdünnungen des Verzweigungsenzyms gegeben. Anschließend wurde die Reaktion durch Zugabe von je 190μl Amylose-Reaktionsmischung (Zubereitung s.u.) gestartet und bei 37°C im Brutschrank inkubiert. Nach genau 30 min wurde die Reaktion durch Zugabe von 100 μl Lugolscher Lösung (0,5mM) gestoppt und die Proben im Mikrotiterplattenlesegerat (Molecular Devices) bei 650 nm gemessen. Als Kontrolle diente ein Ansatz ohne Amylose. Die Referenzprobe mit dem Maximalextinktionswert, die Amylose aber kein Verzweigungsenzym enthielt, wies eine $OD_{650}$ =1,2 auf.
Zur besseren Vergleichbarkeit unabhängiger Assays wird zur Auswertung nur die Probenverdünnung verwendet, die während der Inkubationszeit von 30 min zu einer Abnahme der $OD_{650}$ um 0,5 Einheiten führt.

Definition einer Aktivitäts-Unit (U) des Verzweigungsenzyms:

Eine halbe Unit des Verzweigungsenzyms ist die Enzymmenge, die in dem beschriebenen Test eine Abnahme der $OD_{650}$ um 0,5 Einheiten von 1,2 auf 0,7 in 30 min bewirkt.

Zubereitung der Amylose-Reaktionsmischung;

1 ml 0,5%ige Amyloselösung (Hersteller:Fluka; Amylose aus Kartoffel) w/v in DMSO werden unter Rühren in 10 ml Na-Citratpuffer (100 mM, pH 6,5, 0,02% w/v $NaN_3$) gegeben. Die klare Stammlösung wird für die Messung nochmals 1:4 bis 1:8 mit Na-Citratpuffer verdünnt. Die Absorption mit Lugolscher Lösung sollte im Test bei der eingesetzten Referenzprobe (Maximum) bei 1,2 liegen.

**Beispiel 9**

**Herstellung α-1,6-verzweigter α-1,4-Glucane unterschiedlichen Verzweigungsgrades**

Zur Herstellung α-1,6-verzweigter α-1,4-Glucane unterschiedlichen Verzweigungsgrades wurde eine 20%ige Saccharoselösung (w/v) in einem Reaktionsvolumen von 10,86 ml mit einer gereinigten Amylosucrase aus Neisseria polysaccharea (siehe Beispiel 6) und einem gereinigten Verzweigungsenzym aus Neisseria denitrificans (siehe Beispiel 5) versetzt. Je nach Versuchsansatz wurden diese beiden Enzyme in unterschiedlichen Proteinaktivitätsverhältnissen (Bestimmung Amylosucrase: siehe Beispiel 7; Bestimmung Verzweigungsenzym: siehe Beispiel 8) zueinander eingesetzt (siehe Tabelle 1):
Amylosucrasepräparation: 6,2 U/mg; 1,8 mg/ml
Verzweigungsenzympräparation: 75 U/mg; 6,9 mg/ml

**Tabelle 1**

| Nr. | μl BE | μl Amsu | Units BE | Units Amsu | Units Amsu/Units BE |
|---|---|---|---|---|---|
| 1 | 725 | 140 | 375 | 1,6 | 1/234,4 |
| 2 | 181,3 | 140 | 94 | 1,6 | 1/58,8 |
| 3 | 45,5 | 140 | 24 | 1,6 | 1/15 |
| 4 | 11,4 | 140 | 5,90 | 1,6 | 1/3,7 |
| 5 | 2,8 | 140 | 1,45 | 1,6 | 1,1/1 |
| 6 | 0,713 | 140 | 0,37 | 1,6 | 4,3/1 |
| 7 | 0,179 | 140 | 0,09263 | 1,6 | 17,3/1 |
| 8 | 0,0446 | 140 | 0,02308 | 1,6 | 69,3/1 |

(fortgesetzt)

| Nr. | µl BE | µl Amsu | Units BE | Units Amsu | Units Amsu/Units BE |
|---|---|---|---|---|---|
| 9 | 0,0112 | 140 | 0,00580 | 1,6 | 275,9/1 |
| 10 | 0,0028 | 140 | 0,00145 | 1,6 | 1103,4/1 |
| 11 | 0 | 140 | 0 | 1,6 | ----- |
| 13 | Glycogen | aus | Mytillus | edulis | ----- |
| BE=Verzweigungsenzym<br>Amsu=Amylosucrase<br>Units=Bestimmung siehe Beispiele 7 und 8 | | | | | |

**Beispiel 10**

**Bestimmung des Verzweigungsgrades mittels Methylierungsanalyse**

Der Verzweigungsgrad der erhaltenen Glucane wurde anschließend über eine Methylierungsanalyse bestimmt.

1. Durchgeführte Untersuchungen

- Methylierung aller freien OH-Gruppen der Glucanproben, jeweils Doppelbestimmungen
- Hydrolyse der permethylierten Polymere, gefolgt von Reduktion an C-1 und Acetylierung der Monomermischung
- Gaschromatographische Analyse und Quantifizierung der Reaktionsprodukte

Die Aufklärung des Verzweigungsgrades der Glucanproben erfolgte über eine Methylierungsanalyse (s. Figur 4). Die freien OH-Gruppen des Polymers werden durch Überführung in Methylether markiert.
Der Abbau zu den Monomeren erfolgt säurehydrolytisch und führt zu partiell methylierten Glucosemolekülen, die in pyranosider/furanosider Form sowie als α- und α-Glucoside vorliegen. Diese Varianten werden durch Reduktion mit $NaBH_4$ im jeweiligen partiell methylierten Sorbitderivat fokussiert. Durch die abschließende Acetylierung freier OH-Gruppen lassen sich die Reaktionsprodukte gaschromatographisch untersuchen.
Die folgende Tabelle zeigt die Konsistenz sowie die DMSO-Löslichkeit der erhaltenen Glucane.

**Tabelle 2**

| Probe | Konsistenz | DMSO-Löslichkeit (kalt) | DMSO-Löslichkeit (100°C) |
|---|---|---|---|
| 1 | Schaumstoffartig, farblos | (+) | + (leicht trübe Lösung) |
| 2 | n. d. | n. d. | n. d. |
| 3 | Schaumstoffartig, farblos | (+) | + (leicht trübe Lösung) |
| 4 | n. d. | n. d. | n. d. |
| 5 | Farbloses Pulver | + | + |
| 6 | n. d. | n. d. | n. d. |
| 7 | Farbloses Pulver | + | + |
| 8 | n. d. | n. d. | n. d. |
| 9 | Farbloses Pulver | + | + |
| 10 | n. d. | n. d. | n. d. |
| 11 | Farbloses Pulver | + | + |
| 13 | gelbliches Pulver | (+) | + |
| n. d. = nicht bestimmt | | | |

2. Experimenteller Teil

a) Herstellung der DMSO-Lösungen

Es wurden 1%ige Lösungen (w/v) in DMSO hergestellt. Die Proben waren nicht alle bei Raumtemperatur gut löslich: 1, 3 und 13 mußten für 30 Minuten auf 110°C erhitzt werden.

Bis auf die Lösungen 1 und 3, die leicht trübe waren, ergaben sich optisch klare Lösungen (siehe Tabelle 2).

b) Methylierung

2 ml der DMSO-Lösung (d.h. 20 mg Polymer) wurden in einen 50 ml Stickstoffkolben überführt, im $N_2$-Strom mit 5 Äquivalenten /OH (eq/OH) frisch hergestellter Dimsyllösung versetzt und für 30 Minuten gerührt. Die Lösungen wurden dabei trübe und viskos. Der Kolbeninhalt wurde in einem Eisbad eingefroren, mit 10 eq/OH Methyliodid versetzt und nach dem Auftauen für mindestens 2 h gerührt. Vor dem zweiten Deprotonierungs- und Methylierungsschritt wurde überschüssiges Methyliodid im Vakuum entfernt.

Die Aufarbeitung erfolgte nach Entfernen des überschüssigen Methyliodids durch Zugabe von 50 ml Wasser und 5maliger Extraktion mit je 10 ml Dichlormethan. Die organische Phase wurde anschließend durch 3-malige Extraktion mit Wasser von DMSO-Spuren befreit, mit $CaCl_2$ getrocknet, filtriert und eingeengt. Die Produkte lagen als klare, leicht gelbliche Filme vor.

Anhand der Probe 7 wurde zunächst geprüft, wieviele Methylierungsschritte zur Permethylierung der Hydroxylgruppen notwendig sind. Nach der ersten Methylierung wurde die Hälfte des Ansatzes aufgearbeitet, die andere Hälfte nochmals methyliert. Nach dem Abbau beider Proben wurden die Ergebnisse der GC-Analysen verglichen. Es zeigte sich, daß die Reaktion bereits nach einem Methylierungsschritt fast quantitativ war (siehe Figur 5). Um eine eventuelle Verzweigung an C-3 zu verifizieren, die auch durch eine Untermethylierung an dieser Position vorgetäuscht werden kann, wurde in jedem Fall eine zweite Methylierung angeschlossen.

Figur 5 zeigt ein Diagramm der Analysenergebnisse von Probe 7 nach einem und nach zwei Methylierungsschritten; die Werte für 2,3,6-Methylierung sind 96.12 % bzw. 96.36%

c) Hydrolyse

2 mg der methylierten Probe wurden in ein 1 ml Druckglas eingewogen, mit 0,9 ml 2 M Trifluoressigsäure versetzt und für 2.5 h bei 120°C gerührt. Nach dem Abkühlen des Glases wurde im $N_2$-Strom eingeengt. Zur Entfernung von Säurespuren wurde dreimal Toluol zugegeben und abgeblasen.

**Tabelle 3: Daten der Methylierung**

| | **Probe 1** | **Probe 3** | **Probe 5** | **Probe 7** | **Probe 9** | **Probe 11** | **Probe 13** |
|---|---|---|---|---|---|---|---|
| **Meth. 1** | | | | | | | |
| Einwaage (mg) | 21,9 | 22,7 | 21,7 | 32,5 | 23,4 | 22,6 | 23,5 |
| (mmol) | 0,135 | 0,140 | 0,134 | 0,200 | 0,144 | 0,139 | 0,145 |
| Auswaage (mg) | 30,4 | 29,2 | 28,0 | 25[1)] | 27,7 | 28,8 | 30,4 |
| (mmol) | 0,149 | 0,143 | 0,137 | 0.122[1)] | 0,136 | 0,141 | 0,149 |
| % d. Th.: | 110 | 102 | 102 | -[1)] | 94 | 101 | 103 |
| **Meth. 2** | | | | | | | |
| Einwaage: | 23,7 | 22,1 | 20,7 | 20,8 | 23,1 | 21,5 | 19,5 |
| mmol: | 0,146 | 0,136 | 0,128 | 0,128 | 0,142 | 0,133 | 0,120 |
| Auswaage: | 31,1 | 30,6 | 27,5 | 16,0[2] | 31,4 | 29,4 | 25,5 |
| mmol: | 0,152 | 0,150 | 0,135 | 0,078[2)] | 0,154 | 0,144 | 0,125 |
| % d. Th.: | 104 | 110 | 105 | 61[2)] | 108 | 108 | 104 |

1) Von dieser Probe wurde nach der ersten Methylierung die Hälfte entnommen und aufgearbeitet, so daß die Ausbeuten nicht genau angegeben werden können.

2) Die geringe Ausbeute beruht auf einem Fehler bei der Aufarbeitung.

d) Reduktion

Der Rückstand aus dem vorigen Reaktionsschritt wurde mit 0,5 ml einer 0,5 M ammoniakalischen NaBD4-Lösung versetzt und für 1h bei 60°C gerührt. Das Reagenz wurde vorsichtig mit einigen Tropfen Eisessig zerstört, das entstandene Borat durch fünfmalige Zugabe von 15%iger methanolischer Essigsäure und nachfolgendem Abblasen als Borsäuretrimethylester entfernt.

e) Acetylierung

Der Rückstand aus dem vorigen Reaktionsschritt wurde mit 50 µl Pyridin und 250 µl Essigsäureanhydrid versetzt und für 2 h bei 95 °C gerührt. Nach dem Abkühlen wurde das Reaktionsgemisch in 10 ml gesättigte $NaHCO_3$-Lösung getropft und fünfmal mit Dichlormethan extrahiert. Die Reaktionsprodukte in der organischen Phase wurden gaschromatographisch untersucht (Produkte siehe Figur 4).

f) Gaschromatographie

Die gaschromatographischen Untersuchungen wurden an einem Gerät der Firma Carlo Erba GC 6000 Vega Series 2 mit on-column-Einlaß und FID-Detektor vorgenommen. Die Trennungen erfolgten an einer fused-silica-Kapillarsäule Supelco SPB5 (Innendurchmesser 0,2 mm, Länge 30m) mit Wasserstoff als Trägergas und einem Druck von 80 kPa. Es wurde folgendes Temperaturprogramm verwendet: 60°C (1min) -25°C/min → 130°C-4°C/min → 280°C.

3. Ergebnisse

Die Auswertung der Gaschromatogramme erfolgte durch Identifikation der Peaks, Integration der Peakflächen und Korrektur der Daten mit Hilfe des ECR-Konzeptes von Sweet et all. (Sweet et al., Carbohydr. Res. 40 (1975), 217). Die bei den Proben 1 und 3 zu beobachtenden 1,6-Anhydroverbindungen sind auf den hohen Verzweigungsgrad an C-6 zurückzuführen. Dieser führt während der Hydrolyse zu Monomeren mit einer freien OH-Gruppe an C-6, die unter den Reaktionsbedingungen zu diesen Derivaten weiterreagieren kann. Die Anteile müssen bei der Berechnung des Verzweigungsgrades zum "2,3-Me"-Wert addiert werden.

Figur 6 ist eine graphische Darstellung der Anteile terminaler ("2346Me") und 6-verknüpfter ("23"Me) Glucoseeinheiten der untersuchten Glucanproben gezeigt.

**Tabelle 4** Analysenergebnisse in Mol-%: die Kurzbezeichnungen (A, B, usw.) entsprechen denen in Abb.1; "16AnhPy"= 1,6-Anhydro-4-O-acetyl-2,3-di-O-methyl-D-glucopyranose, "16AnhFu"= 1,6 Anhydro-5-O-acetyl-2,3-di-O-methyl-D-glucofuranose; "Me1" und "Me2" bezeichnen zwei unabhängige Methylierungsanalysen der jeweiligen Proben

| | Probe 1 | | | Probe 3 | | |
|---|---|---|---|---|---|---|
| | Me1 | Me2 | Mittelwert | Me1 | Me2 | Mittelwert |
| 16AnhPy | 0,37 | Spuren | 0,19 | Spuren | Spuren | – |
| 16AnhFu | 0,53 | 0,47 | 0,50 | Spuren | Spuren | – |
| 2346-Me (A) | 11,73 | 11,94 | 11,84 | 9,49 | 10,68 | 10,08 |
| 234-Me (B) | Spuren | Spuren | – | – | – | – |
| 236-Me (C) | 76,37 | 77,80 | 77,09 | 82,97 | 80,67 | 81,82 |
| 23-Me (D) | 9,75 | 9,16 | 9,46 | 7,54 | 8,34 | 7,94 |
| 26-Me (E) | 0,45 | 0,31 | 0,38 | Spuren | 0,32 | 0,16 |
| 36-Me | 0,44 | 0,31 | 0,38 | Spuren | Spuren | – |
| 2-Me | 0,20 | – | 0,10 | – | – | – |
| 3-Me | – | – | – | – | – | – |
| 6-Me | – | – | – | – | – | – |
| Un-Me | 0,20 | – | 0,10 | – | – | – |

| | Probe 5 | | | Probe 7 | | |
|---|---|---|---|---|---|---|
| | Me1 | Me2 | Mittelwert | Me1 | Me2 | Mittelwert |
| 16AnhPy | – | – | – | – | – | – |
| 16AnhFu | – | – | – | – | – | – |
| 2346-Me (A) | 2,42 | 2,51 | 2,47 | 2,60 | 2,77 | 2,69 |
| 234-Me (B) | – | – | – | – | – | – |
| 236-Me (C) | 95,54 | 96,18 | 95,86 | 96,36 | 96,89 | 96,63 |
| 23-Me (D) | 1,36 | 1,05 | 1,21 | 0,48 | 0,33 | 0,41 |
| 26-Me (E) | 0,37 | Spuren | 0,19 | 0,26 | Spuren | 0,13 |
| 36-Me | 0,30 | 0,26 | 0,28 | 0,29 | Spuren | 0,15 |
| 2-Me | – | – | – | – | | |
| 3-Me | – | – | – | – | | |
| 6-Me | – | – | – | – | | |
| Un-Me | – | – | – | – | | |

| | Probe 9 Me1 | Me2 | Mittelwert | Probe 11 Me1 | Me2 | Mittelwert |
|---|---|---|---|---|---|---|
| 16AnhPy | - | - | - | - | - | - |
| 16AnhFU | - | - | - | - | - | - |
| 2346-Me (A) | 2,89 | 2,79 | 2,84 | 2,60 | 2,49 | 2,55 |
| 234-Me (B) | - | - | - | - | - | - |
| 236-Me (C) | 95,62 | 95,62 | 95,62 | 96,21 | 97,20 | 96,70 |
| 23-Me (D) | 0,67 | 0,69 | 0,68 | 0,52 | 0,31 | 0,42 |
| 26-Me (E) | 0,36 | 0,42 | 0,39 | 0,36 | Spuren | 0,18 |
| 36-Me | 0,47 | 0,48 | 1,47 | 0,30 | Spuren | 0,15 |
| 2-Me | - | - | - | - | - | - |
| 3-Me | - | - | - | - | - | - |
| 6-Me | - | - | - | - | - | - |
| Un-Me | - | - | - | - | - | - |

| | Probe 13 Me1 | Me2 | Mittelwert |
|---|---|---|---|
| 16AnhPy | Spuren | Spuren | - |
| 16AnhFU | Spuren | Spuren | - |
| 2346-Me (A) | 8,91 | 7,46 | 8,19 |
| 234-Me (B) | Spuren | Spuren | - |
| 236-Me (C) | 83,71 | 85,45 | 84,58 |
| 23-Me (D) | 7,07 | 6,87 | 6,97 |
| 26-Me (E) | 0,32 | 0,22 | 0,27 |
| 36-Me | Spuren | Spuren | - |
| 2-Me | - | - | - |
| 3-Me | - | - | - |
| 6-Me | - | - | - |
| Un-Me | - | - | - |

**Beispiel 11**

**Herstellung α-1,6-verzweigter α-1,4-Glucane unterschiedlichen Molekulargewichts**

Zur Herstellung α-1,6-verzweigter α-1,4-Glucane unterschiedlichen Molekulargewichts wurde eine 20%ige Saccharoselösung (w/v) in einem Reaktionsvolumen von 10,86 ml mit einer gereinigten Amylosucrase aus Neisseria polysaccharea (siehe Beispiel 6) und einem gereinigten Verzweigungsenzym aus Neisseria denitrificans (siehe Beispiel 5) versetzt. Je nach Versuchsansatz wurden diese beiden Enzyme in unterschiedlichen Proteinaktivitätsverhältnissen (Bestimmung Amylosucraseaktivität; siehe Beispiel 7; Verzweigungsenzym: siehe Beispiel 8) zueinander eingesetzt (siehe Tabelle 1). Die Bestimmung der Molekulargewichte und des Trägheitsradius Rg erfolgte durch Laser-Streuung (Light Scattering from Polymer solutions, editor: Huglin, M. B., Academic Press, London, 1972). Dazu wurden die getrockneten Proben 1-11 in DMSO. $H_2O$ (im Verhältnis 90:10) gelöst und verschiedene Verdünnungen (ca. 2,5g/l bis 025 g/l) in einem Lichtstreuungsmeßgerät (SOFICA, Societe francaise d'instruments de controle et d'analyses. Le Mesnil Saint-Denis, Frankreich) anayisiert. Die so erhaltenen Daten wurden nach Berry (J. Chem. Phys. 44 (1966), 4550ff.) ausgewertet.

**Tabelle 5**

| Probe | Verhältnis Amylosucrase : Verzweigungsenzym | Trägheitsradius Rg in nm | Molekulargewicht in g/ mol |
|---|---|---|---|
| 1 | 0,05 | 104 | $282 \times 10^6$ |
| 2 | 0,2 | 154 | $499 \times 10^6$ |
| 3 | 0,8 | 76 | $228 \times 10^4$ |
| 4 | 3,21 | 64 | $76 \times 10^6$ |
| 5 | 12,84 | 63 | $20 \times 10^6$ |
| 6 | 51,22 | 38 | $1.1 \times 10^5$ |
| 7 | 204,03 | 277 | 472.000 |
| 8 | 818,87 | n. d. | n. d. |
| 9 | 3275,49 | 170 | 469.000 |
| 10 | 13043,48 | n. d. | n. d. |
| 11 | Kein Verzweigungsenzym | 143 | 262.000 |
| 13 | Glycogen (Miesmuscheln) | 14,3 | $1,59 \times 10^6$ g/mol (Burchard, W.: Macromolecules 10: 919 (1977)) |
| n. d. = nicht bestimmt | | | |

**Beispiel 12**

**Herstellung einer Expressionskassette für die Transformation von Pflanzen zur plastidären Expression eines Verzweigungsenzyms aus Neisseria denitrificans**

Unter der Verwendung der Oligonukleotide BE-5' und BE-3' (SEQ ID NO:6 und SEQ ID NO:7) wurde die für das Verzweigungsenzym aus Neisseria denitrificans codierende Sequenz ausgehend von dem Plasmid pBB48 (hinterlegt bei der Deutschen Sammlung für Mikroorgansimen und Zellkulturen (DSMZ) in Braunschweig, Bundesrepublik Deutschland unter der Hinterlegungsnummer DSM 12425) mittels PCR amplifiziert. Die daraus erhaltenen Amplifikate wurden mit den Restriktionsendonuclease SalI und SdaI verdaut und in das mit SalI und SdaI geschnittene Plasmid pBinAR-fnr cloniert. Das daraus resultierende Plasmid wurde mit pBE-fnr-Km (Fig.9) bezeichnet.

Bedingungen der PCR Reaktion:

Puffer und Polymerase von Boehringer Mannheim (Pwo Polymerase Nr.: 1644947)

| | |
|---|---|
| DNA | 0,2 ng |
| 10x Puffer + $MgSO_4$ | 5 µl |
| dNTPs (je 10 mM) | 1 µl |
| Primer BE-5' | 120 nM |
| Primer BE-3' | 120 nM |
| Pwo Polymerase | 1,0 Einheiten |
| Dest. Wasser | ad 50 µl |

Reaktionsbedingungen

| | | |
|---|---|---|
| Schritt 1 | 95°C | 2:00 Min |
| Schritt 2 | 95°C | 0:30 Min. |

(fortgesetzt)

| | | |
|---|---|---|
| Schritt 3 | 66°C | 0:30 Min. |
| Schritt 4 | 72°C | 2:00 Min. (plus 1 Sec pro Zyklus) |
| Schritt 5 | 72°C | 8:00 Min. |
| Die Schritte 2 bis 4 wurden zyklisch 40 mal wiederholt. | | |

Das Plasmid pBE-fnr-Km wurde zur Transformation von Kanoffelpflanzen nach Standardmethoden (s.o.) verwendet.

**Beispiel 13**

**Identifizierung und Nachweis von transgenen Kartoffelpflanzen mit Verzweigungsenzymaktivität**

Mittels Northern Blot Analyse konnten unter den gemäß Beispiel 12 hergestellten transgenen Kartoffelpflanzen solche identifiziert werden, die die mRNA eines Verzweigungsenzyms aus Neisseria denitrificans aufwiesen. Zum Nachweis der Aktivität des Verzweigungsenzyms in stabil transformierten Pflanzen wurde Blattmaterial der zu untersuchenden Pflanzen in flüssigem Stickstoff eingefroren und anschließend in einem mit flüssigem Stickstoff vorgekühlten Mörser zerkleinert. Bevor das zerkleinerte Material auftaute, erfolgte eine Zugabe von Extraktrionspuffer (50 mM Natriumcitrat, pH 6,5, 4 mM DTT, 2 mM Calciumchlorid). Zu ca. 100 mg Pflanzenmaterial (Frischgewicht) wurden ca. 200 µl Extraktionspuffer gegeben. Feste Bestandteile der Suspension aus zerkleinertem Pflanzenmaterial und Extraktionspuffer wurden durch Zentrifugation (10000x g) abgetrennt. Ein Aliquot des daraus erhaltenen klaren Überstandes wurde mit einem Viertel des Extraktvolumens Laufpuffer (40% Glycerin, 250 mM Tris pH 8,8, 0,02% Bromphenolblau) vermischt und im Polyacrylamid Gel (siehe unten), bei konstanter Stromstärke von 20 mA pro Gel aufgetrennt. (Bevor die Proteinextrakte aufgetragen wurden, erfolgte eine Elektrophorese der Gele für 20 Minuten unter den oben angegebenen Bedingungen). Nachdem der im Laufpuffer vorhandene Farbstoff Bromphenolblau aus dem Gel herausgelaufen war, wurde die Elektrophorese beendet. Das Gel wurde anschließend fünf mal in Waschpuffer (100 mM Natriumcitrat pH 6,5) in jeweils dem fünffachen Volumen des Gelvolumens für jeweils 20 Minuten unter Schwenken bei Raumtemperatur equilibriert. Anschließend wurde das Gel in der fünffachen Menge des Gelvolumens Inkubationspuffer (100 mM Natriumcitrat pH 6,5, 5% Saccharose, 0,625 Einheiten gereinigte Amylosucrase aus Neisseria polysaccharea (Reinigung des Enzyms und Bestimmung der Aktivität s.o.) bei 30°C für 16 Stunden inkubiert. Nach Abgießen des Inkubationspuffers wird nach Zugabe Lugolscher Lösung (1:5 verdünnt) das von der Amylosucrase in Kombination mit dem Verzweigungsenzym gebildete Glucan als bläulich-braune Bande (Fig. 10 )sichtbar. Das gesamte restliche Polyacrylamidgel färbt sich dabei durch die von der im Inkubationspuffer vorhandene Amylosucraseaktivität blau.

Zusammensetzung des Polyacrylamidgels:

a) Trenngel

375 mM Tris pH 8,8
7,5% Polyacrylamid (Biorad Nr. EC-890)
Für die Polymerisation:
1/2000 Volumen TEMED
1/100 Volumen Ammoniumpersulfat

b) Sammelgel

125 mM Tris pH 6,8
4% Polyacrylamid (Biorad Nr. EC-890)
Für die Polymerisation:
1/2000 Volumen TEMED
1/100 Volumen Ammoniumpersulfat

c) Elektrophoresepuffer

375 mM Tris pH 8,8
200 mM Glycin

**Beispiel 14**

**Analyse der Stärke von Pflanzen mit erhöhter Verzweigungsenzymaktivität**

Aus transgenen Kartoffelpflanzen, die gemäß Beispiel 12 und 13 hergestellt wurden, wurde nach Standardverfahren Stärke isoliert und hinsichtlich ihrer physikalischen und chaemischen Eigenschaften untersucht. Dabei stellte sich heraus, daß sich die von den transgenen Kartoffelpflanzen gebildete Stärke z. B. von in Wildtyp-Pflanzen synthetisierter Stärke in ihrem Phosphatgehalt und in den mittels RVA bestimmten Viskositäts- und Verkleisterungseigenschaften unterscheidet. Die Ergebnisse der physico-chemischen Charakterisierung der modifizierten Stärken, die auf den oben beschriebenen Analysemethoden basieren, sind in der folgenden Tabelle dargestellt.

| Nr. | Genotyp | Phosphat in C6 (%) | Amylosegehalt | RVA Max (%) | RVA Min (%) | RVA Fin (%) | RVA Set (%) | RVA T (%) | Gelfestigkeit (%) |
|---|---|---|---|---|---|---|---|---|---|
| 1 | Desiree (Wildtyp) | 100 | 22,0 | 100 | 100 | 100 | 100 | 100 | 100 |
| 2 | 143-13A | 36 | 20,9 | 50 | 83 | 82 | 79 | 79 | 162 |
| 3 | 143-11A | --- | 22,5 | 92 | 90 | 88 | 80 | 99,5 | --- |
| 4 | 143-59A | 22 | 20,9 | 36 | 69 | 78 | 114 | 99 | 225 |

Legende:
143-13A; 143-11A, 143-59A: transgene Kartoffelpflanzen, die das Verzweigungsenzym aus Neisseria denitrificans überexprimieren.
RVA = Rapid Visco Analyzer
Max = maximale Viskosität=Peakviskosität
Min = minimale Viskosität
Fin = Viskosität am Ende der Messung
Set = Setback = Differenz aus Min und Fin
T = Verkleisterungstemperatur
Die Prozentwerte sind mit Ausnahme des Amylosegehaltes auf den Wildtyp (= 100 %) bezogen.

Die Ergebnisse RVA-Analyse, der Analyse der Korngrößenverteilung und der Gelfestigkeit sind auch in den Figuren 11 bis 15 wiedergegeben.
Ferner zeigen die Figuren 16 bis 18 die Ergebnisse von HPLC-Chromotographien, die das Muster der Seitenkettenverteilung der Linien 143-WT (=Wildtyp), 143-13A und 143-59A darstellen. Die Figur 19 zeigt dabei den Elutionsgradienten, der im Zusammenhang mit der HPLC-Analyse verwendet wurde. In Figur 20 ist darüberhinaus die prozentuale Abweichung von Seitenketten bestimmter Kettenlängen vom Wildtyp dargestellt.
Die folgenden zwei Tabellen verdeutlichen, wie dabei die Berechnung der Seitenkettenanteile erfolgte.

**Tabelle 7**

| **143-59A (Messung 1)** | | | **143-59A (Messung 2)** | | |
|---|---|---|---|---|---|
| **Bezeichnung des Peaks** | **Peakfläche** | **Anteil an der Summe [%]** | **Peakfläche** | **Anteil an der Summe [%]** | **Mittelwert der Flächenanteile** |
| | **A 2** | **B 2** | **C 2** | **D 2** | **E 2** |
| DP 6 | 577122 | 4,50 | 690167 | 5,08 | 4,79 |
| DP 7 | 504371 | 3,93 | 544770 | 4,01 | 3,97 |
| DP 8 | 341520 | 2,66 | 377170 | 2,77 | 2,72 |
| DP 9 | 387706 | 3,02 | 462686 | 3,40 | 3,21 |
| DP 10 | 511664 | 3,99 | 602911 | 4,43 | 4,21 |
| DP 11 | 684394 | 5,34 | 776228 | 5,71 | 5,52 |

(fortgesetzt)

| 143-59A (Messung 1) | | | 143-59A (Messung 2) | | |
|---|---|---|---|---|---|
| **Bezeichnung des Peaks** | **Peakfläche** | **Anteil an der Summe [%]** | **Peakfläche** | **Anteil an der Summe [%]** | **Mittelwert der Flächenanteile** |
| | **A 2** | **B 2** | **C 2** | **D 2** | **E 2** |
| DP 12 | 884346 | 6,90 | 976001 | 7,18 | 7,04 |
| DP 13 | 1038389 | 8,10 | 1138027 | 8,37 | 8,23 |
| DP 14 | 1080589 | 8,43 | 1175544 | 8,65 | 8,54 |
| DP 15 | 1046585 | 8,16 | 1144404 | 8,42 | 8,29 |
| DP 16 | 977127 | 7,62 | 1016555 | 7,48 | 7,55 |
| DP 17 | 850092 | 6,63 | 881777 | 6,49 | 6,56 |
| DP 18 | 720854 | 5,62 | 739080 | 5,44 | 5,53 |
| DP 19 | 626277 | 4,88 | 627135 | 4,61 | 4,75 |
| DP 20 | 526159 | 4,10 | 522122 | 3,84 | 3,97 |
| DP 21 | 439356 | 3,43 | 431106 | 3,17 | 3,30 |
| DP 22 | 354956 | 2,77 | 336907 | 2,48 | 2,62 |
| DP 23 | 281320 | 2,19 | 266412 | 1,96 | 2,08 |
| DP 24 | 224165 | 1,75 | 200219 | 1,47 | 1,61 |
| DP 25 | 176641 | 1,38 | 169596 | 1,25 | 1,31 |
| DP 26 | 152651 | 1,19 | 145821 | 1,07 | 1,13 |
| DP 27 | 153046 | 1,19 | 123171 | 0,91 | 1,05 |
| DP 28 | 117125 | 0,91 | 103599 | 0,76 | 0,84 |
| DP 29 | 92294 | 0,72 | 85067 | 0,63 | 0,67 |
| DP 30 | 73885 | 0,58 | 59729 | 0,44 | 0,51 |
| | ΣA2 | | ΣC2 | | |
| Summe | 12822634 | 100,00 | 13596204 | 100,00 | 100,00 |
| Die Peakflächen in Spalte A 1, A 2, C 1 und C 2 wurden mit dem Anwendungsprogramm AI-450 Version 3.31 der Firma Dionex ermittelt. | | | | | |

**Tabelle 8**

| 143-WT (Messung 1) | | | 143-WT (Messung 2) | | |
|---|---|---|---|---|---|
| **Bezeichnung des Peaks** | **Peakfläche** | **Anteil an der Summe [%]** | **Peakfläche** | **Anteil an der Summe [%]** | **Mittelwert der Flächenanteile** |
| | **A 1** | **B 1** | **C 1** | **D 1** | **E 1** |
| DP 6 | 123190 | 1,75 | 160046 | 1,68 | 1,72 |
| DP 7 | 95526 | 1,36 | 137396 | 1,45 | 1,40 |
| DP 8 | 87365 | 1,24 | 126639 | 1,33 | 1,29 |
| DP 9 | 158742 | 2,26 | 210845 | 2,22 | 2,24 |
| DP 10 | 308544 | 4,39 | 382957 | 4,03 | 4,21 |

(fortgesetzt)

| 143-WT (Messung 1) | | | 143-WT (Messung 2) | | |
|---|---|---|---|---|---|
| **Bezeichnung des Peaks** | **Peakfläche** | **Anteil an der Summe [%]** | **Peakfläche** | **Anteil an der Summe [%]** | **Mittelwert der Flächenanteile** |
| | **A 1** | **B 1** | **C 1** | **D 1** | **E 1** |
| DP 11 | 465107 | 6,61 | 5817.74 | 6,12 | 6,36 |
| DP 12 | 574882 | 8,17 | 721814 | 7,59 | 7,88 |
| DP 13 | 634154 | 9,01 | 796824 | 8,38 | 8,70 |
| DP 14 | 633566 | 9,01 | 798684 | 8,40 | 8,70 |
| DP 15 | 594327 | 8,45 | 766484 | 8,06 | 8,25 |
| DP 16 | 537537 | 7,64 | 699141 | 7,35 | 7,50 |
| DP 17 | 470522 | 6,69 | 609229 | 6,41 | 6,55 |
| DP 18 | 403081 | 5,73 | 539584 | 5,67 | 5,70 |
| DP 19 | 352504 | 5,01 | 486633 | 5,12 | 5,06 |
| DP 20 | 313708 | 4,46 | 432720 | 4,55 | 4,51 |
| DP 21 | 265289 | 3,77 | 385358 | 4,05 | 3,91 |
| DP 22 | 211722 | 3,01 | 323248 | 3,40 | 3,20 |
| DP 23 | 179015 | 2,54 | 274938 | 2,89 | 2,72 |
| DP 24 | 148758 | 2,11 | 227219 | 2,39 | 2,25 |
| DP 25 | 119135 | 1,69 | 197839 | 2,08 | 1,89 |
| DP 26 | 103902 | 1,48 | 177493 | 1,87 | 1,67 |
| DP 27 | 88686 | 1,26 | 147919 | 1,56 | 1,41 |
| DP 28 | 67024 | 0,95 | 131325 | 1,38 | 1,17 |
| DP 29 | 61086 | 0,87 | 104515 | 1,10 | 0,98 |
| DP 30 | 37850 | 0,54 | 87704 | 0,92 | 0,73 |
| | Σ A1 | | Σ C1 | | |
| Summe | 7035222 | 100,00 | 9508328 | 100,00 | 100,00 |

**Patentansprüche**

1. Nucleinsäuremolekül codierend ein Verzweigungsenzym aus einem Bakterium der Gattung Neisseria, ausgewählt aus der Gruppe bestehend aus

   (a) Nucleinsäuremolekülen, die ein Protein codieren, das die unter SEQ ID NO:2 angegebene Aminosäuresequenz umfaßt;
   (b) Nucleinsäuremolekülen, die die unter SEQ ID NO:1 angegebene codierende Region umfassen;
   (c) Nucleinsäuremolekülen, die ein Protein codieren, das die Aminosäuresequenz umfasst, die von der Insertion in Plasmid DSM 12425 codiert wird;
   (d) Nucleinsäuremolekülen, die die in der Insertion des Plasmids DSM 12425 enthaltene codierende Region für ein Verzweigungsenzym umfassen;
   (e) Nucleinsäuremolekülen, die ein Verzweigungsenzym aus einem Bakterium der Gattung Neisseria codieren, dessen Sequenz im Bereich der ersten 100 Aminosäuren eine Sequenzidentität von mindestens 65% zu der unter SEQ ID NO:2 angegebenen Aminosäuresequenz aufweist;
   (f) Nucleinsäuremolekülen, deren komplementärer Strang mit einem Nucleinsäuremolekül nach (a), (b), (c), (d) und/oder (e) unter stringenten Bedingungen hybridisiert und die ein Verzweigungsenzym aus einem Bakterium der Gattung Neisseria codieren; und
   (g) Nucleinsäuremolekülen, deren Sequenz aufgrund der Degeneration des genetischen Codes von der Sequenz eines Nucleinsäuremoleküls nach (f) abweicht.

2. Vektor enthaltend ein Nucleinsäuremolekül nach Anspruch 1.

3. Vektor nach Anspruch 2, wobei das Nucleinsäuremolekül in sense-Orientierung mit regulatorischen Sequenzen verknüpft ist, die die Transkription in prokaryontischen oder eukaryontischen Zellen gewährleisten.

4. Wirtszelle, die mit einem Vektor nach Anspruch 2 oder 3 transformiert ist.

5. Verfahren zur Herstellung eines Verzweigungsenzyms aus einem Bakterium der Gattung Neisseria, wobei eine Wirtszelle nach Anspruch 4 unter Bedingungen kultiviert wird, die die Expression des Proteins erlauben, und das Protein aus den kultivierten Zellen und/oder dem Kulturmedium isoliert wird.

6. Verfahren zur Herstellung eines Verzweigungsenzyms aus einem Bakterium der Gattung Neisseria, wobei das Protein in einem in vitro-Transkriptions- und -Translationssystem unter Verwendung eines Nucleinsäuremoleküls nach Anspruch 1 hergestellt wird.

7. Verzweigungsenzym codiert durch ein Nucleinsäuremolekül nach Anspruch 1 oder erhältlich durch ein Verfahren nach Anspruch 5 oder 6.

8. Antikörper, der spezifisch ein Protein nach Anspruch 7 erkennt.

9. Verwendung eines Proteins nach Anspruch 7 zur Herstellung von $\alpha$-1,6-verzweigten $\alpha$-1,4-Glucanen in in-vitro Systemen.

10. Transgene Pflanzenzelle enthaltend ein Nucleinsäuremolekül nach Anspruch 1, wobei das Nucleinsäuremolekül mit regulatorischen Sequenzen verknüpft ist, die die Transkription in pflanzlichen Zellen gewährleisten.

11. Transgene Pflanzenzelle nach Anspruch 10, wobei das Nucleinsäuremolekül verknüpft ist mit einer Sequenz, die eine Signalsequenz codiert, die die Lokalisation des codierten Proteins in den Plastiden der Zellen gewährleistet.

12. Transgene Pflanze enthaltend Pflanzenzellen nach Anspruch 10 oder 11.

13. Verfahren zur Herstellung einer transgenen Pflanze, wobei

(a) eine pflanzliche Zelle genetisch modifiziert wird durch die Einführung eines Nucleinsäuremoleküls nach Anspruch 1 oder eines Vektors nach Anspruch 2 oder 3;
(b) aus der gemäß Schritt (a) hergestellten Zelle eine Pflanze regeneriert wird; und
(c) aus der gemäß Schritt (b) erzeugten Pflanze gegebenenfalls weitere Pflanzen erzeugt werden, die Zellen enthalten, die ein Nucleinsäuremolekül nach Anspruch 1 oder einen Vektor nach Anspruch 2 oder 3 enthalten.

14. Erntebare Pflanzenteile von Pflanzen nach Anspruch 12 oder von Pflanzen erhältlich durch ein Verfahren nach Anspruch 13, wobei diese Pflanzenteile transgene Pflanzenzellen nach Anspruch 10 oder 11 enthalten.

15. Transgene Pflanzenzelle nach Anspruch 10 oder 11, transgene Pflanze nach Anspruch 12, Verfahren nach Anspruch 13 und Pflanzenteile nach Anspruch 14, wobei die Pflanze Kartoffel ist und die Pflanzenzellen und Pflanzenteile aus Kartoffel sind.

16. Verfahren zur Herstellung von Stärke, umfassend das Extrahieren von Stärke aus transgenen Pflanzen nach Anspruch 12 oder 15, aus transgenen Pflanzen erhältlich durch ein Verfahren nach Anspruch 13 oder 15, aus stärkespeichernden Teilen einer solchen Pflanze oder aus Pflanzenzellen nach Anspruch 10, 11 oder 15.

17. Verfahren gemäß Anspruch 16, wobei Kartoffelstärke hergestellt wird, die einen im Vergleich zu Stärke aus entsprechenden Wildtyp-Pflanzen um mindestens 10% erhöhten prozentualen Anteil an Stärkekörnern mit einer Korngröße von bis zu 15 $\mu$m aufweist.

18. Regulatorische Region, enthaltend eine Nucleotidsequenz ausgewählt aus der Gruppe bestehend aus:

(a) Nucleotidsequenzen, die die Nucleotide 1 bis 169 der in SEQ ID NO:1 dargestellten Nucleotidsequenz umfassen; und
(b) der Nucleotidsequenz der regulatorischen Region, die in der Insertion des Plasmids DSM 12425 enthalten ist.

## Claims

1. Nucleic acid molecule coding for a branching enzyme from a bacterium of the genus Neisseria, selected from the group consisting of

   (a) nucleic acid molecules coding for a protein which comprises the amino acid sequence depicted under SEQ ID NO:2;
   (b) nucleic acid molecules comprising the coding region depicted under SEQ ID NO:1;
   (c) nucleic acid molecules coding for a protein comprising the amino acid sequence encoded by the insertion in plasmid DSM 12425;
   (d) nucleic acid molecules comprising the coding region for a branching enzyme, which is contained in the insertion of plasmid DSM 12425;
   (e) nucleic acid molecules coding for a branching enzyme from a bacterium of the genus Neisseria, the sequence of which has a sequence identity of at least 65% in the region of the first 100 amino acids to the amino acid sequence depicted under SEQ ID NO:2;
   (f) nucleic acid molecules the complementary strand of which hybridises to a nucleic acid molecule according to (a), (b), (c), (d) and/or (e) under stringent conditions, and which code for a branching enzyme from the bacterium of the genus Neisseria; and
   (g) nucleic acid molecules the sequence of which deviates from the sequence of a nucleic acid molecule according to (f) due to the degeneration of the genetic code.

2. Vector containing a nucleic acid molecule according to claim 1.

3. Vector according to claim 2, wherein the nucleic acid molecule is linked in sense orientation to the regulatory sequences, which ensure the transcription in prokaryotic or eukaryotic cells.

4. Host cell, which is transformed with a vector according to claim 2 or 3.

5. Method for the production of a branching enzyme from a bacterium of the genus Neisseria, wherein a host cell according to claim 4 is cultivated under conditions which allow for the expression of the protein, and the protein is isolated from the cultivated cells and/or the culture medium.

6. Method for the production of a branching enzyme from a bacterium of the genus Neisseria, wherein the protein is produced in an in vitro transcription and translation system using a nucleic acid molecule according to claim 1.

7. Branching enzyme encoded by the nucleic acid molecule according to claim 1 or obtainable by a method according to claim 5 or 6.

8. Antibody which specifically recognises a protein according to claim 7.

9. Use of a protein according to claim 7 for the production of $\alpha$-1,6-branched $\alpha$-1,4-glucans in in vitro systems.

10. Transgenic plant cell containing a nucleic acid molecule according to claim 1, wherein the nucleic acid molecule is linked with regulatory sequences which ensure the transcription in plant cells.

11. Transgenic plant cell according to claim 10, wherein the nucleic acid molecule is linked with a sequence coding for a signal sequence, which ensures the localisation of the coded protein in the plastids of the cells.

12. Transgenic plant containing plant cells according to claim 10 or 11.

13. Method for the production of a transgenic plant, wherein

    (a) a plant cell is genetically modified by the introduction of a nucleic acid molecule according to claim 1 or a vector according to claim 2 or 3;
    (b) a plant is regenerated from the cell produced according to step (a); and
    (c) optionally further plants are produced from the plant produced according to step (b), which contain cells containing a nucleic acid molecule according to claim 1 or a vector according to claim 2 or 3.

**14.** Harvestable plant parts of plants according to claim 12 or of plants obtainable by a method according to claim 13, wherein said plant parts contain transgenic plant cells according to claim 10 or 11.

**15.** Transgenic plant cell according to claim 10 or 11, transgenic plant according to claim 12, method according to claim 13 and plant parts according to claim 14, wherein the plant is potato and the plant cells and plant parts are from potato.

**16.** Method for the production of starch, comprising the extraction of starch from transgenic plants according to claim 12 or 15, from transgenic plants obtainable by a method according to claim 13 or 15, from starch-storing parts of said plant or from plant cells according to claim 10, 11 or 15.

**17.** Method according to claim 16, wherein potato starch is produced which has a content of starch granules with a granule size of up to 15 $\mu$m, which is increased by at least 10% compared to starch from corresponding wildtype plants.

**18.** Regulatory region containing a nucleotide sequence selected from the group consisting of:

(a) nucleotide sequences comprising the nucleotides 1 to 169 of the nucleotide sequence depicted under SEQ ID NO:1; and
(b) the nucleotide sequence of the regulatory region which is contained in the insertion of the plasmid DSM 12425.

**Revendications**

**1.** Molécule d'acide nucléique codant un enzyme de ramification issu d'une bactérie du genre Neisseria, choisie dans le groupe constitué de

(a) molécules d'acides nucléiques qui codent une protéine qui comprend la séquence d'acides aminés indiquée en SEQ ID NO:2,
(b) molécules d'acides nucléiques qui comprennent la région codante indiquée en SEQ ID NO:1,
(c) molécules d'acides nucléiques qui codent une protéine comprenant la séquence d'acides aminés qui est codée par l'insertion dans le plasmide DSM 12425,
(d) molécules d'acides nucléiques qui comprennent la région codante contenue dans l'insertion du plasmide DSM 12425 pour un enzyme de ramification,
(e) molécules d'acides nucléiques qui codent un enzyme de ramification issu d'une bactérie du genre Neisseria, dont la séquence présente dans la région des cent premiers acides aminés une identité de séquence d'au moins 65 % relativement à la séquence d'acides aminés indiquée en SEQ ID NO:2,
(f) molécules d'acides nucléiques dont le brin complémentaire s'hybride avec une molécule d'acide nucléique selon (a), (b), (c), (d) et/ou (e) dans des conditions stringentes et qui codent un enzyme de ramification issu d'une bactérie du genre Neisseria ; et
(g) molécules d'acides nucléiques dont la séquence s'écarte de celle d'une molécule d'acide nucléique selon (f) par suite de la dégénérescence du code génétique.

**2.** Vecteur contenant une molécule d'acide nucléique selon la revendication 1.

**3.** Vecteur selon la revendication 2, pour lequel la molécule d'acide nucléique est liée selon l'orientation sens à des séquences régulatrices qui assurent la transcription dans des cellules procaryotiques ou eucaryotiques.

**4.** Cellule hôte, qui est transformée avec un vecteur selon la revendication 2 ou 3.

**5.** Procédé de préparation d'un enzyme de ramification issu d'une bactérie du genre Neisseria, dans lequel une cellule hôte selon la revendication 4 est cultivée dans des conditions qui permettent l'expression de la protéine, et la protéine est isolée des cellules obtenues par culture et/ou du milieu de culture.

**6.** Procédé de préparation d'un enzyme de ramification issu d'une bactérie du genre Neisseria, dans lequel la protéine est produite dans un système de transcription et traduction in vitro avec l'utilisation d'une molécule d'acide nucléique selon la revendication 1.

**7.** Enzyme de ramification codé par une molécule d'acide nucléique selon la revendication 1 ou obtenu par un procédé selon la revendication 5 ou 6.

**8.** Anticorps qui reconnaît spécifiquement une protéine selon la revendication 7.

**9.** Utilisation d'une protéine selon la revendication 7 pour la préparation d'a-1,4-glucanes α-1,6-ramifiés dans des systèmes in vitro.

**10.** Cellule végétale transgénique contenant une molécule d'acide nucléique selon la revendication 1, dans laquelle la molécule d'acide nucléique est liée à des séquences régulatrices qui assurent la transcription dans des cellules végétales.

**11.** Cellule végétale transgénique selon la revendication 10, dans laquelle la molécule d'acide nucléique est liée à une séquence qui code une séquence signal qui assure la localisation de la protéine codée dans les plastides des cellules.

**12.** Plante transgénique contenant des cellules végétales selon la revendication 10 ou 11.

**13.** Procédé de production d'une plante transgénique, dans lequel

(a) une cellule végétale est génétiquement modifiée par l'introduction d'une molécule d'acide nucléique selon la revendication 1 ou d'un vecteur selon la revendication 2 ou 3,
(b) une plante est régénérée à partir de la cellule produite selon l'étape (a); et
(c) d'autres plantes comprenant des cellules qui contiennent une molécule d'acide nucléique selon la revendication 1 ou un vecteur selon la revendication 2 ou 3 sont éventuellement produites à partir de la plante obtenue selon l'étape (b).

**14.** Parties récoltables de plantes selon la revendication 12 ou de plantes obtenues par un procédé selon la revendication 13, ces parties de plantes contenant des cellules végétales transgéniques selon la revendication 10 ou 11.

**15.** Parties de plantes transgéniques selon la revendication 10 ou 11, plante transgénique selon la revendication 12, procédé selon la revendication 13 et parties de plantes selon la revendication 14, la plante étant une pomme de terre et les cellules végétales et les parties de plantes étant issues de pomme de terre.

**16.** Procédé de production d'amidon, comprenant l'extraction d'amidon de plantes transgéniques selon la revendication 12 ou 15, de plantes transgéniques obtenues par un procédé selon la revendication 13 ou 15, de parties emmagasinant de l'amidon d'une telle plante ou de cellules végétales selon la revendication 10, 11 ou 15.

**17.** Procédé selon la revendication 16, dans lequel est produite de la fécule de pomme de terre qui présente un pourcentage de grains de fécule de grandeur allant jusqu'à 15 μm augmenté d'au moins 10 % comparativement à une fécule issue de plants correspondants de type sauvage.

**18.** Région régulatrice contenant une séquence de nucléotides choisie dans le groupe constitué de

(a) séquences nucléotidiques qui comprennent les nucléotides 1 à 169 de la séquence nucléotidique représentée en SEQ ID NO:1; et
(b) la séquence nucléotidique de la région régulatrice qui est contenue dans l'insertion du plasmide DSM 12425.

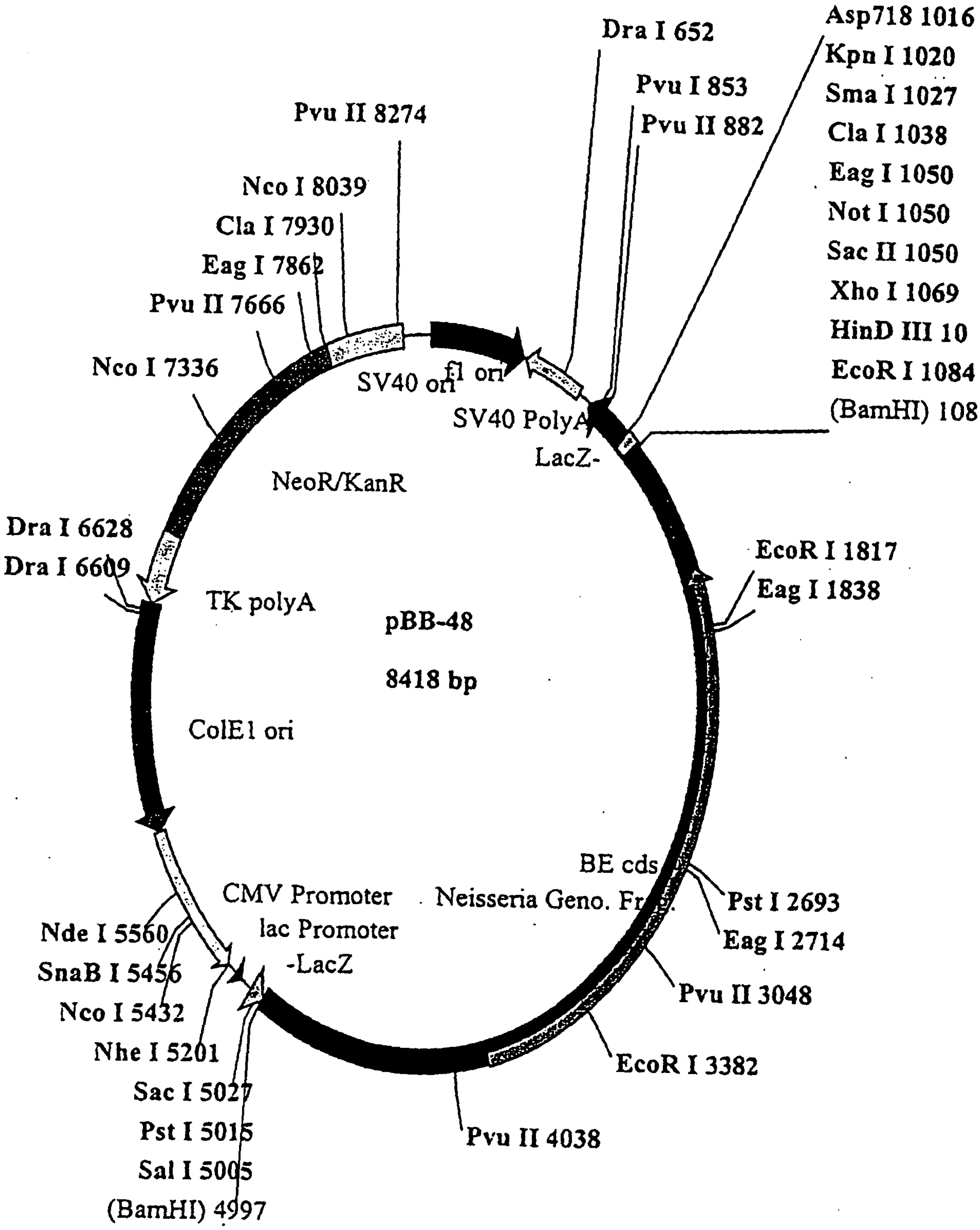

Dra I 652
Asp718 1016
Kpn I 1020
Sma I 1027
Cla I 1038
Eag I 1050
Not I 1050
Sac II 1050
Xho I 1069
HinD III 10
EcoR I 1084
(BamHI) 108
Pvu I 853
Pvu II 882
Pvu II 8274
Nco I 8039
Cla I 7930
Eag I 7862
Pvu II 7666
Nco I 7336
SV40 ori
f1 ori
SV40 PolyA
LacZ-
NeoR/KanR
Dra I 6628
Dra I 6609
TK polyA
pBB-48
8418 bp
EcoR I 1817
Eag I 1838
ColE1 ori
BE cds
Neisseria Geno. Fr.
CMV Promoter
lac Promoter
-LacZ
Pst I 2693
Eag I 2714
Pvu II 3048
EcoR I 3382
Pvu II 4038
Nde I 5560
SnaB I 5456
Nco I 5432
Nhe I 5201
Sac I 5027
Pst I 5015
Sal I 5005
(BamHI) 4997


Figur 1

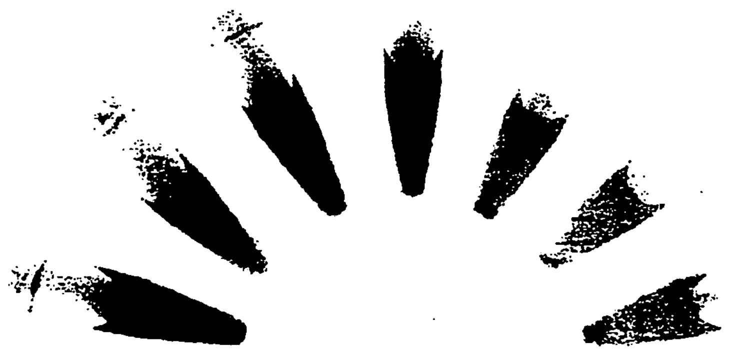

Figur 2

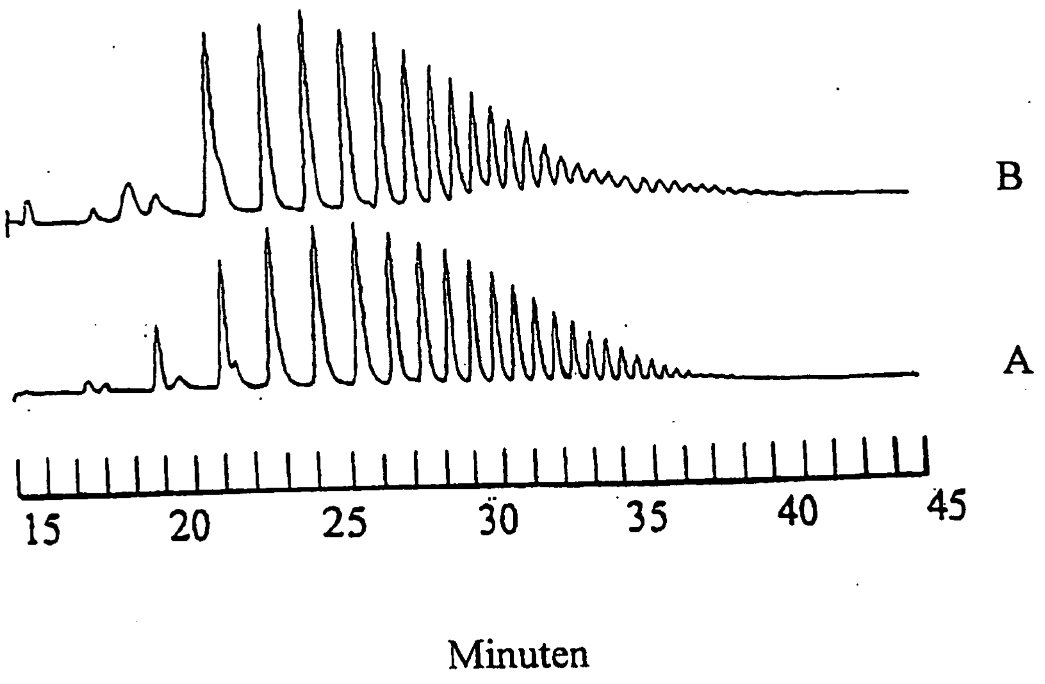


Figur 3

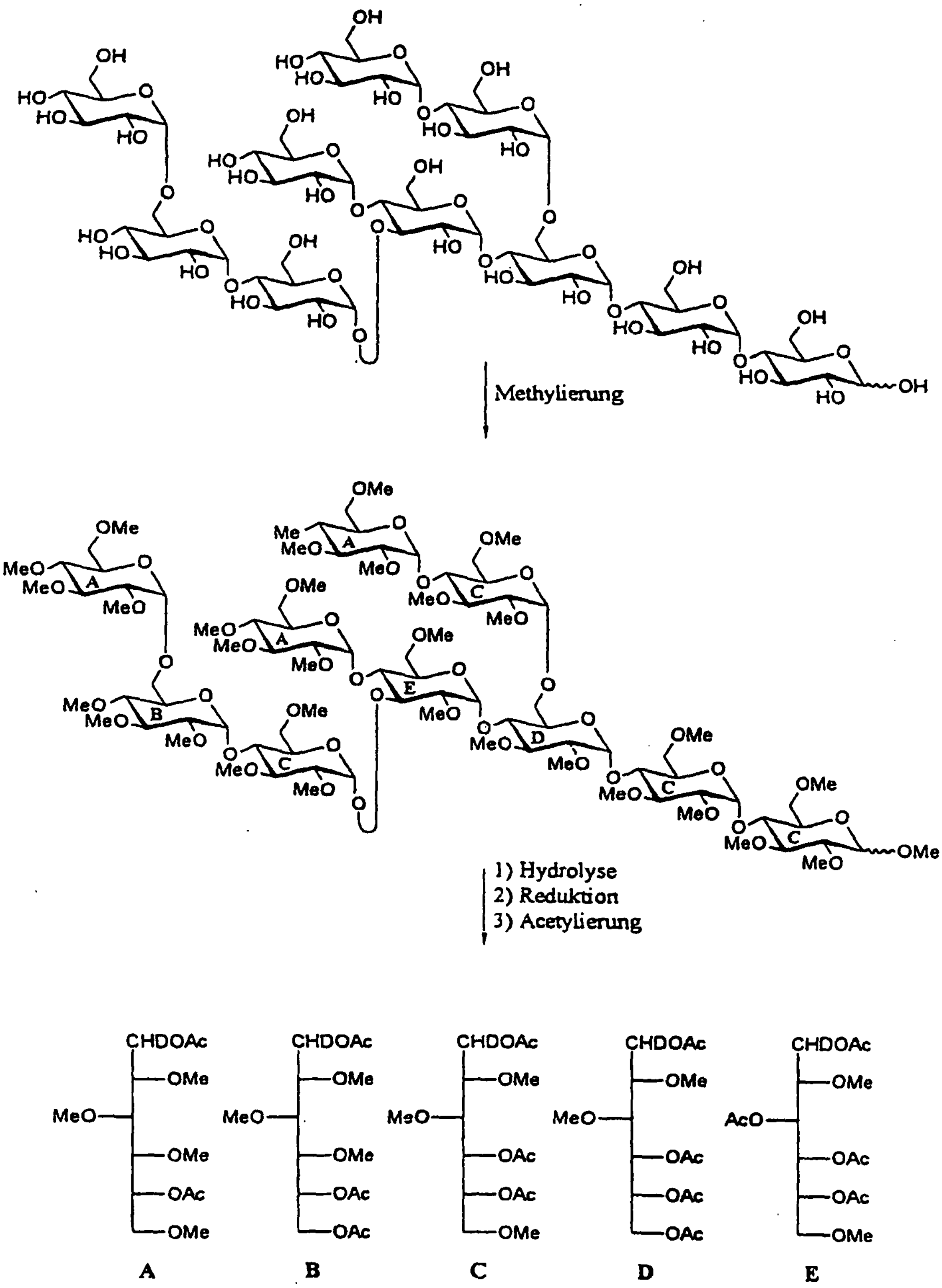


A B C D E

Figur 4

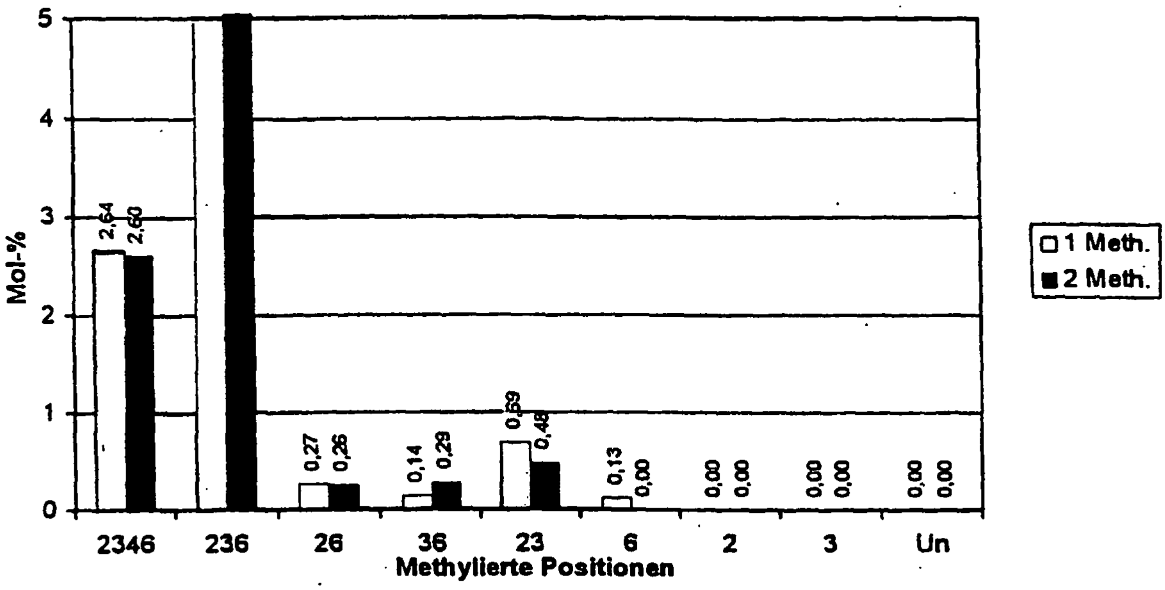
5
4
3
2
1
0
Mol-%
2,64
2,60
0,27
0,26
0,14
0,29
0,69
0,48
0,13
0,00
0,00
0,00
0,00
0,00
0,00
0,00
2346
236
26
36
23
6
2
3
Un
Methylierte Positionen
1 Meth.
2 Meth.

Figur 5

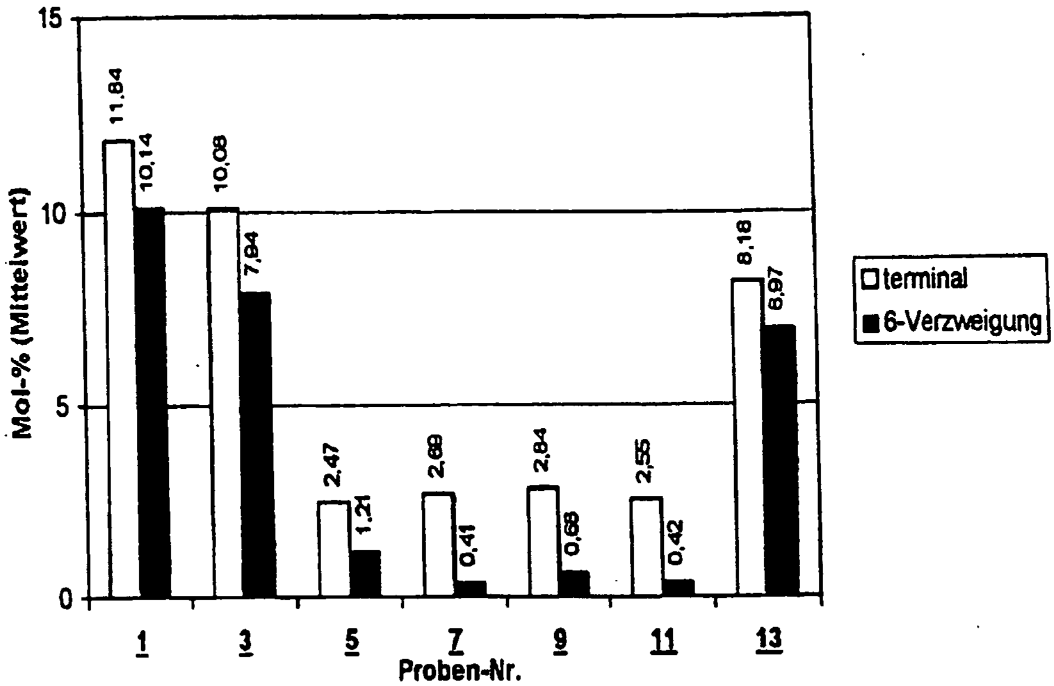
15
10
5
0
Mol-% (Mittelwert)
11,84
10,14
10,08
7,94
2,47
1,21
2,69
0,41
2,84
0,68
2,55
0,42
8,18
6,97
1
3
5
7
9
11
13
Proben-Nr.
terminal
6-Verzweigung

Figur 6

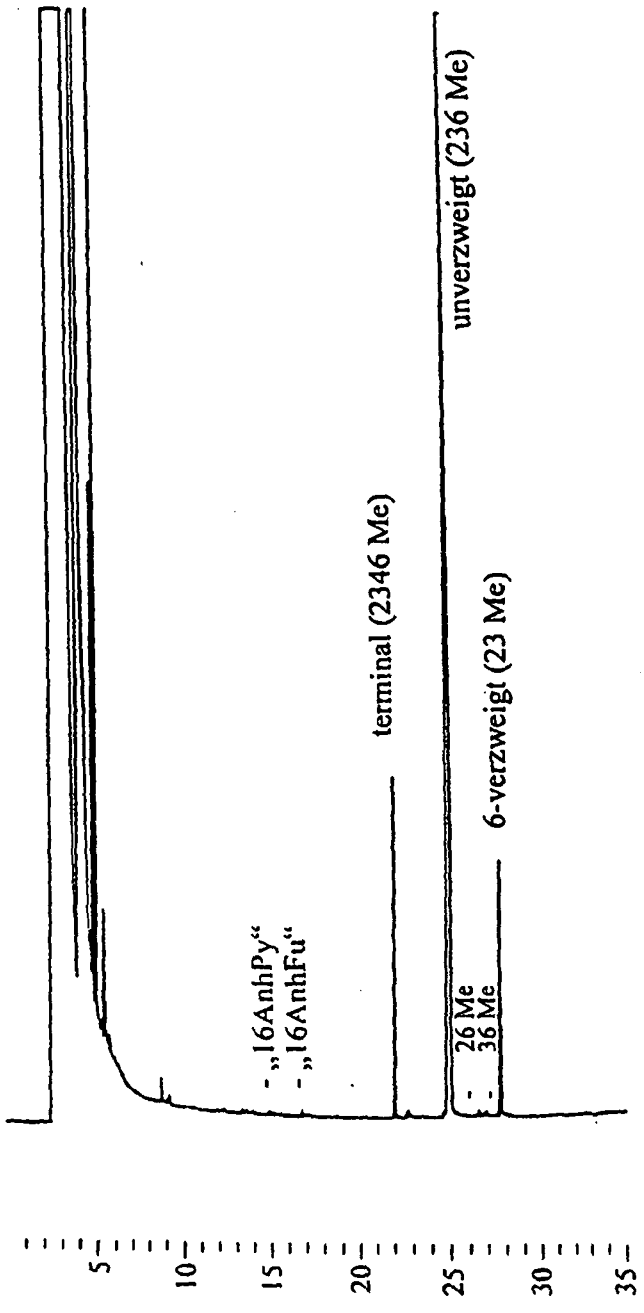

unverzweigt (236 Me)
terminal (2346 Me)
6-verzweigt (23 Me)
„16AnhPy“
„16AnhFu“
26 Me
36 Me
5
10
15
20
25
30
35


Figur 7

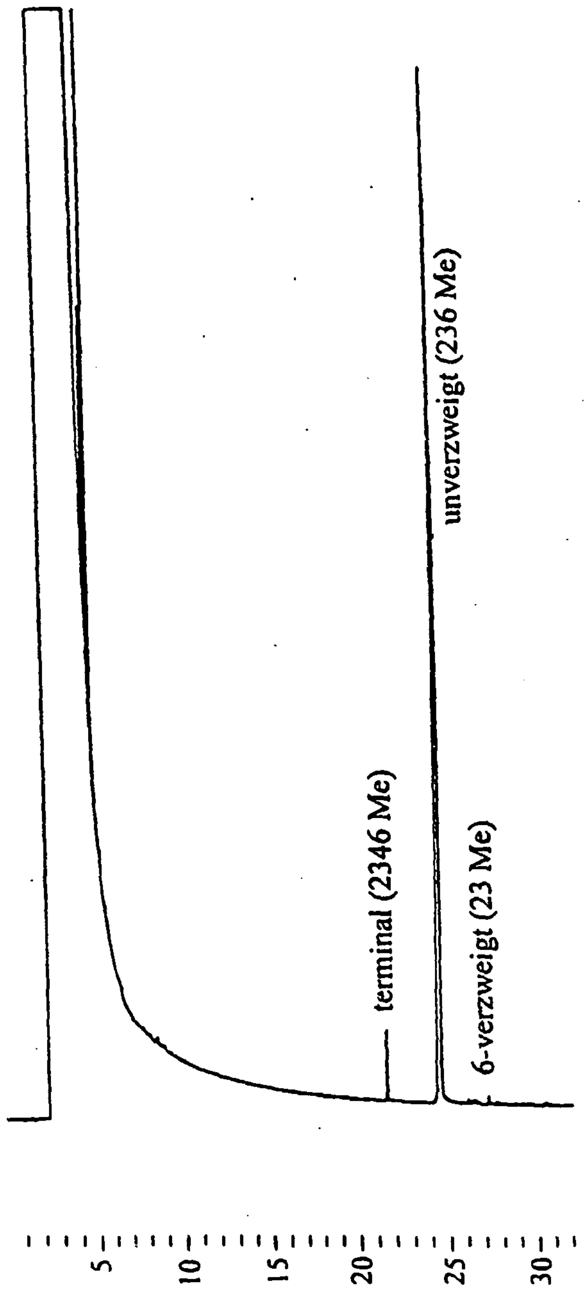
unverzweigt (236 Me)
terminal (2346 Me)
6-verzweigt (23 Me)
5
10
15
20
25
30

Figur 8

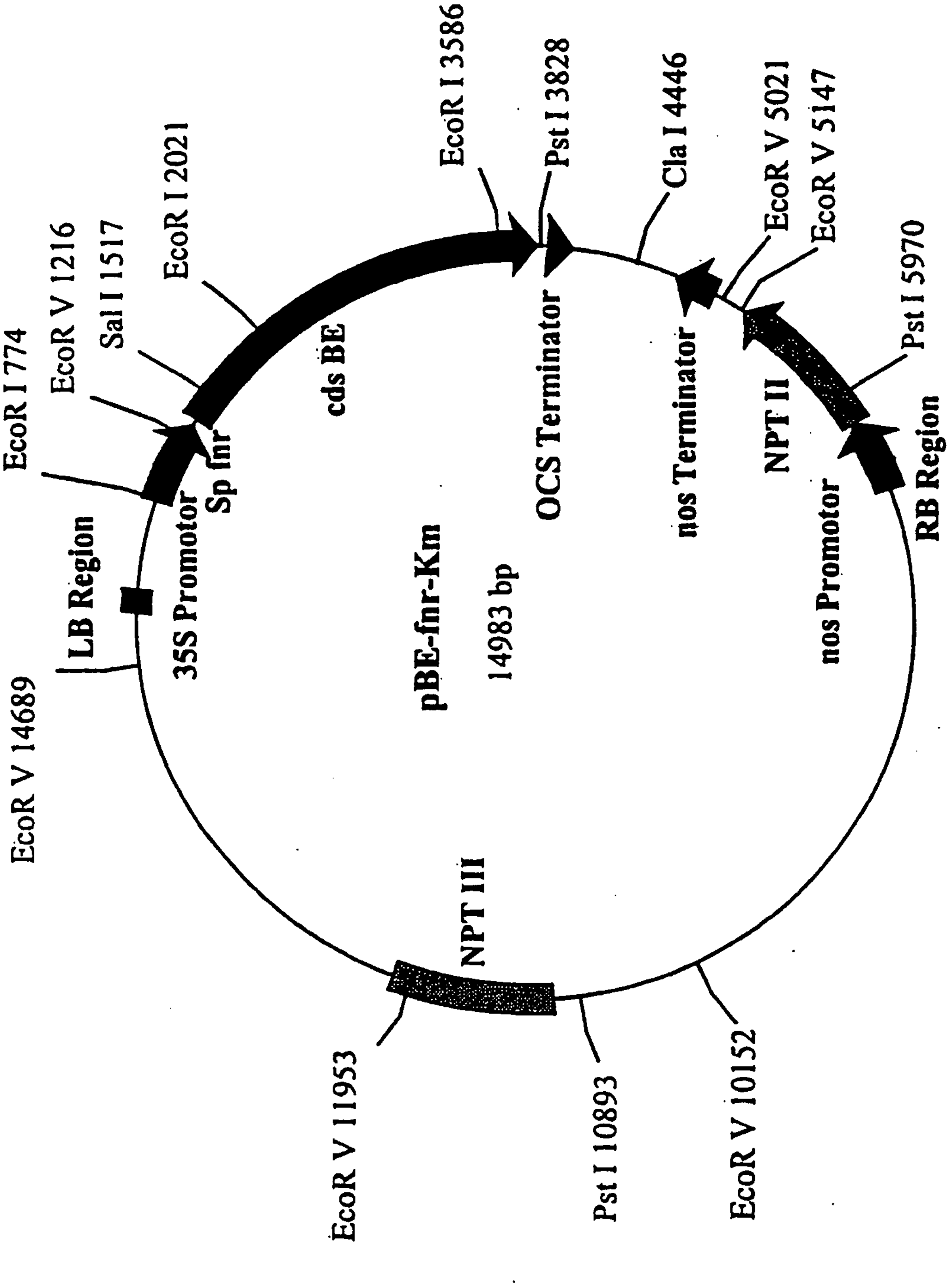

pBE-fnr-Km
14983 bp
EcoR I 3586
Pst I 3828
Cla I 4446
EcoR V 5021
EcoR V 5147
Pst I 5970
EcoR I 2021
Sal I 1517
EcoR V 1216
EcoR I 774
LB Region
EcoR V 14689
35S Promotor
Sp fnr
cds BE
OCS Terminator
nos Terminator
NPT II
nos Promotor
RB Region
NPT III
EcoR V 11953
Pst I 10893
EcoR V 10152


Figur 9

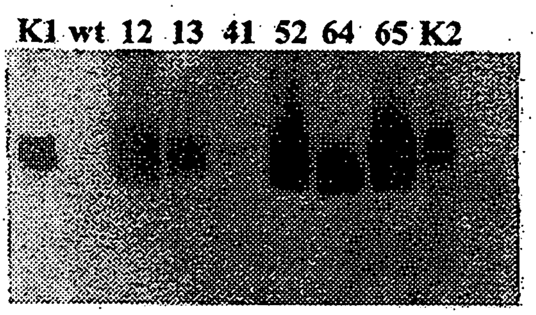


Figur 10

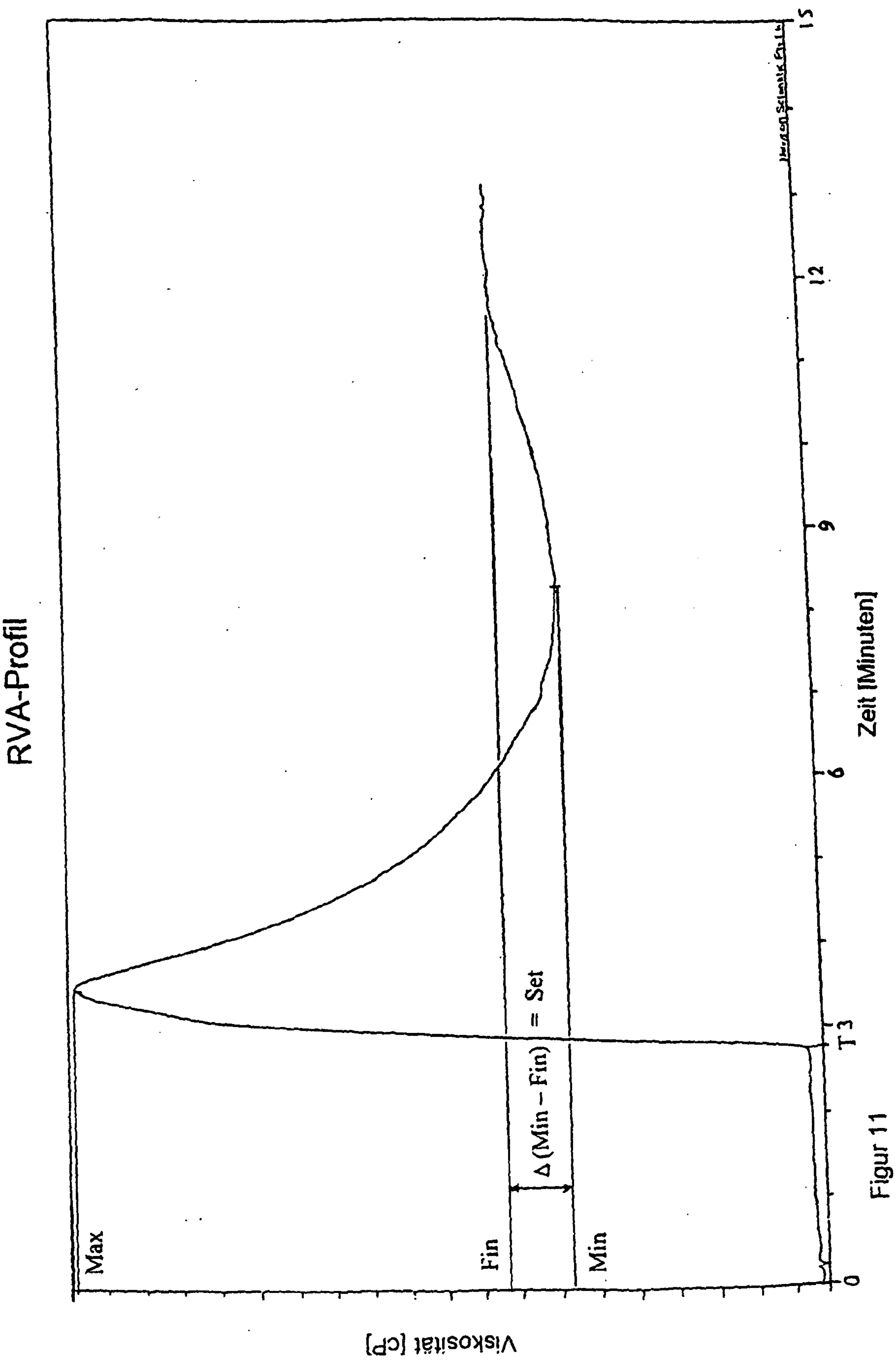
RVA-Profil
Max
Fin
Min
Δ (Min – Fin) = Set
0
T3
6
9
12
15
Zeit [Minuten]
Viskosität [cP]

Figur 11

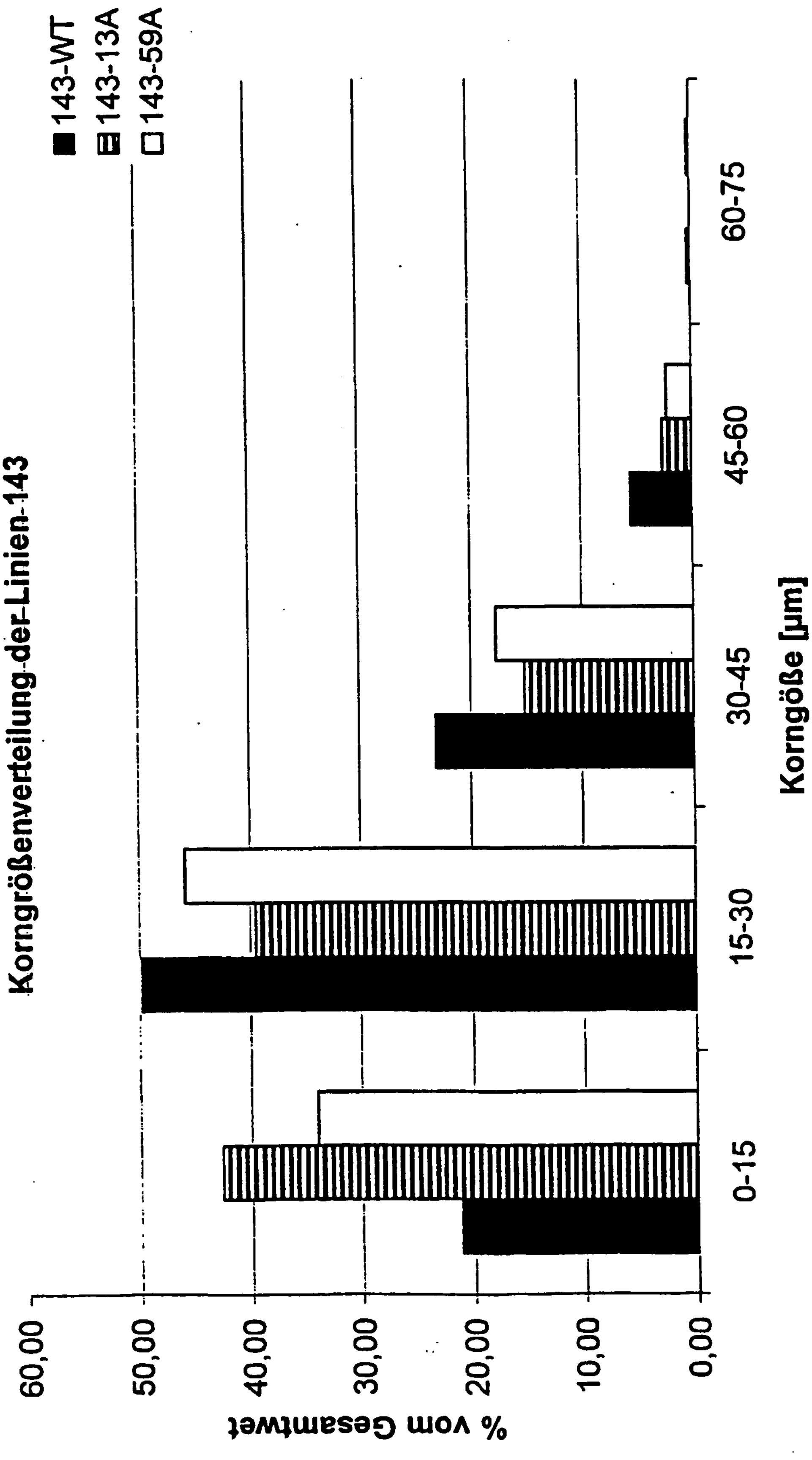
Korngrößenverteilung der Linien 143
143-WT
143-13A
143-59A
60,00
50,00
40,00
30,00
20,00
10,00
0,00
% vom Gesamtwet
0-15
15-30
30-45
45-60
60-75
Korngöße [µm]

Figur 12

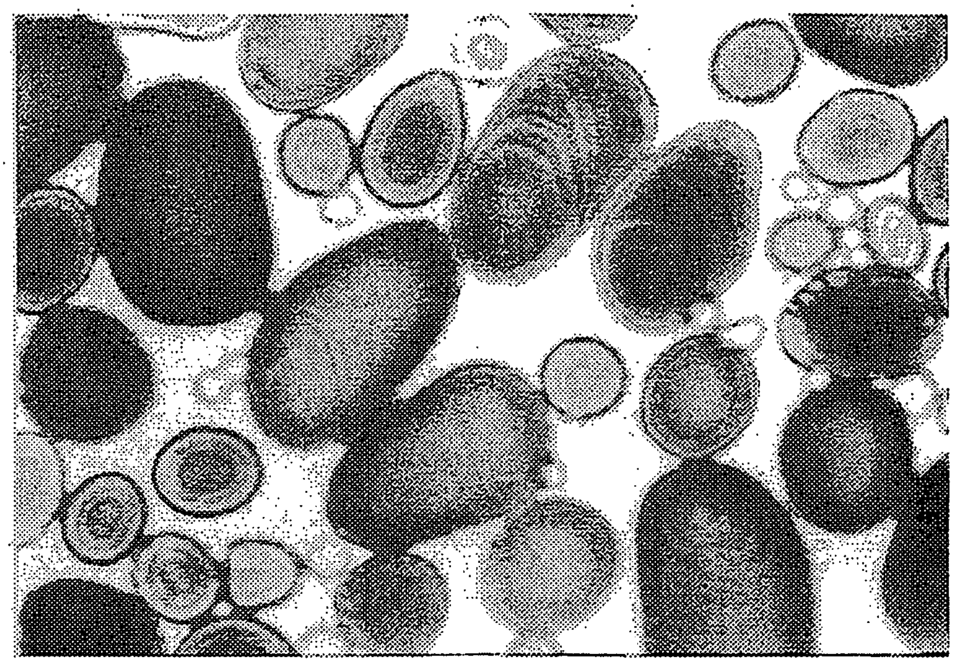

143-WT (1000:1)

Figur 13a

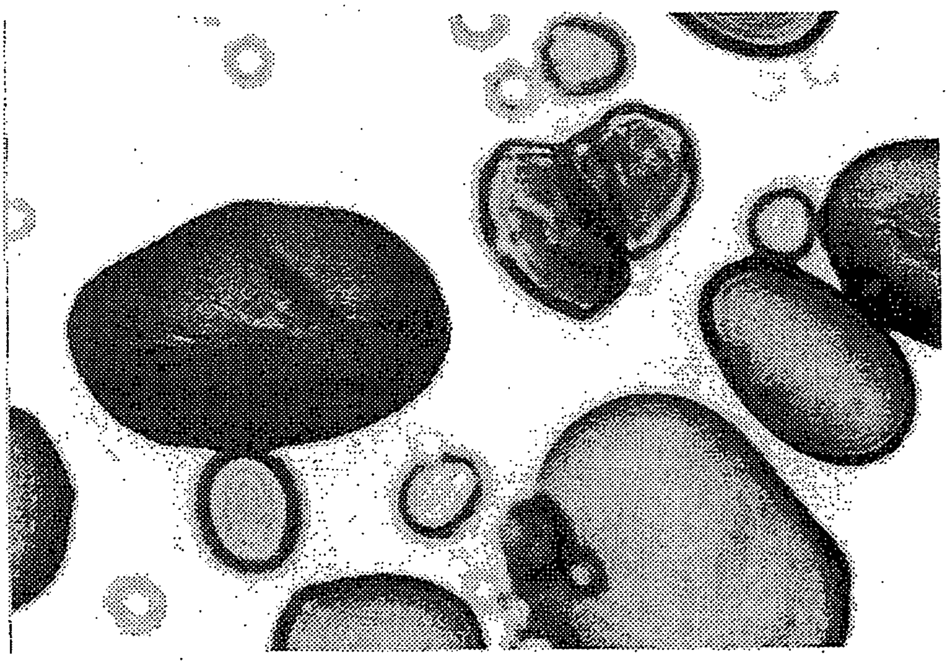

143-11A (1000:1)

Figur 13b

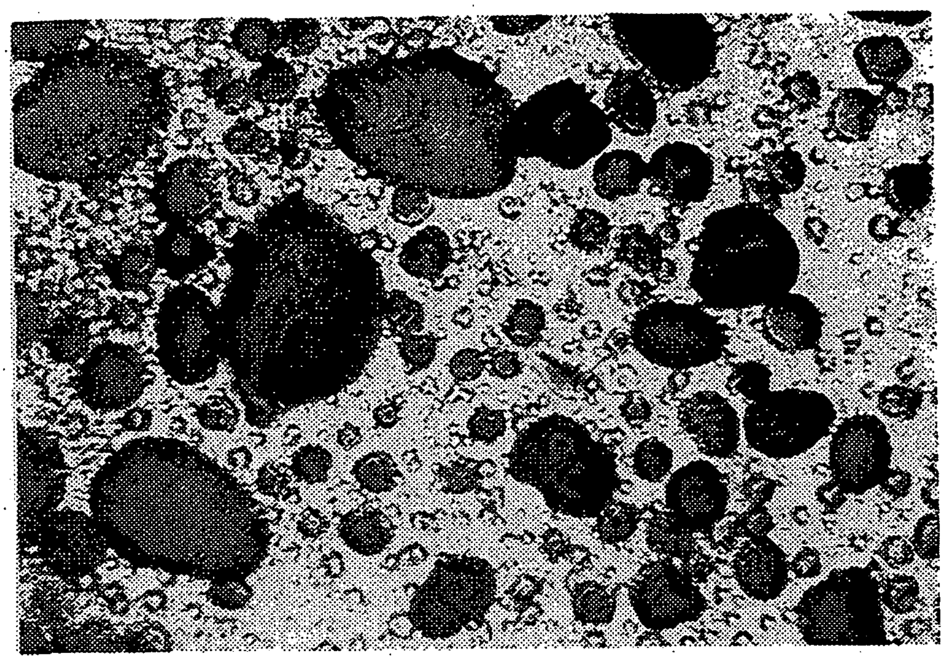

143-13A (1000:1)

**Figur 13c**

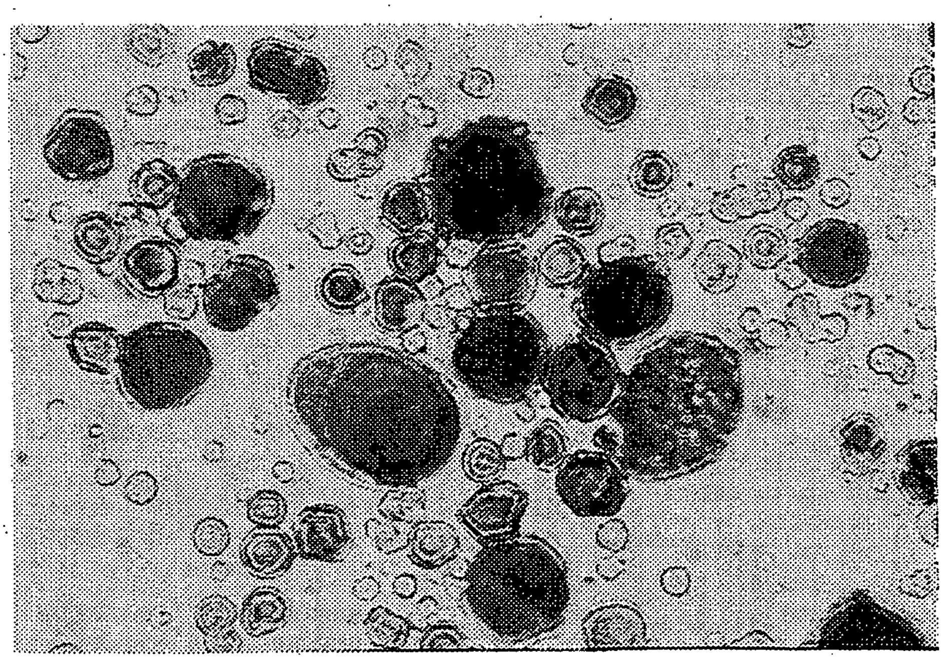

143-59A (1000:1)

**Figur 13d**

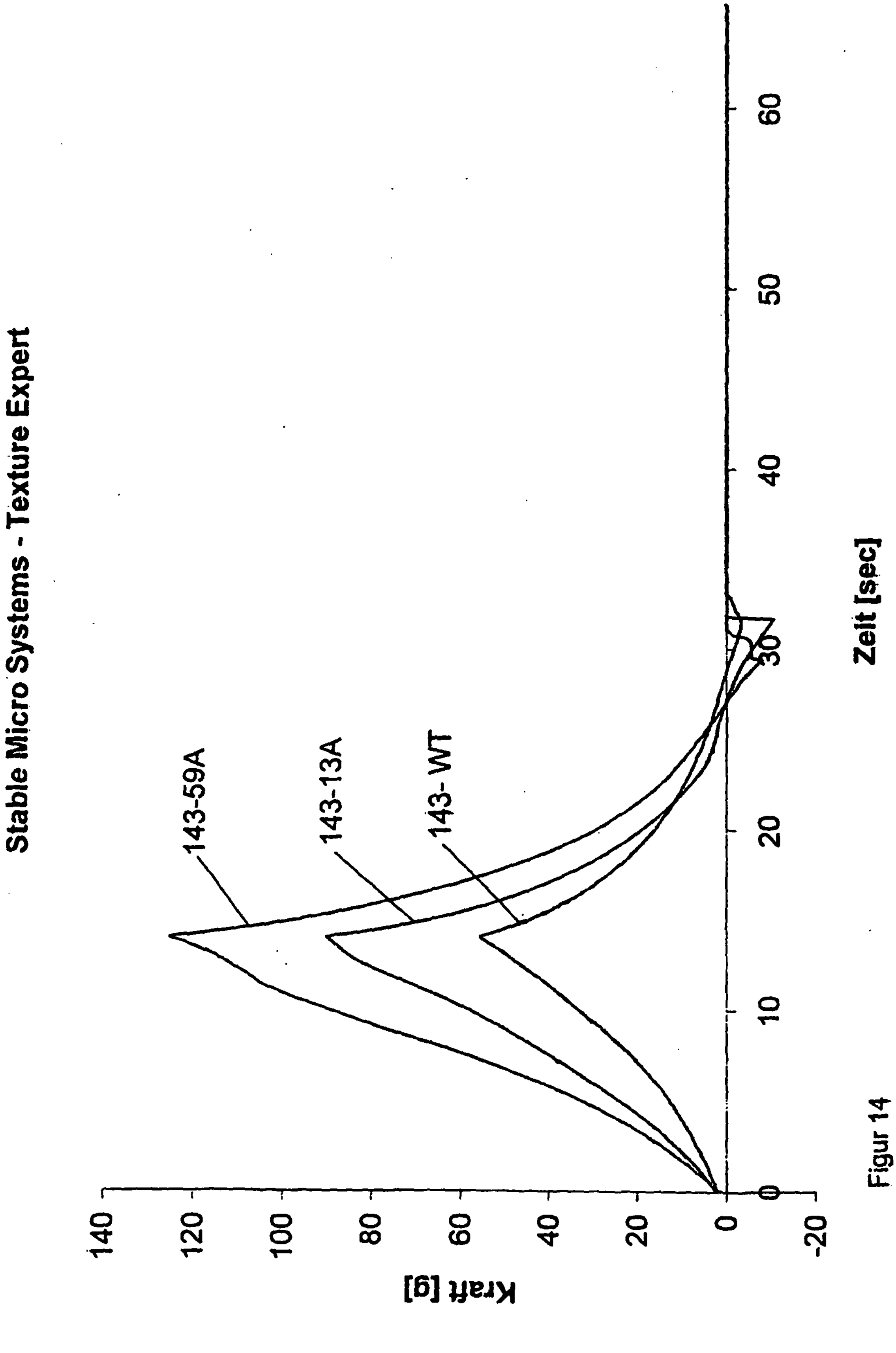
Stable Micro Systems - Texture Expert
143-59A
143-13A
143- WT
Kraft [g]
140
120
100
80
60
40
20
0
-20
0
10
20
30
40
50
60
Zeit [sec]

Figur 14

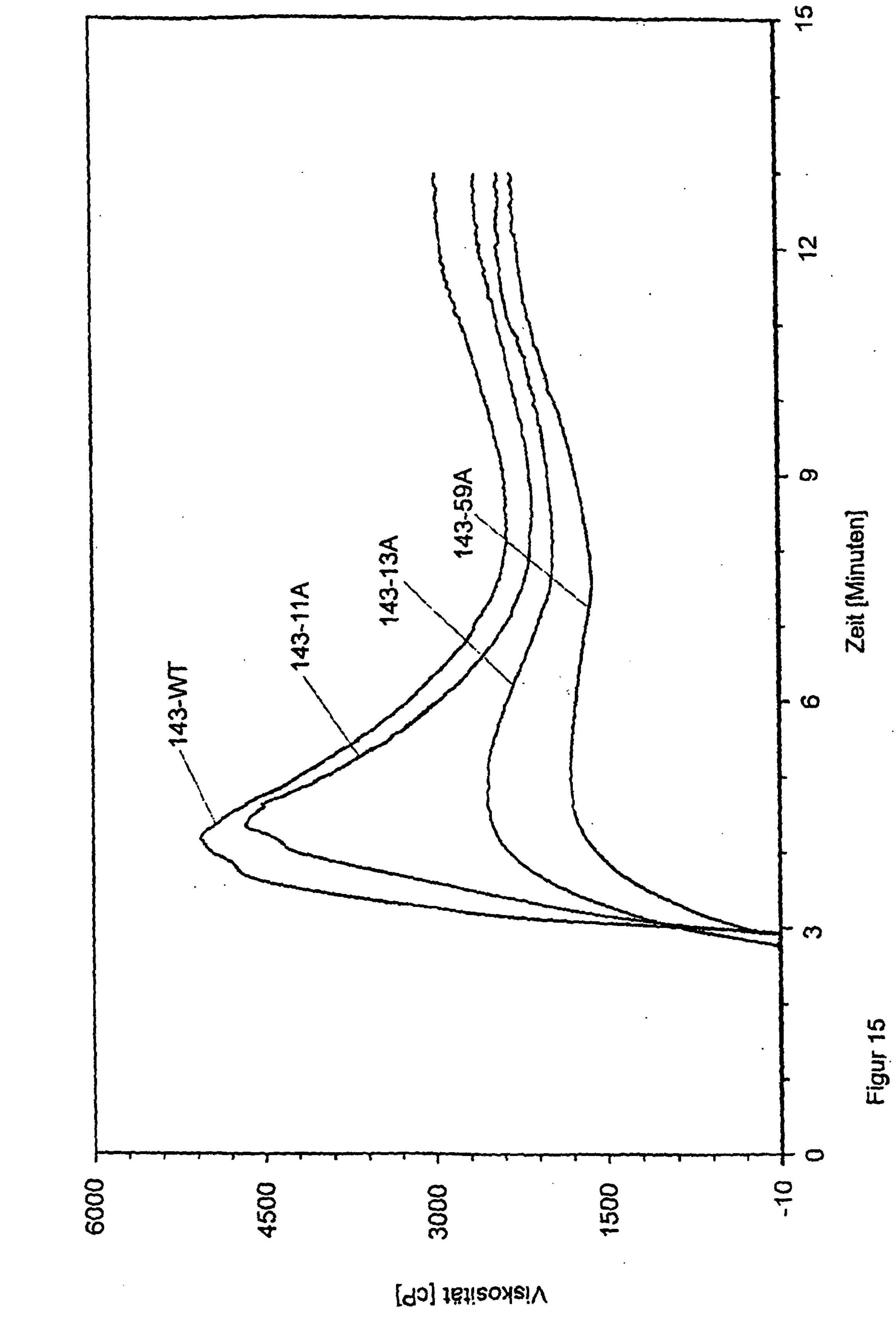
143-WT
143-11A
143-13A
143-59A
Viskosität [cP]
6000
4500
3000
1500
-10
0
3
6
9
12
15
Zeit [Minuten]

Figur 15

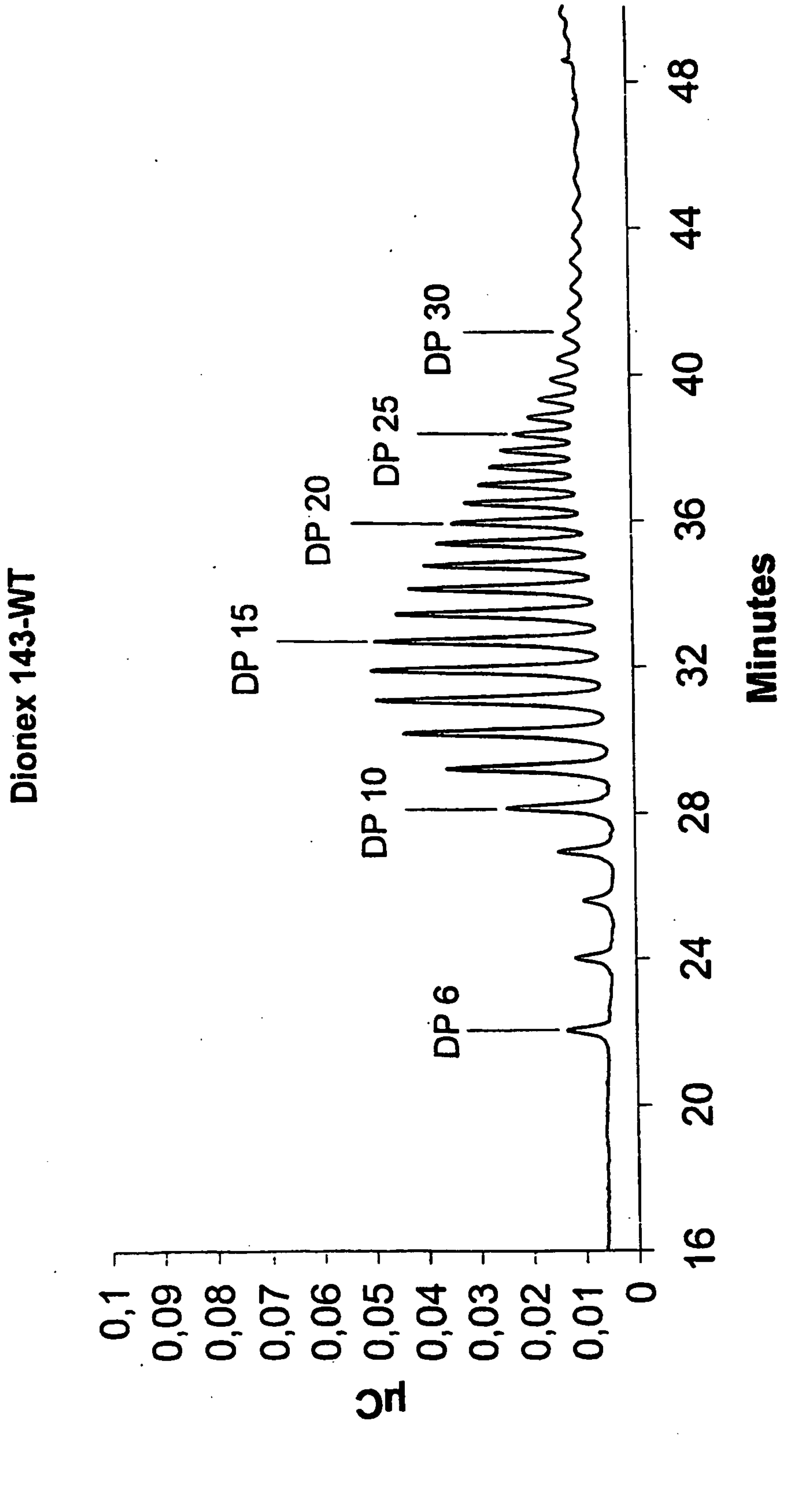
Dionex 143-WT
µC
0,1
0,09
0,08
0,07
0,06
0,05
0,04
0,03
0,02
0,01
0
DP 6
DP 10
DP 15
DP 20
DP 25
DP 30
16
20
24
28
32
36
40
44
48
Minutes

Figur 16

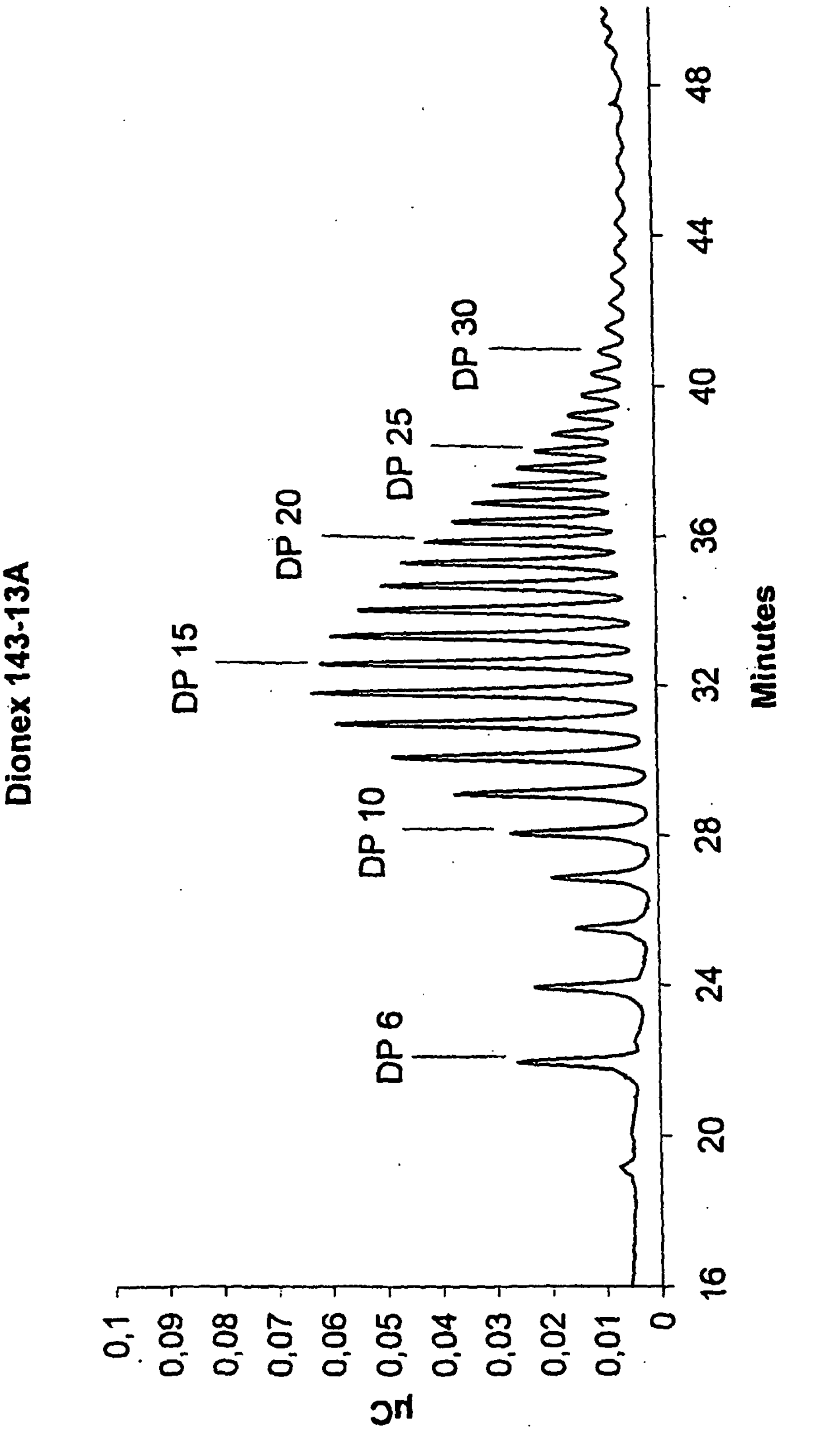
Dionex 143-13A
DP 6
DP 10
DP 15
DP 20
DP 25
DP 30
μC
0,1
0,09
0,08
0,07
0,06
0,05
0,04
0,03
0,02
0,01
0
16
20
24
28
32
36
40
44
48
Minutes

Figur 17

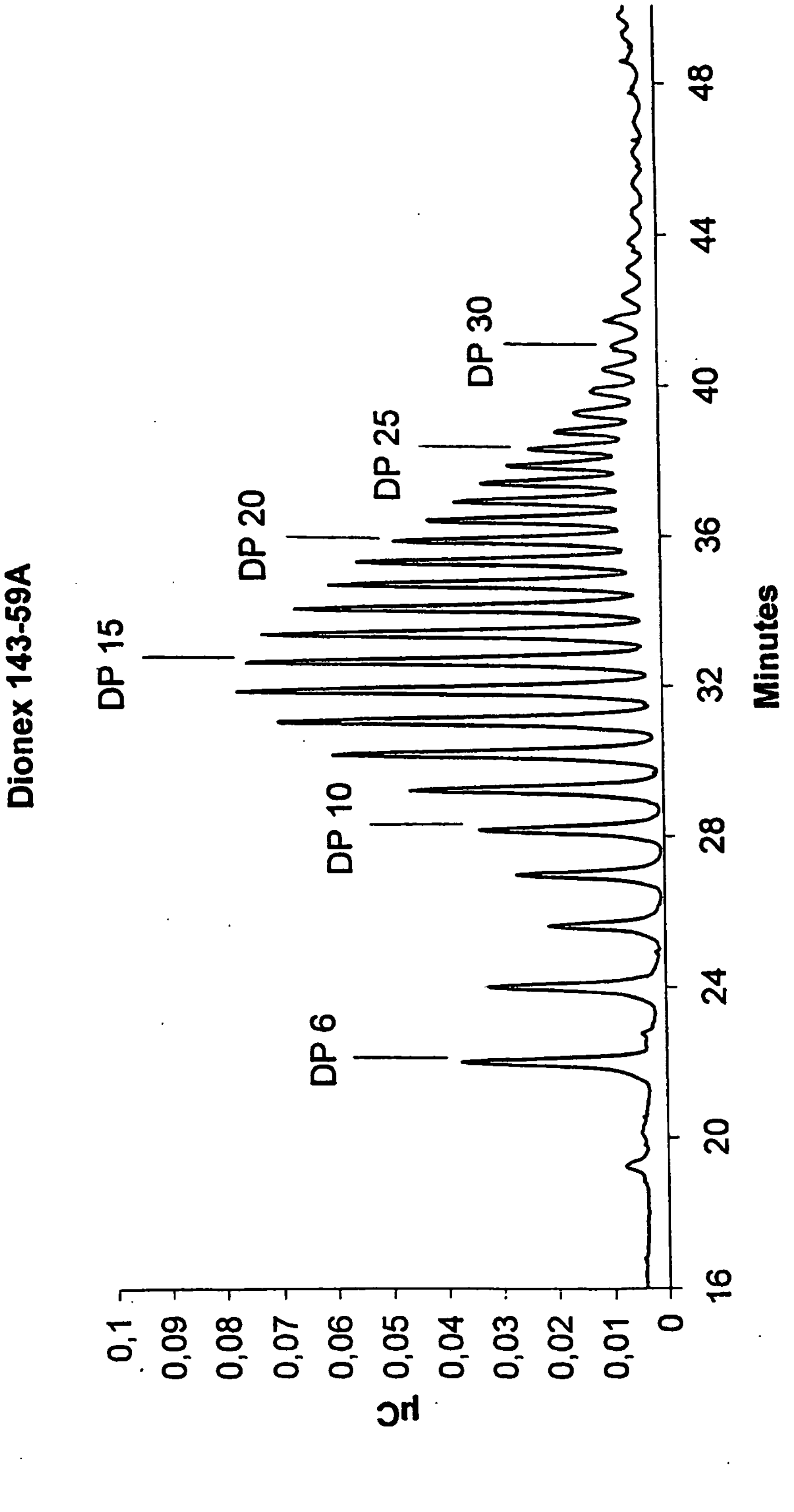
Dionex 143-59A
DP 6
DP 10
DP 15
DP 20
DP 25
DP 30
μC
0,1
0,09
0,08
0,07
0,06
0,05
0,04
0,03
0,02
0,01
0
16
20
24
28
32
36
40
44
48
Minutes

Figur 18

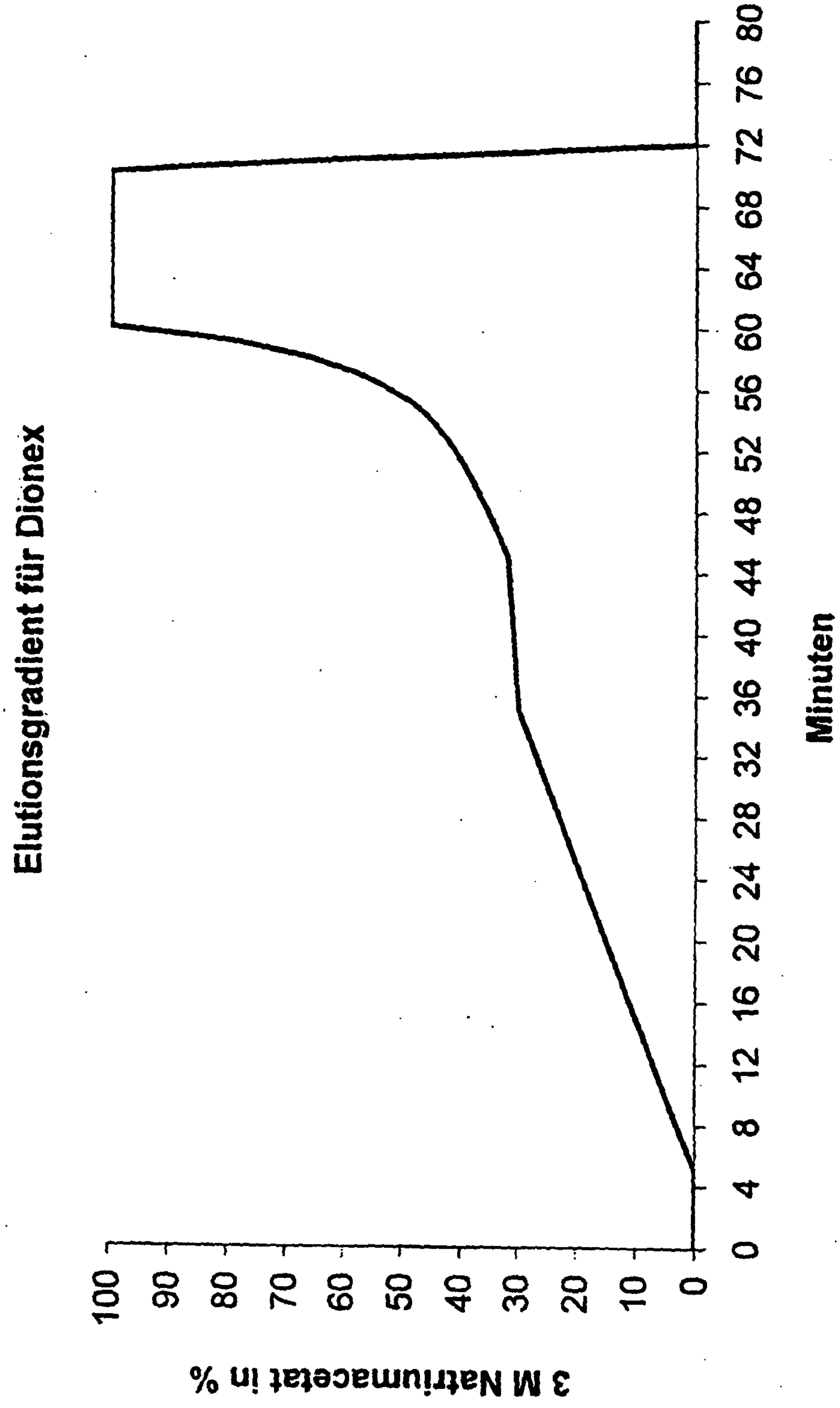
Elutionsgradient für Dionex
3 M Natriumacetat in %
100
90
80
70
60
50
40
30
20
10
0
0 4 8 12 16 20 24 28 32 36 40 44 48 52 56 60 64 68 72 76 80
Minuten

Figur 19

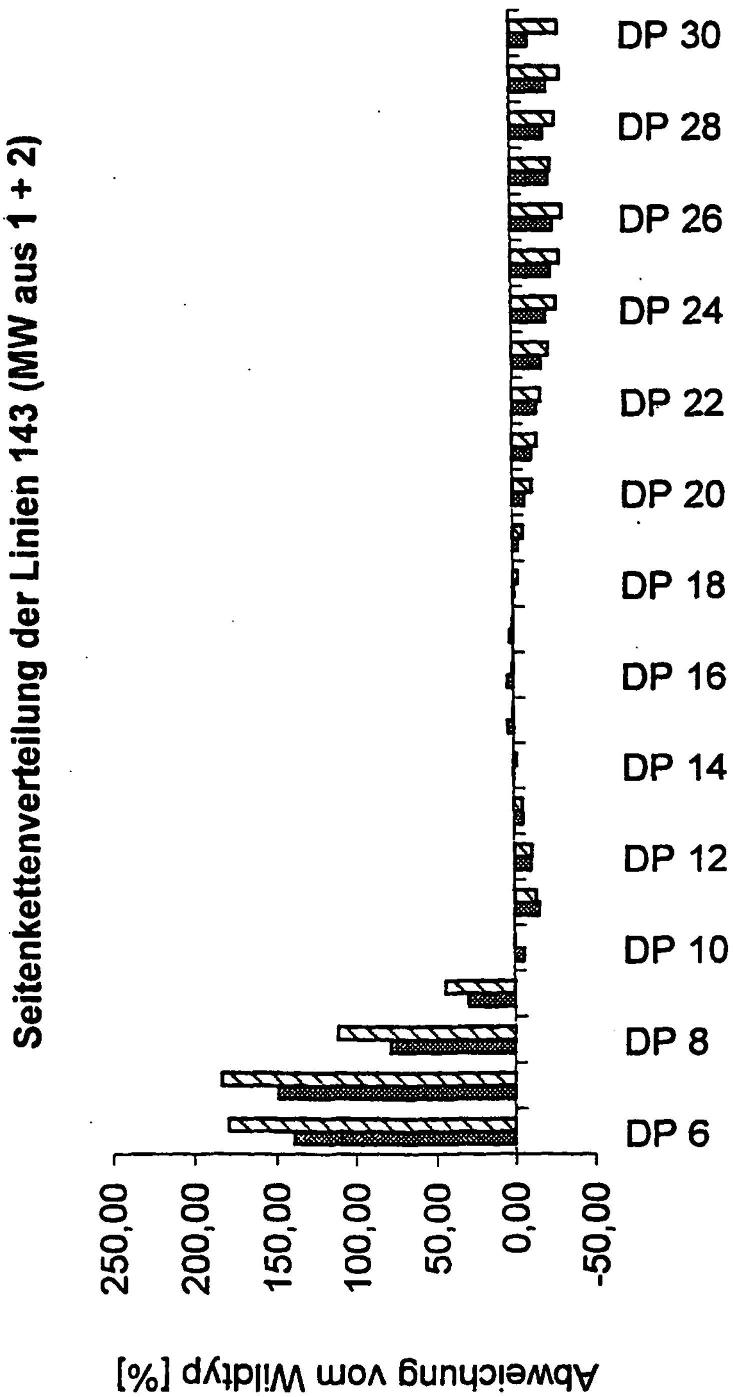
Seitenkettenverteilung der Linien 143 (MW aus 1 + 2)
Abweichung vom Wildtyp [%]
250,00
200,00
150,00
100,00
50,00
0,00
-50,00
DP 6
DP 8
DP 10
DP 12
DP 14
DP 16
DP 18
DP 20
DP 22
DP 24
DP 26
DP 28
DP 30

Figur 20

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4454161 A
- EP 0690170 A1
- US 5352605 A
- WO 9401571 A
- US 5614399 A
- WO 9307279 A
- US 5656496 A
- US 5639952 A
- US 5034322 A
- US 4962028 A
- WO 9101373 A
- EP 120516 A
- WO 9506128 A
- EP 0513849 A
- EP 0465875 A
- EP 292435 A
- WO 9531553 A


### In der Beschreibung aufgeführte Nicht-Patentliteratur


- **Voet ; Voet.** Biochemistry. John Wiley & Sons, 1990
- **Shewmaker et al.** *Plant. Physiol.,* 1994, vol. 104, 1159-1166
- **Jacobsen et al.** *Euphytica,* 1989, vol. 44, 43-48
- **Kortstee et al.** *Plant J.,* 1996, vol. 10, 83-90
- **D. J. Manners.** Advances in Carbohydrate Chemistry. Academic Press, 1957, 261-298
- **Geddes et al.** *Carbohydr. Res.,* 1994, vol. 261, 79-89
- **Bell et al.** *Biochem. J.,* 1934, vol. 28, 882
- **Bueding ; Orrell.** *J. Biol. Chem.,* 1961, vol. 236, 2854
- **Borovsky et al.** *Eur. J. Biochem.,* 1975, vol. 59, 615-625
- **Boyer ; Preiss.** *Biochemistry,* 1977, vol. 16, 3693-3699
- **Rumbak et al.** *J. Bacteriol.,* 1991, vol. 173, 6732-6741
- **Illingwort et al.** *Proc. Nat. Acad. Sci. USA,* 1961, vol. 47, 469-478
- **Mais. Doi.** *Biochimica et Biophysica Acta,* 1969, vol. 184, 477-485
- **Parodi et al.** *Arch. Biochem. Biophys.,* 1969, vol. 132, 11-117
- **Büttcher et al.** *J. Bacteriol.,* 1997, vol. 179, 3324-3330
- **Sambrock et al.** Molecular Cloning, A Laboratory Manual. Cold Spring Harbor Laboratory Press, 1989
- **Sambrook et al.** Molecular Cloning, A Laboratory Manual. Cold Spring Harbor Laboratory Press, 1989
- *Methods in Enzymology,* 1987, vol. 153, 383-516
- **Bitter et al.** *Methods in Enzymology,* 1987, vol. 153, 516-544
- **Chong et al.** *Gene,* 1997, vol. 192, 271-281
- **Hopp et al.** *Bio/Technology,* 1988, vol. 6, 1204-1210
- **Sassenfeld.** *Trends Biotechnol.,* 1990, vol. 8, 88-93
- **Fiedler et al.** *J. Mol. Biol.,* 1996, vol. 256, 279-291
- **Schlegel.** Allgemeine Mikrobiologie. Georg Thieme Verlag, 1985, 1-2
- **Odell et al.** *Nature,* 1985, vol. 313, 810-812
- **Stockhaus et al.** *Proc. Natl. Acad. Sci. USA,* 1987, vol. 84, 7943-7947
- **Stockhaus et al.** *EMBO J.,* 1989, vol. 8, 2445-2451
- **Bansal et al.** *Proc. Natl. Acad. Sci. USA,* 1992, vol. 89, 3654-3658
- **Rocha-Sosa et al.** *EMBO J.,* 1989, vol. 8, 23-29
- **Fiedler et al.** *Plant Mol. Biol.,* 1993, vol. 22, 669-679
- **Bäumlein et al.** *Mol Gen. Genet.,* 1991, vol. 225, 459-467
- **Leisy et al.** *Plant Mol. Biol.,* 1990, vol. 14, 41-50
- **Zheng et al.** *Plant J.,* 1993, vol. 4, 357-366
- **Pedersen et al.** *Cell,* 1982, vol. 29, 1015-1026
- **Quatroccio et al.** *Plant Mol. Biol.,* 1990, vol. 15, 81-93
- **Werr et al.** *EMBO J.,* 1985, vol. 4, 1373-1380
- **Gielen et al.** *EMBO J.,* 1989, vol. 8, 23-29
- The Binary Plant Vector System. **Hoekema.** Crit. Rev. Plant. Sci. Offsetdrukkerij Kanters B.V, 1985, vol. 4, 1-46
- **An.** *EMBO J.,* 1985, vol. 4, 277-287

- Transgenic plants. **Willmitzer, L.** Biotechnology, A Multi-Volume Comprehensive Treatise. VCH, 1993, vol. 2, 627-659
- **B. Lusardi.** *Plant J.,* 1994, vol. 5, 571-582
- **Paszkowski.** *Biotechnology,* 1992, vol. 24, 387-392
- **Chan.** *Plant Mol. Biol.,* 1993, vol. 22, 491-506
- **Hiei.** *Plant J.,* 1994, vol. 6, 271-282
- **Bytebier.** *Proc. Natl. Acad. Sci. USA,* 1987, vol. 84, 5345-5349
- **Raineri.** *Bio/Technology,* 1990, vol. 8, 33-38
- **Gould.** *Plant Physiol.,* 1991, vol. 95, 426-434
- **Mooney.** *Plant, Cell Tiss. & Org. Cult.,* 1991, vol. 25, 209-218
- **Li.** *Plant Mol. Biol.,* 1992, vol. 20, 1037-1048
- **Jähne.** *Euphytica,* 1995, vol. 85, 35-44
- **Maheshwari.** *Critical Reviews in Plant Science,* 1995, vol. 14 (2), 149-178
- **Fromm et al.** *Biotechnology,* 1990, vol. 8, 833-844
- **Gordon-Kamm et al.** *Plant Cell,* 1990, vol. 2, 603-618
- **Koziel et al.** *Biotechnology,* 1993, vol. 11, 194-200
- **Moroc et al.** *Theor. Appl. Genet.,* 1990, vol. 80, 721-726
- **Krens et al.** *Nature,* 1982, vol. 296, 72-74
- **Nehra et al.** *Plant J.,* 1994, vol. 5, 285-297
- **Braun.** *EMBO J.,* 1992, vol. 11, 3219-3227
- **Sonnewald.** *Plant J.,* 1991, vol. 1, 95-106
- **Rocha-Sosa.** *EMBO J.,* 1989, vol. 8, 23-29
- **Jansen et al.** *Current Genetics,* 1988, vol. 13, 517-522
- **Klösgen et al.** *Mol Gen Genet.,* 1989, vol. 217, 155-161
- **Wolter et al.** *Proc. Natl. Acad. Sci. USA,* 1988, vol. 85, 846-850
- **Nawrath et al.** *Proc. Natl. Acad. Sci. USA,* 1994, vol. 91, 12760-12764
- **Gallardo et al.** *Planta,* 1995, vol. 197, 324-332
- **Creissen et al.** *Plant J.,* 1995, vol. 8, 167-175
- **Lloyd et al.** *Biochem. J.,* 1999, vol. 338, 515-521
- Starch: Chemistry and Technology. Academic Press Inc. London Ltd, 1994, 412-468
- **Watson.** Tapioca-, Arrowroot- und Sagostärken: Herstellung. 469-479
- **Corbishley ; Miller.** Kartoffelstärke: Herstellung und Verwendungen. 479-490
- **Mitch.** Weizenstärke: Herstellung, Modifizierung und Verwendungen. 491-506
- **Knight ; Oson.** Reisstärke: Herstellung und Verwendungen. 507-528
- **Rohmer ; Klem ; Eckhoff et al.** Maisstärke. *Cereal Chem.,* 1996, vol. 73, 54-57
- **MacKenzie et al.** *Can. J. Microbiol.,* 1978, vol. 24, 357-362
- **Okada ; Hehre.** *J. Biol. Chem.,* 1974, vol. 249, 126-135
- **MacKenzie et al.** *Can. J. Microbiol.,* 1977, vol. 23, 1303-1307
- **Riou et al.** *Can. J. Microbiol.,* 1986, vol. 32, 909-911
- *Methods in Enzymology,* 1987, vol. 153, 385-516
- **Sawers et al.** *Applied Microbiology and Biotechnology,* 1996, vol. 46, 1-9
- **Billman-Jacobe.** *Current Opinion in Biotechnology,* 1996, vol. 7, 500-504
- **Hockney.** *Trends in Biotechnology,* 1994, vol. 12, 456-463
- **Griffiths et al.** *Methods in Molecular Biology,* 1997, vol. 75, 427-440
- **Studier et al.** *Methods in Enzymology,* 1990, vol. 185, 60-89
- **DeBoer et al.** Promotors, Structure and Function. 1982, 462-481
- **DeBoer et al.** *Proc. Natl. Acad. Sci. USA,* 1983, 21-25
- **Boros et al.** *Gene,* 1986, vol. 42, 97-100
- **Bernard R. Glick ; Jack J. Pasternak.** Molekulare Biotechnologie. Spektrum Akademischer Verlag, 1995, 342
- **deBoer et al.** *Proc. Natl. Acad. Sci. USA,* 1983, vol. 80, 21-25
- **Sambrook et al.** Molecular Cloning: A Laboratory Course Manual. Cold Spring Harbor Press, 1989
- *Methods in Enzymology,* 135, 136, 137
- Light Scattering from Polymer solutions. Academic Press, 1972
- **Berry.** *J. Chem. Phys.,* 1966, vol. 44, 4550ff
- **Hovenkamp-Hermelink et al.** *Methode (Potato Research,* 1988, vol. 31, 241-246
- **Nielsen et al.** *Plant Physiol.,* 1994, vol. 105, 11-117
- **Ames.** *Methods in Enzymology,* 1966, vol. VIII, 115-118
- **Stitt et al.** *Methods in Enzymology,* 1989, vol. 174, 518-552
- **Lloyd et al.** *Biochem. J.,* 1999, vol. 338, 515-521
- **Ausubel et al.** Current Protocolls in Molecular Biology. J. Wiley & Sons, 1987
- **Adhya ; Schwartz.** *J. Bacteriol.,* 1971, vol. 108, 621-626
- **Birmboim-Doly.** *Nucleic Acid Res.,* vol. 7, 1513-1523
- **Krisman et al.** *Analytical Biochemistry,* 1985, vol. 147, 491-496
- **Brown ; Brown.** *Meth. Enzymol.,* 1966, vol. 8, 395-403
- **Sweet et al.** *Carbohydr. Res.,* 1975, vol. 40, 217

• **Burchard, W.** *Macromolecules,* 1977, vol. 10, 919

SEQUENCE LISTING

<110> PlantTec Biotechnologie GmbH
Max-Planck-Gesellschaft zur Förderung der Wissenschaften e.V.

<120> Nucleinsäuremoleküle codierend ein Verzweigungsenzym aus Bakterien der Gattung Neisseria sowie Verfahren zur Herstellung von alpha-1,6-verzweigten alpha-1,4-Glucanen

<130> C1434PCT

<140>
<141>

<160> 34

<170> PatentIn Ver. 2.1

<210> 1
<211> 2475
<212> DNA
<213> Neisseria denitrificans

<220>
<221> CDS
<222> (170)..(2458)

<400> 1

```
actgtatgcc gtgcagctgg aaaacctgct gggcgtacgc gacaacctca atattcccgg 60

cgtggccgaa ggctatccga actgggcgcg caaaatgccg cagcctctgg aagcctttgc 120

ccgccacccg caaatgggca agcagcttgc catgatggga gacatccgc atg aac cga 178
                                                      Met Asn Arg
                                                        1

aac cgc cat atc cga cgc ggc tac cac ccg gaa gcc gga gaa cgc caa   226
Asn Arg His Ile Arg Arg Gly Tyr His Pro Glu Ala Gly Glu Arg Gln
    5                  10                  15

atc atc gac agc ctg ttt gcc gcc acc cac agc gat ccg ttt gcc tat   274
Ile Ile Asp Ser Leu Phe Ala Ala Thr His Ser Asp Pro Phe Ala Tyr
 20                  25                  30                  35

ctt ggg cgg cat cgt gtc aac gac gaa cgc gaa gcc gtg cgc gtg ctg   322
Leu Gly Arg His Arg Val Asn Asp Glu Arg Glu Ala Val Arg Val Leu
                 40                  45                  50

cgt ccc gac gcg cac cac atc gac atc atc gac cgc cac aca ggc gca   370
Arg Pro Asp Ala His His Ile Asp Ile Ile Asp Arg His Thr Gly Ala
             55                  60                  65

gtc atc atg ccg tct gaa aaa atc gac gag cgc ggc ctg ttt gcc gcc   418
Val Ile Met Pro Ser Glu Lys Ile Asp Glu Arg Gly Leu Phe Ala Ala
         70                  75                  80

gta ttg ccc gaa cac gcg ccc gac tac gcc ctg ctg gtg aca tac cac   466
Val Leu Pro Glu His Ala Pro Asp Tyr Ala Leu Leu Val Thr Tyr His
     85                  90                  95

gag ggc gaa gcc gcc gta cgc gaa gaa gat gac tac cgc ttc ggc agc   514
```

Glu Gly Glu Ala Ala Val Arg Glu Glu Asp Asp Tyr Arg Phe Gly Ser
100 105 110 115

gcg ctg caa cat acc gat gcc tgg ctg ctg ggc gaa ggc acg cac ctg 562
Ala Leu Gln His Thr Asp Ala Trp Leu Leu Gly Glu Gly Thr His Leu
120 125 130

cgc cct tat gaa acg ctg ggc gca cat ttc gcc gaa atg gac ggc gta 610
Arg Pro Tyr Glu Thr Leu Gly Ala His Phe Ala Glu Met Asp Gly Val
135 140 145

tcc ggc gtg cgc ttt gcc gtt tgg gcg ccc aac gcg cgg cgg gta tcg 658
Ser Gly Val Arg Phe Ala Val Trp Ala Pro Asn Ala Arg Arg Val Ser
150 155 160

gtc atc ggc gaa ttc aac ggc tgg gac agc cgc cgc cat gcc atg cgt 706
Val Ile Gly Glu Phe Asn Gly Trp Asp Ser Arg Arg His Ala Met Arg
165 170 175

ccg cac aca ggc aac ggc ctg tgg gac atc ttt atc ccc ggc gtc ggc 754
Pro His Thr Gly Asn Gly Leu Trp Asp Ile Phe Ile Pro Gly Val Gly
180 185 190 195

ctc aac gcg ctg tat aaa ttc tcc gta ctc gat gcc aac ggc aac atc 802
Leu Asn Ala Leu Tyr Lys Phe Ser Val Leu Asp Ala Asn Gly Asn Ile
200 205 210

cgc gaa aaa gcc gac ccc tac gca ttc ggc gcg gag ctg cgc ccg acc 850
Arg Glu Lys Ala Asp Pro Tyr Ala Phe Gly Ala Glu Leu Arg Pro Thr
215 220 225

acc gca tcc gtg gtg cgc ggc ttg ccg gcc aaa gcc gaa gcg ccc gct 898
Thr Ala Ser Val Val Arg Gly Leu Pro Ala Lys Ala Glu Ala Pro Ala
230 235 240

ttc cgc cgc cgc gcc aac tcc gtg gaa gcg ccc atc agc att tac gaa 946
Phe Arg Arg Arg Ala Asn Ser Val Glu Ala Pro Ile Ser Ile Tyr Glu
245 250 255

gtc cat ctc ggc tcg tgg cgg cgc aat ccc gaa aac aac tac tgg ctc 994
Val His Leu Gly Ser Trp Arg Arg Asn Pro Glu Asn Asn Tyr Trp Leu
260 265 270 275

acc tac acg cag ctg gcc gac gaa ttg gtg aac tat gta aaa gac atg 1042
Thr Tyr Thr Gln Leu Ala Asp Glu Leu Val Asn Tyr Val Lys Asp Met
280 285 290

ggc ttc acc cac atc gag ctg ctg ccc ttg tcc gaa tat ccg ttc gac 1090
Gly Phe Thr His Ile Glu Leu Leu Pro Leu Ser Glu Tyr Pro Phe Asp
295 300 305

ggc tca tgg ggc tac caa gcc acc ggc ctg tat gca ccg acc agc cgc 1138
Gly Ser Trp Gly Tyr Gln Ala Thr Gly Leu Tyr Ala Pro Thr Ser Arg
310 315 320

ttc ggc tcg ccc gat gag ctg aaa gcc ctg att gac gcc gcc cac gcc 1186
Phe Gly Ser Pro Asp Glu Leu Lys Ala Leu Ile Asp Ala Ala His Ala
325 330 335

gcc ggc atc agc gtg att ctc gac tgg gta gcg ggg cac ttc ccc acc 1234
Ala Gly Ile Ser Val Ile Leu Asp Trp Val Ala Gly His Phe Pro Thr
340 345 350 355

```
gac gac cac ggc ctc aac acc ttc gac ggc acg gcg ctt tac gaa cac     1282
Asp Asp His Gly Leu Asn Thr Phe Asp Gly Thr Ala Leu Tyr Glu His
                360                 365                 370

gcc gac ccg cgc gaa ggc tac cat cag gat tgg aac acg ctg att tac     1330
Ala Asp Pro Arg Glu Gly Tyr His Gln Asp Trp Asn Thr Leu Ile Tyr
            375                 380                 385

aac ttc ggc cgc aac gaa gtc aaa aac ttc ctg cag ggc aac gcg ctc     1378
Asn Phe Gly Arg Asn Glu Val Lys Asn Phe Leu Gln Gly Asn Ala Leu
        390                 395                 400

tac tgg att gag cgt ttc ggc ttc gac ggc atc cgc gtg gac gcc gtg     1426
Tyr Trp Ile Glu Arg Phe Gly Phe Asp Gly Ile Arg Val Asp Ala Val
    405                 410                 415

gcc tcg atg att tac cgc aac tac tcg cgc aaa gac ggc gag tgg att     1474
Ala Ser Met Ile Tyr Arg Asn Tyr Ser Arg Lys Asp Gly Glu Trp Ile
420                 425                 430                 435

ccc aac cgc tac ggc ggc agc gaa aat ctg gaa gcc atc gcc ttt ttg     1522
Pro Asn Arg Tyr Gly Gly Ser Glu Asn Leu Glu Ala Ile Ala Phe Leu
                440                 445                 450

cgc caa acc aat gcc gtc tta aaa agc gaa aca ccc ggc gcc ggc tcg     1570
Arg Gln Thr Asn Ala Val Leu Lys Ser Glu Thr Pro Gly Ala Gly Ser
            455                 460                 465

ttt gcc gaa gaa tcg act tcc ttt gcc gac gta acc cgc gaa gcc ggc     1618
Phe Ala Glu Glu Ser Thr Ser Phe Ala Asp Val Thr Arg Glu Ala Gly
        470                 475                 480

ctg aac ttc gat ttc aaa tgg aat atg ggc tgg atg aac gac acc ctg     1666
Leu Asn Phe Asp Phe Lys Trp Asn Met Gly Trp Met Asn Asp Thr Leu
    485                 490                 495

cgc tat atg cag gaa gac ccc gtc cac cgc aaa tac cac cac ggc aaa     1714
Arg Tyr Met Gln Glu Asp Pro Val His Arg Lys Tyr His His Gly Lys
500                 505                 510                 515

atg aca ttc ggc atg atg tac caa tac agc gaa aac ttc gtt ctg ccc     1762
Met Thr Phe Gly Met Met Tyr Gln Tyr Ser Glu Asn Phe Val Leu Pro
                520                 525                 530

ctg tcg cac gac gaa gtg gta cac ggc aaa cgc tcg ctg ctg ggc aaa     1810
Leu Ser His Asp Glu Val Val His Gly Lys Arg Ser Leu Leu Gly Lys
            535                 540                 545

atg ccg ggc gac tgc tgg cag cag ttt gcc aac ctg cgc gcc tat tac     1858
Met Pro Gly Asp Cys Trp Gln Gln Phe Ala Asn Leu Arg Ala Tyr Tyr
        550                 555                 560

ggc ttt atg tac ggc ttc ccc ggc aaa aaa ctc cta ttt atg ggc aac     1906
Gly Phe Met Tyr Gly Phe Pro Gly Lys Lys Leu Leu Phe Met Gly Asn
    565                 570                 575

gaa ttt gcc caa ggc cgc gag tgg aat tat cag gaa gga ctg gat tgg     1954
Glu Phe Ala Gln Gly Arg Glu Trp Asn Tyr Gln Glu Gly Leu Asp Trp
580                 585                 590                 595

cat ctg ctc gac gaa gcg ggc ggc tgg cac aaa ggc gtg cag gat tat     2002
```

His Leu Leu Asp Glu Ala Gly Gly Trp His Lys Gly Val Gln Asp Tyr
600 605 610

gta cgc gac ctg aac cac atc tac acc gcc cac gcc ccg ctc tac cag 2050
Val Arg Asp Leu Asn His Ile Tyr Thr Ala His Ala Pro Leu Tyr Gln
615 620 625

ctc gac cag cag ccc gag ggc ttt gaa tgg ctg gtg gcc gac gac agc 2098
Leu Asp Gln Gln Pro Glu Gly Phe Glu Trp Leu Val Ala Asp Asp Ser
630 635 640

gac aat tcg gta ttc gta ttc gag cgc cgc gac cgc gca ggc aac cgc 2146
Asp Asn Ser Val Phe Val Phe Glu Arg Arg Asp Arg Ala Gly Asn Arg
645 650 655

atc atc gtc atc agc aac ttt acc ccg gtg gtg cgc gaa cac tac cgc 2194
Ile Ile Val Ile Ser Asn Phe Thr Pro Val Val Arg Glu His Tyr Arg
660 665 670 675

ttc ggc gtc aac gcg ccc ggc cgc tat acc gaa atc ctg aat tcc gac 2242
Phe Gly Val Asn Ala Pro Gly Arg Tyr Thr Glu Ile Leu Asn Ser Asp
680 685 690

cgc acg cag tat caa ggc agc ggc atc gca aac ggc gcg gac atc acg 2290
Arg Thr Gln Tyr Gln Gly Ser Gly Ile Ala Asn Gly Ala Asp Ile Thr
695 700 705

gcg gaa aac gtg cct tcg cac ggc aaa gcg cag tcg ctg agc ctg acc 2338
Ala Glu Asn Val Pro Ser His Gly Lys Ala Gln Ser Leu Ser Leu Thr
710 715 720

ctg ccg ccg ctg gcc acg gtc tat ctg tat cag aaa gcc gcg ccc gca 2386
Leu Pro Pro Leu Ala Thr Val Tyr Leu Tyr Gln Lys Ala Ala Pro Ala
725 730 735

acg gaa att cag acg gcc ttg cgc gcc gac aag cag ccg gcg gta aaa 2434
Thr Glu Ile Gln Thr Ala Leu Arg Ala Asp Lys Gln Pro Ala Val Lys
740 745 750 755

gat aag cag gca aaa gcc aaa taa agcggcacca tactgcc 2475
Asp Lys Gln Ala Lys Ala Lys
760

<210> 2
<211> 762
<212> PRT
<213> Neisseria denitrificans

<400> 2

```
Met Asn Arg Asn Arg His Ile Arg Arg Gly Tyr His Pro Glu Ala Gly
 1               5                  10                  15

Glu Arg Gln Ile Ile Asp Ser Leu Phe Ala Ala Thr His Ser Asp Pro
            20                  25                  30

Phe Ala Tyr Leu Gly Arg His Arg Val Asn Asp Glu Arg Glu Ala Val
        35                  40                  45

Arg Val Leu Arg Pro Asp Ala His His Ile Asp Ile Ile Asp Arg His
    50                  55                  60
```

Thr Gly Ala Val Ile Met Pro Ser Glu Lys Ile Asp Glu Arg Gly Leu
65 70 75 80

Phe Ala Ala Val Leu Pro Glu His Ala Pro Asp Tyr Ala Leu Leu Val
85 90 95

Thr Tyr His Glu Gly Glu Ala Ala Val Arg Glu Glu Asp Asp Tyr Arg
100 105 110

Phe Gly Ser Ala Leu Gln His Thr Asp Ala Trp Leu Leu Gly Glu Gly
115 120 125

Thr His Leu Arg Pro Tyr Glu Thr Leu Gly Ala His Phe Ala Glu Met
130 135 140

Asp Gly Val Ser Gly Val Arg Phe Ala Val Trp Ala Pro Asn Ala Arg
145 150 155 160

Arg Val Ser Val Ile Gly Glu Phe Asn Gly Trp Asp Ser Arg Arg His
165 170 175

Ala Met Arg Pro His Thr Gly Asn Gly Leu Trp Asp Ile Phe Ile Pro
180 185 190

Gly Val Gly Leu Asn Ala Leu Tyr Lys Phe Ser Val Leu Asp Ala Asn
195 200 205

Gly Asn Ile Arg Glu Lys Ala Asp Pro Tyr Ala Phe Gly Ala Glu Leu
210 215 220

Arg Pro Thr Thr Ala Ser Val Val Arg Gly Leu Pro Ala Lys Ala Glu
225 230 235 240

Ala Pro Ala Phe Arg Arg Arg Ala Asn Ser Val Glu Ala Pro Ile Ser
245 250 255

Ile Tyr Glu Val His Leu Gly Ser Trp Arg Arg Asn Pro Glu Asn Asn
260 265 270

Tyr Trp Leu Thr Tyr Thr Gln Leu Ala Asp Glu Leu Val Asn Tyr Val
275 280 285

Lys Asp Met Gly Phe Thr His Ile Glu Leu Leu Pro Leu Ser Glu Tyr
290 295 300

Pro Phe Asp Gly Ser Trp Gly Tyr Gln Ala Thr Gly Leu Tyr Ala Pro
305 310 315 320

Thr Ser Arg Phe Gly Ser Pro Asp Glu Leu Lys Ala Leu Ile Asp Ala
325 330 335

Ala His Ala Ala Gly Ile Ser Val Ile Leu Asp Trp Val Ala Gly His
340 345 350

Phe Pro Thr Asp Asp His Gly Leu Asn Thr Phe Asp Gly Thr Ala Leu
355 360 365

Tyr Glu His Ala Asp Pro Arg Glu Gly Tyr His Gln Asp Trp Asn Thr
370 375 380

Leu Ile Tyr Asn Phe Gly Arg Asn Glu Val Lys Asn Phe Leu Gln Gly
385 390 395 400

```
Asn Ala Leu Tyr Trp Ile Glu Arg Phe Gly Phe Asp Gly Ile Arg Val
                405                 410                 415

Asp Ala Val Ala Ser Met Ile Tyr Arg Asn Tyr Ser Arg Lys Asp Gly
            420                 425                 430

Glu Trp Ile Pro Asn Arg Tyr Gly Gly Ser Glu Asn Leu Glu Ala Ile
        435                 440                 445

Ala Phe Leu Arg Gln Thr Asn Ala Val Leu Lys Ser Glu Thr Pro Gly
    450                 455                 460

Ala Gly Ser Phe Ala Glu Glu Ser Thr Ser Phe Ala Asp Val Thr Arg
465                 470                 475                 480

Glu Ala Gly Leu Asn Phe Asp Phe Lys Trp Asn Met Gly Trp Met Asn
                485                 490                 495

Asp Thr Leu Arg Tyr Met Gln Glu Asp Pro Val His Arg Lys Tyr His
            500                 505                 510

His Gly Lys Met Thr Phe Gly Met Met Tyr Gln Tyr Ser Glu Asn Phe
        515                 520                 525

Val Leu Pro Leu Ser His Asp Glu Val Val His Gly Lys Arg Ser Leu
    530                 535                 540

Leu Gly Lys Met Pro Gly Asp Cys Trp Gln Gln Phe Ala Asn Leu Arg
545                 550                 555                 560

Ala Tyr Tyr Gly Phe Met Tyr Gly Phe Pro Gly Lys Lys Leu Leu Phe
                565                 570                 575

Met Gly Asn Glu Phe Ala Gln Gly Arg Glu Trp Asn Tyr Gln Glu Gly
            580                 585                 590

Leu Asp Trp His Leu Leu Asp Glu Ala Gly Gly Trp His Lys Gly Val
        595                 600                 605

Gln Asp Tyr Val Arg Asp Leu Asn His Ile Tyr Thr Ala His Ala Pro
    610                 615                 620

Leu Tyr Gln Leu Asp Gln Gln Pro Glu Gly Phe Glu Trp Leu Val Ala
625                 630                 635                 640

Asp Asp Ser Asp Asn Ser Val Phe Val Phe Glu Arg Arg Asp Arg Ala
                645                 650                 655

Gly Asn Arg Ile Ile Val Ile Ser Asn Phe Thr Pro Val Val Arg Glu
            660                 665                 670

His Tyr Arg Phe Gly Val Asn Ala Pro Gly Arg Tyr Thr Glu Ile Leu
        675                 680                 685

Asn Ser Asp Arg Thr Gln Tyr Gln Gly Ser Gly Ile Ala Asn Gly Ala
    690                 695                 700

Asp Ile Thr Ala Glu Asn Val Pro Ser His Gly Lys Ala Gln Ser Leu
705                 710                 715                 720

Ser Leu Thr Leu Pro Pro Leu Ala Thr Val Tyr Leu Tyr Gln Lys Ala
```

725 730 735

Ala Pro Ala Thr Glu Ile Gln Thr Ala Leu Arg Ala Asp Lys Gln Pro
740 745 750

Ala Val Lys Asp Lys Gln Ala Lys Ala Lys
755 760

<210> 3
<211> 6
<212> PRT
<213> Neisseria denitrificans

<400> 3

Met Asn Arg Asn Xaa His
1 5

<210> 4
<211> 2914
<212> DNA
<213> Neisseria polysaccharea

<220>
<221> CDS
<222> (957)..(2867)

<400> 4

```
gagttttgcg ttcccgaacc gaacgtgatg cttgagccga acacctgtcc ggcaaggcgg 60
ctgaccgccc ccttttgccc catcgacatc gtaacaatcg gtttggtggc aagctctttc 120
gctttgagcg tggcagaaag caaagtcagc acgtcttccg cgctttgcgg catcaccgca 180
attttgcaga tgtccgcgcc gcagtcctcc atctgtttca gacggcatac gatttcttct 240
tgcggcggcg tgcggtgaaa ctcatgattg cagagcaggg cggcgatgcc gtttttttga 300
gcatgcgcca cggcgcgccg gacggcggtt tcgccggaaa aaagctcgat atcgataatg 360
tcgggcaggc ggctttcaat cagcgagtcg agcagttcaa aataataatc gtccgaacac 420
gggaacgagc cgccttcgcc atgccgtctg aacgtaaaca gcagcggctt gtcgggcagc 480
gcgtcgcgga cggtctgcgt gtggcgcaat acttcgccga tgctgcccgc gcattccaaa 540
aaatcggcgc ggaactcgac gatatcgaag ggcaggtttt tgatttggtc aagtacggcg 600
gaaagtacgg cggcatcgcg ggcgacaagc ggcacggcga ttttggtgcg tccgcttccg 660
ataacggtgt ttttgacggt caggctggtg tgcatggcgg ttgttgcggc tgaaaggaac 720
ggtaaagacg caattatagc aaaggcacag gcaatgtttc agacggcatt tctgtgcggc 780
cggcttgata tgaatcaagc agcatccgca tatcggaatg cagacttggc acaagccctg 840
tcttttctag tcagtccgca gttcttgcag tatgattgca cgacacgccc tacacggcat 900
ttgcaggata cggcggcaga ccgccggtcg gaaacttcag aatcggagca ggcatc atg 959
                                                              Met
                                                              1
```

```
ttg acc ccc acg cag caa gtc ggt ttg att tta cag tac ctc aaa aca    1007
Leu Thr Pro Thr Gln Gln Val Gly Leu Ile Leu Gln Tyr Leu Lys Thr
            5                  10                  15

cgc atc ttg gac atc tac acg ccc gaa cag cgc gcc ggc atc gaa aaa    1055
Arg Ile Leu Asp Ile Tyr Thr Pro Glu Gln Arg Ala Gly Ile Glu Lys
        20                  25                  30

tcc gaa gac tgg cgg cag ttt tcg cgc cgc atg gat acg cat ttc ccc    1103
Ser Glu Asp Trp Arg Gln Phe Ser Arg Arg Met Asp Thr His Phe Pro
    35                  40                  45

aaa ctg atg aac gaa ctc gac agc gtg tac ggc aac aac gaa gcc ctg    1151
Lys Leu Met Asn Glu Leu Asp Ser Val Tyr Gly Asn Asn Glu Ala Leu
50                  55                  60                  65

ctg cct atg ctg gaa atg ctg ctg gcg cag gca tgg caa agc tat tcc    1199
Leu Pro Met Leu Glu Met Leu Leu Ala Gln Ala Trp Gln Ser Tyr Ser
                70                  75                  80

caa cgc aac tca tcc tta aaa gat atc gat atc gcg cgc gaa aac aac    1247
Gln Arg Asn Ser Ser Leu Lys Asp Ile Asp Ile Ala Arg Glu Asn Asn
            85                  90                  95

ccc gat tgg att ttg tcc aac aaa caa gtc ggc ggc gtg tgc tac gtt    1295
Pro Asp Trp Ile Leu Ser Asn Lys Gln Val Gly Gly Val Cys Tyr Val
        100                 105                 110

gat ttg ttt gcc ggc gat ttg aag ggc ttg aaa gat aaa att cct tat    1343
Asp Leu Phe Ala Gly Asp Leu Lys Gly Leu Lys Asp Lys Ile Pro Tyr
    115                 120                 125

ttt caa gag ctt ggt ttg act tat ctg cac ctg atg ccg ctg ttt aaa    1391
Phe Gln Glu Leu Gly Leu Thr Tyr Leu His Leu Met Pro Leu Phe Lys
130                 135                 140                 145

tgc cct gaa ggc aaa agc gac ggc ggc tat gcg gtc agc agc tac cgc    1439
Cys Pro Glu Gly Lys Ser Asp Gly Gly Tyr Ala Val Ser Ser Tyr Arg
                150                 155                 160

gat gtc aat ccg gca ctg ggc aca ata ggc gac ttg cgc gaa gtc att    1487
Asp Val Asn Pro Ala Leu Gly Thr Ile Gly Asp Leu Arg Glu Val Ile
            165                 170                 175

gct gcg ctg cac gaa gcc ggc att tcc gcc gtc gtc gat ttt atc ttc    1535
Ala Ala Leu His Glu Ala Gly Ile Ser Ala Val Val Asp Phe Ile Phe
        180                 185                 190

aac cac acc tcc aac gaa cac gaa tgg gcg caa cgc tgc gcc gcc ggc    1583
Asn His Thr Ser Asn Glu His Glu Trp Ala Gln Arg Cys Ala Ala Gly
    195                 200                 205

gac ccg ctt ttc gac aat ttc tac tat att ttc ccc gac cgc cgg atg    1631
Asp Pro Leu Phe Asp Asn Phe Tyr Tyr Ile Phe Pro Asp Arg Arg Met
210                 215                 220                 225

ccc gac caa tac gac cgc acc ctg cgc gaa atc ttc ccc gac cag cac    1679
Pro Asp Gln Tyr Asp Arg Thr Leu Arg Glu Ile Phe Pro Asp Gln His
                230                 235                 240

ccg ggc ggc ttc tcg caa ctg gaa gac gga cgc tgg gtg tgg acg acc    1727
```

```
Pro Gly Gly Phe Ser Gln Leu Glu Asp Gly Arg Trp Val Trp Thr Thr
            245                 250                 255

ttc aat tcc ttc caa tgg gac ttg aat tac agc aac ccg tgg gta ttc    1775
Phe Asn Ser Phe Gln Trp Asp Leu Asn Tyr Ser Asn Pro Trp Val Phe
        260                 265                 270

cgc gca atg gcg ggc gaa atg ctg ttc ctt gcc aac ttg ggc gtt gac    1823
Arg Ala Met Ala Gly Glu Met Leu Phe Leu Ala Asn Leu Gly Val Asp
    275                 280                 285

atc ctg cgt atg gat gcg gtt gcc ttt att tgg aaa caa atg ggg aca    1871
Ile Leu Arg Met Asp Ala Val Ala Phe Ile Trp Lys Gln Met Gly Thr
290                 295                 300                 305

agc tgc gaa aac ctg ccg cag gcg cac gcc ctc atc cgc gcg ttc aat    1919
Ser Cys Glu Asn Leu Pro Gln Ala His Ala Leu Ile Arg Ala Phe Asn
                310                 315                 320

gcc gtt atg cgt att gcc gcg ccc gcc gtg ttc ttc aaa tcc gaa gcc    1967
Ala Val Met Arg Ile Ala Ala Pro Ala Val Phe Phe Lys Ser Glu Ala
            325                 330                 335

atc gtc cac ccc gac caa gtc gtc caa tac atc ggg cag gac gaa tgc    2015
Ile Val His Pro Asp Gln Val Val Gln Tyr Ile Gly Gln Asp Glu Cys
        340                 345                 350

caa atc ggt tac aac ccc ctg caa atg gca ttg ttg tgg aac acc ctt    2063
Gln Ile Gly Tyr Asn Pro Leu Gln Met Ala Leu Leu Trp Asn Thr Leu
    355                 360                 365

gcc acg cgc gaa gtc aac ctg ctc cat cag gcg ctg acc tac cgc cac    2111
Ala Thr Arg Glu Val Asn Leu Leu His Gln Ala Leu Thr Tyr Arg His
370                 375                 380                 385

aac ctg ccc gag cat acc gcc tgg gtc aac tac gtc cgc agc cac gac    2159
Asn Leu Pro Glu His Thr Ala Trp Val Asn Tyr Val Arg Ser His Asp
                390                 395                 400

gac atc ggc tgg acg ttt gcc gat gaa gac gcg gca tat ctg ggc ata    2207
Asp Ile Gly Trp Thr Phe Ala Asp Glu Asp Ala Ala Tyr Leu Gly Ile
            405                 410                 415

agc ggc tac gac cac cgc caa ttc ctc aac cgc ttc ttc gtc aac cgt    2255
Ser Gly Tyr Asp His Arg Gln Phe Leu Asn Arg Phe Phe Val Asn Arg
        420                 425                 430

ttc gac ggc agc ttc gct cgt ggc gta ccg ttc caa tac aac cca agc    2303
Phe Asp Gly Ser Phe Ala Arg Gly Val Pro Phe Gln Tyr Asn Pro Ser
    435                 440                 445

aca ggc gac tgc cgt gtc agt ggt aca gcc gcg gca ttg gtc ggc ttg    2351
Thr Gly Asp Cys Arg Val Ser Gly Thr Ala Ala Ala Leu Val Gly Leu
450                 455                 460                 465

gcg caa gac gat ccc cac gcc gtt gac cgc atc aaa ctc ttg tac agc    2399
Ala Gln Asp Asp Pro His Ala Val Asp Arg Ile Lys Leu Leu Tyr Ser
                470                 475                 480

att gct ttg agt acc ggc ggt ctg ccg ctg att tac cta ggc gac gaa    2447
Ile Ala Leu Ser Thr Gly Gly Leu Pro Leu Ile Tyr Leu Gly Asp Glu
            485                 490                 495
```

```
gtg ggt acg ctc aat gac gac gac tgg tcg caa gac agc aat aag agc    2495
Val Gly Thr Leu Asn Asp Asp Asp Trp Ser Gln Asp Ser Asn Lys Ser
        500                     505                     510

gac gac agc cgt tgg gcg cac cgt ccg cgc tac aac gaa gcc ctg tac    2543
Asp Asp Ser Arg Trp Ala His Arg Pro Arg Tyr Asn Glu Ala Leu Tyr
    515                     520                     525

gcg caa cgc aac gat ccg tcg acc gca gcc ggg caa atc tat cag ggc    2591
Ala Gln Arg Asn Asp Pro Ser Thr Ala Ala Gly Gln Ile Tyr Gln Gly
530                     535                     540                 545

ttg cgc cat atg att gcc gtc cgc caa agc aat ccg cgc ttc gac ggc    2639
Leu Arg His Met Ile Ala Val Arg Gln Ser Asn Pro Arg Phe Asp Gly
                550                     555                     560

ggc agg ctg gtt aca ttc aac acc aac aac aag cac atc atc ggc tac    2687
Gly Arg Leu Val Thr Phe Asn Thr Asn Asn Lys His Ile Ile Gly Tyr
            565                     570                     575

atc cgc aac aat gcg ctt ttg gca ttc ggt aac ttc agc gaa tat ccg    2735
Ile Arg Asn Asn Ala Leu Leu Ala Phe Gly Asn Phe Ser Glu Tyr Pro
        580                     585                     590

caa acc gtt acc gcg cat acc ctg caa gcc atg ccc ttc aag gcg cac    2783
Gln Thr Val Thr Ala His Thr Leu Gln Ala Met Pro Phe Lys Ala His
    595                     600                     605

gac ctc atc ggt ggc aaa act gtc agc ctg aat cag gat ttg acg ctt    2831
Asp Leu Ile Gly Gly Lys Thr Val Ser Leu Asn Gln Asp Leu Thr Leu
610                     615                     620                 625

cag ccc tat cag gtc atg tgg ctc gaa atc gcc tga cgcacgcttc         2877
Gln Pro Tyr Gln Val Met Trp Leu Glu Ile Ala
                630                     635

ccaaatgccg tctgaaccgt ttcagacggc atttgcg                           2914
```

<210> 5
<211> 636
<212> PRT
<213> Neisseria polysaccharea

<400> 5

Met Leu Thr Pro Thr Gln Gln Val Gly Leu Ile Leu Gln Tyr Leu Lys
1 5 10 15

Thr Arg Ile Leu Asp Ile Tyr Thr Pro Glu Gln Arg Ala Gly Ile Glu
20 25 30

Lys Ser Glu Asp Trp Arg Gln Phe Ser Arg Arg Met Asp Thr His Phe
35 40 45

Pro Lys Leu Met Asn Glu Leu Asp Ser Val Tyr Gly Asn Asn Glu Ala
50 55 60

Leu Leu Pro Met Leu Glu Met Leu Leu Ala Gln Ala Trp Gln Ser Tyr
65 70 75 80

Ser Gln Arg Asn Ser Ser Leu Lys Asp Ile Asp Ile Ala Arg Glu Asn

```
                    85                  90                  95

Asn Pro Asp Trp Ile Leu Ser Asn Lys Gln Val Gly Gly Val Cys Tyr
            100                 105                 110

Val Asp Leu Phe Ala Gly Asp Leu Lys Gly Leu Lys Asp Lys Ile Pro
        115                 120                 125

Tyr Phe Gln Glu Leu Gly Leu Thr Tyr Leu His Leu Met Pro Leu Phe
    130                 135                 140

Lys Cys Pro Glu Gly Lys Ser Asp Gly Gly Tyr Ala Val Ser Ser Tyr
145                 150                 155                 160

Arg Asp Val Asn Pro Ala Leu Gly Thr Ile Gly Asp Leu Arg Glu Val
                165                 170                 175

Ile Ala Ala Leu His Glu Ala Gly Ile Ser Ala Val Val Asp Phe Ile
            180                 185                 190

Phe Asn His Thr Ser Asn Glu His Glu Trp Ala Gln Arg Cys Ala Ala
        195                 200                 205

Gly Asp Pro Leu Phe Asp Asn Phe Tyr Tyr Ile Phe Pro Asp Arg Arg
    210                 215                 220

Met Pro Asp Gln Tyr Asp Arg Thr Leu Arg Glu Ile Phe Pro Asp Gln
225                 230                 235                 240

His Pro Gly Gly Phe Ser Gln Leu Glu Asp Gly Arg Trp Val Trp Thr
                245                 250                 255

Thr Phe Asn Ser Phe Gln Trp Asp Leu Asn Tyr Ser Asn Pro Trp Val
            260                 265                 270

Phe Arg Ala Met Ala Gly Glu Met Leu Phe Leu Ala Asn Leu Gly Val
        275                 280                 285

Asp Ile Leu Arg Met Asp Ala Val Ala Phe Ile Trp Lys Gln Met Gly
    290                 295                 300

Thr Ser Cys Glu Asn Leu Pro Gln Ala His Ala Leu Ile Arg Ala Phe
305                 310                 315                 320

Asn Ala Val Met Arg Ile Ala Ala Pro Ala Val Phe Phe Lys Ser Glu
                325                 330                 335

Ala Ile Val His Pro Asp Gln Val Val Gln Tyr Ile Gly Gln Asp Glu
            340                 345                 350

Cys Gln Ile Gly Tyr Asn Pro Leu Gln Met Ala Leu Leu Trp Asn Thr
        355                 360                 365

Leu Ala Thr Arg Glu Val Asn Leu Leu His Gln Ala Leu Thr Tyr Arg
    370                 375                 380

His Asn Leu Pro Glu His Thr Ala Trp Val Asn Tyr Val Arg Ser His
385                 390                 395                 400

Asp Asp Ile Gly Trp Thr Phe Ala Asp Glu Asp Ala Ala Tyr Leu Gly
                405                 410                 415
```

```
Ile Ser Gly Tyr Asp His Arg Gln Phe Leu Asn Arg Phe Phe Val Asn
            420                     425                     430

Arg Phe Asp Gly Ser Phe Ala Arg Gly Val Pro Phe Gln Tyr Asn Pro
        435                     440                     445

Ser Thr Gly Asp Cys Arg Val Ser Gly Thr Ala Ala Ala Leu Val Gly
    450                     455                     460

Leu Ala Gln Asp Asp Pro His Ala Val Asp Arg Ile Lys Leu Leu Tyr
465                     470                     475                     480

Ser Ile Ala Leu Ser Thr Gly Gly Leu Pro Leu Ile Tyr Leu Gly Asp
                485                     490                     495

Glu Val Gly Thr Leu Asn Asp Asp Asp Trp Ser Gln Asp Ser Asn Lys
            500                     505                     510

Ser Asp Asp Ser Arg Trp Ala His Arg Pro Arg Tyr Asn Glu Ala Leu
        515                     520                     525

Tyr Ala Gln Arg Asn Asp Pro Ser Thr Ala Ala Gly Gln Ile Tyr Gln
    530                     535                     540

Gly Leu Arg His Met Ile Ala Val Arg Gln Ser Asn Pro Arg Phe Asp
545                     550                     555                     560

Gly Gly Arg Leu Val Thr Phe Asn Thr Asn Asn Lys His Ile Ile Gly
                565                     570                     575

Tyr Ile Arg Asn Asn Ala Leu Leu Ala Phe Gly Asn Phe Ser Glu Tyr
            580                     585                     590

Pro Gln Thr Val Thr Ala His Thr Leu Gln Ala Met Pro Phe Lys Ala
        595                     600                     605

His Asp Leu Ile Gly Gly Lys Thr Val Ser Leu Asn Gln Asp Leu Thr
    610                     615                     620

Leu Gln Pro Tyr Gln Val Met Trp Leu Glu Ile Ala
625                     630                     635
```

<210> 6
<211> 27
<212> DNA
<213> Artificial Sequence

<220>
<223> Description of Artificial Sequence: artificial sequence

<400> 6
gtcgacatga accgaaaccg ccatatc 27

<210> 7
<211> 29
<212> DNA
<213> Artificial Sequence

<220>

<223> Description of Artificial Sequence: artificial sequence

<400> 7
cctgcaggta tggtgccgct ttatttggc 29

<210> 8
<211> 7
<212> PRT
<213> Neisseria denitrificans

<400> 8

Met Asn Arg Asn Arg His Ile
1 5

<210> 9
<211> 6
<212> PRT
<213> Neisseria denitrificans

<400> 9

Arg Pro Asp Ala His His
1 5

<210> 10
<211> 7
<212> PRT
<213> Neisseria denitrificans

<400> 10

His Ala Pro Asp Tyr Ala Leu
1 5

<210> 11
<211> 5
<212> PRT
<213> Neisseria denitrificans

<400> 11

Glu Gly Glu Ala Ala
1 5

<210> 12
<211> 5
<212> PRT
<213> Neisseria denitrificans

<400> 12

Asp Asp Tyr Arg Phe
1 5

<210> 13
<211> 5
<212> PRT
<213> Neisseria denitrificans

<400> 13

Ser Ala Leu Gln His
1 5

<210> 14
<211> 5
<212> PRT
<213> Neisseria denitrificans

<400> 14

Tyr Glu Thr Leu Gly
1 5

<210> 15
<211> 5
<212> PRT
<213> Neisseria denitrificans

<400> 15

Val Ser Gly Val Arg
1 5

<210> 16
<211> 5
<212> PRT
<213> Neisseria denitrificans

<400> 16

Val Ser Val Ile Gly
1 5

<210> 17
<211> 5
<212> PRT
<213> Neisseria denitrificans

<400> 17

Phe Asn Gly Trp Asp
1 5

<210> 18
<211> 5
<212> PRT
<213> Neisseria denitrificans

<400> 18

Leu Tyr Lys Phe Ser
1 5

<210> 19
<211> 5
<212> PRT
<213> Neisseria denitrificans

<400> 19

Pro Tyr Ala Phe Gly
1 5

<210> 20
<211> 6
<212> PRT
<213> Neisseria denitrificans

<400> 20

Arg Pro Thr Thr Ala Ser
1 5

<210> 21
<211> 5
<212> PRT
<213> Neisseria denitrificans

<400> 21

Phe Arg Arg Arg Ala
1 5

<210> 22
<211> 6
<212> PRT
<213> Neisseria denitrificans

<400> 22

Asp Glu Leu Val Asn Tyr
1 5

<210> 23
<211> 6
<212> PRT
<213> Neisseria denitrificans

<400> 23

Leu Pro Leu Ser Glu Tyr
1 5

<210> 24
<211> 6
<212> PRT
<213> Neisseria denitrificans

<400> 24

Tyr Gln Ala Thr Gly Leu
1 5

<210> 25
<211> 5
<212> PRT
<213> Neisseria denitrificans

<400> 25

Asp Asp His Gly Leu
1 5

<210> 26
<211> 5
<212> PRT
<213> Neisseria denitrificans

<400> 26

His Gln Asp Trp Asn
1 5

<210> 27
<211> 5
<212> PRT
<213> Neisseria denitrificans

<400> 27

Asp Gly Ile Arg Val
1 5

<210> 28
<211> 6
<212> PRT
<213> Neisseria denitrificans

<400> 28

Tyr Gly Gly Ser Glu Asn
1 5

<210> 29
<211> 6
<212> PRT
<213> Neisseria denitrificans

<400> 29

Ser Phe Ala Glu Glu Ser

1 5

<210> 30
<211> 5
<212> PRT
<213> Neisseria denitrificans

<400> 30

Asp Pro Val His Arg
1 5

<210> 31
<211> 6
<212> PRT
<213> Neisseria denitrificans

<400> 31

Trp Gln Gln Phe Ala Asn
1 5

<210> 32
<211> 5
<212> PRT
<213> Neisseria denitrificans

<400> 32

Glu Ile Leu Asn Ser
1 5

<210> 33
<211> 8
<212> PRT
<213> Neisseria denitrificans

<400> 33

Ala Thr Glu Ile Gln Thr Ala Leu
1 5

<210> 34
<211> 9
<212> PRT
<213> Neisseria denitrificans

<400> 34

Val Lys Asp Lys Gln Ala Lys Ala Lys
1 5

204
17